# EUROPEAN PATENT SPECIFICATION

(11) **EP 2 110 669 B1**
(45) Date of publication and mention of the grant of the patent: **03.08.2011**
(21) Application number: 09010043.9
(22) Date of filing: 13.06.2003
(51) Int. Cl.: G01N 33/68, G01N 33/569, C12N 15/10, G01N 33/574

(54) **Methods for the identification of allo-antigens and their use for cancer therapy and transplantation**
Verfahren für die Identifizierung von Allo-Antigenen und ihre Verwendung für Krebstherapie und Transplantation
Procédés d'identification d'allo-antigènes et leur utilisation dans la thérapie anticancéreuse et la transplantation

(30) Priority: 13.06.2002 EP 02013423
(43) Date of publication of application: 21.10.2009
(62) Divisional of application: 03738030.0
(73) Proprietor: Merck Patent GmbH, 64293 Darmstadt (DE)
(72) Inventor: Strittmatter, Wolfgang, 64372 Ober-Ramstadt (DE); Moll, Heidrun, 97074 Würzburg (DE); Scharm, Burkhard, 64367 Mühltal (DE)

(56) References cited:
- WO-A-00/42181
- WO-A-01/78708
- WO-A-97/05169
- US-A1- 2001 006 792

## Description

### Field of the invention

The present invention delivers a new concept with respect to the broad definition of allo-antigens that drive graft-versus-tumor (GVT) reaction and/or graft-versus-host disease (GVHD). The new method for the identification of allo-antigens, which has so far been an unresolved technical problem, furthermore calls for new strategies in immunotherapy.

Because the analysis of HLA-binding proteins can now be done with allow-antigens for which the corresponding amino acid sequence is known, the present invention also allows the separation of reactive-reactive T cells that only recognize tumor cells from those that mediate GVHD. Antigens defined with the new technology are especially useful for diagnosis and vaccination in cancer and transplantation-related diseases.

### Background of the invention

It is known in the art that a large portion of tumors express elevated levels of sometimes altered self-protein, which can be regarded as potential targets for immune responses. It further has been shown that the cellular arm of the immune system (T lymphocytes) is capable of recognizing cancer cells in experimental models and human subjects, nevertheless tumors grow progressively.

One hypothesis to explain this paradox is that T lymphocytes do not function properly in the tumor-bearing host. The other alternative is the ability of the tumors to downregulate the antigen-presenting machinery, thus becoming invisible for the T lymphocytes. Therefore, it remains uncertain whether over-expressed or altered protein can stimulate tumor-reactive cytotoxic T lymphocytes (CTLs) and contribute to immunosurveillance of tumor growth. Furthermore, most of the tumor proteins are ubiquitously expressed proteins and they are likely to mediate deletion of specific CTLs from the autologous T-cell repertoire. Auto-reactive T cells are normally deleted at an immature stage of their development by antigen-induced apoptosis or negative selection. In addition to antigen, negative selection may be modulated by different sets of costimulatory signals derived from (APC) (MacKinnon et al., Br. J. Haematol. 2000, 110: 12-17), leading to the formation of an immune system that is tolerant towards self antigens. Despite those unfavorable findings, there is a tremendous interest and expectation that tumor vaccination might work and enable treatments to overcome the shortcomings of current therapeutic approaches.

Chemotherapy in combination with radiotherapy and bone marrow transplantation (BMT) has been explored over the past 20-30 years for some metabolic and hematopoetic disorders, and it became evident that the therapeutic effect is only partially caused by the eradication of leukemia cells using high-dose chemotherapy and irradiation. Numerous clinical observations provide over-convincing evidence that, moreover, (donor T cell) immune responses contribute substantially to the elimination of residual cancerous cells and especially to the subsequent long-term success of BMT-based therapies. In retrospect, the standard therapeutic strategy in BMT overestimated the anticancer potential of even very high doses of chemotherapy and radiotherapy and underestimated the efficacy of immunotherapy mediated by BMT-derived allogeneic donor lymphocytes.

The clinical successes observed after the treatment of hematopoetic disorders (leukemia) with allogeneic bone-marrow transplants (allo-BMT) have to a large extent fulfilled the fundamentals of a curative immunotherapy.

The term allogeneic is used to describe a situation in which the donor and recipient is a different individual, compared to the term syngeneic in which the donor and recipient are identical twins and have an identical tissue type since their genetic make-up is the same. Autologous transplants are derived from an individual which later in the process gets his or her own cells back. But, strictly speaking, this is not a transplantation since no immunologic transplantation barriers exist.

There are two types of allogeneic donors: related, usually sibling donors, and unrelated, usually found from very large pools of volunteers and matched to a tissue type that is the same as the patient's.

Allogeneic transplantation, whether from a related or unrelated donor, differs from either syngeneic or autologous transplantation in that the potential exists for immune rejection of the donated stem cells by the recipient (host- versus-graft effect) and the immune reaction by the donor's immune cells against the tissues of the recipient (graft-versus-host disease).

The immune rejection is usually prevented by intensive treatment of the recipient before the transplantation (conditioning) to suppress the immune system. Conditioning schemes vary according to the transplantation center and the malignancy involved. For instance in treatment of leukemia, the patient is undergoing myeloablative conditioning comprising a combination of high-dose cyclophosphamide and total body irradiation prior to BMT. Post-transplantation the immune reaction is combated by giving immune suppressive drugs, including methotrexate, glucocorticoid hormones (steroids), cyclosporine or a microemulsion thereof (Neoral®), tacrolimus (Prograf®) and mycophenolate mofetil (Cellcept®), for a limited time period in order to prevent acute attack and injure of the patient's tissues. Improvements of supportive care in addition to controlled immunosuppression have reduced toxicity of the conditioning and the post-BMT immune reaction substantially. However, severe complications still occur at oropharynx, gastrointestinal tract, liver, lung, skin, kidney, urinary tract and nervous system and, consequently, allo-BMT is limited to younger, medically fit patients.

In the art, it is generally accepted that hematological cancers cannot always be eradicated by high doses of chemotherapy-radiation conditioning only, but need allo-BMT in addition. Thus, conventional allo-BMT-based therapies have become a standard procedure for the treatment of many human hematological malignancies and provide the benchmark for all immunotherapies - the possibility of a "cure".

Donors for allo-BMT are selected according to their expression of major histocompatibility complex (MHC) molecules: the human leukocyte antigens (HLA). HLA types are genetically determined. Thus, an individual's HLA type is inherited from his or her parents. There are three major genes in a cluster that seem to be particularly important in transplantation: HLA-A, HLA-B, and HLA-DR. Each individual carries two copies of each of to the genes in the HLA cluster. In addition, many allelic versions correspond each of the HLA genes.

To get an ideal 6-out-of-6 match, two people have to carry, the same alleles at each of their two HLA-A, HLA-B, and HLA-DR genes and there is a 1 in 200 chance that a parent and child will be HLA-matched.

When a HLA-matched relative is unavailable and there is time to conduct a search, an unrelated donor is usually considered. The chance of any 2 unrelated individuals being matched for all 6 HLA genes is 1 in a million. Because of the polymorphism of the HLA system, the ethnic background and the median age at diagnosis, transplants from HLA-matched related donors are currently available to 15-60% of newly diagnosed patients. Alternative donors include relatives with minor degrees of incompatibility and HLA-compatible unrelated volunteers. The probability of finding suitable unrelated donors, matched or partially mismatched, has increased with the development of a network of registries now containing more than 4.7 million donors worldwide and with access to other sources such as fetal cord blood.

A bone-marrow transplant mainly consists of hematopoetic stem cells which may be obtained from the bone marrow, blood or fetal cord blood. The hematopoetic stem cells are usually aspirated from the bone marrow. An alternative procedures involves a 3- to 5-days treatment of donors with granulocyte colony-stimulating factor (G-CSF) to mobilize stem cells and progenitor cells from the marrow into the blood. The appropriate cells are then collected from the donor by leukapheresis.

Blood contained in the placenta and umbilical cord of newborn babies is emerging as a new source of stem cells. Cord blood contains significant numbers of stem cells; it has advantages over BMT or adult blood stem cell transplantation for certain patients and may be considered if a matched unrelated marrow stem cell donor is unavailable. One advantage of using umbilical cord blood is that it does not need to be a perfect tissue match with the recipient.

Patients preconditioned as described above receive the stem cell preparation and two to five weeks after transplantation, the engraftment of donated cells becomes apparent by the emergence of normal white cells in the blood of the patient. Red cells and platelets are transfused periodically until marrow function is restored by the transplanted stem cells. The time to hematopoetic recovery is shorter with blood stem cells than with bone marrow cells. Some of the new chimerical immune cells recognize the host as foreign and are going to produce a graft-versus-leukemia effect, hereinafter referred to as graft-versus-tumor (GVT) activity which is usually accompanied by graft-versus-host disease (GVHD). The GVHD reaction occurs when the donor's immune cells, especially the T lymphocytes, recognize that the host cells are different from themselves.

Allo-BMT-induced GVHD is an immune function closely related to GVT which may occur soon after the transplanted cells begin to appear in the recipient. Both types of immune responses are mediated by T cells recognizing cells that are not genetically identical and this could explain the historical finding that transplants between identical twins are less successful than those between matched siblings in the treatment of chronic myeloid leukemia (CML) (Gale et al., Ann. Intem. Med. 1994, 120: 646-652). In the case of stem cell transplantation, the donor cells carefully inspect the cells of the recipient's tissue for signs of differences and attack them if they find significant variations. In the initial phase after transplantation, for instance, residual patient-derived APC are present and will be scanned by donor-derived T cells for differences based on polymorphic genes. A cytotoxic response will be initiated if the donated T cells recognize host cells presenting foreign antigens, which are basically all the immune cells. Whether the T-cell response turns into a dreadful GVHD or a beneficial GVT is determined by the fact that the genetically manifested differences are either presented in the context of cells that belong to the cancerous tissues or organs or, worse, are part of essential non-diseased organs such as skin, joints, lung, liver or kidney. Depending on the importance of the affected organ, GVHD ranges in severity from only small rash to life-threatening illness. Allo-BMT in general remains a somewhat crude approach, with significant transplant-related morbidity and mortality. A recent compilation of reports places the risk of death at 20-41 %, and, despite the availability of potent immunosuppressive drugs, up to 70% of the treated patients still suffer from GVHD. The broad identification of allo-antigens that are responsible for a disease-promoting process as well as the definition of allo-antigens that are useful for the disease-fighting option is therefore the central aspect of the present invention.

Nonetheless, immunotherapy based on BMT offers up to 70% of the patients a leukemia-free survival after transplantion (Clift et al. Haematol. 1997, 10: 319-336).

However, more than 60% of CML patients do not receive allo-BMT owing to disease status, advanced age or lack of a suitable donor.

BMT and/or stem cell transplantation are accepted treatment options for acute myeloid leukemia (AML) in first or subsequent complete remission, AML early relapse or induction failure, acute lymphoblastic leukemia (ALL) in first or subsequent complete remission, ALL in early relapse or induction failure, CML, myelodysplasia, aplastic anemia, Hodgkin's disease sensitive and resistant relapse, aggressive lymphoma sensitive and resistant relapse, and low grade lymphoma.

Graft-versus-host reaction results when the donor's immune cells, especially the T lymphocytes, sense that the host cells are different from themselves. The differences may involve a broad spectrum of proteins that are not detected by HLA typing, or there may be faint differences in HLA type that permit transplantation but not without engendering the reaction. The differences reflect more limited polymorphism in individual codons of the corresponding HLA molecules outside the codons used for HLA typing and matching. It is known that, with the exception of identical twins, some incompatibility will exist even though HLA testing indicates sufficient similarity to permit a transplant to be successful. HLA-typing methods do only cover polymorphisms that have empirically been screened as important. With the growing information coming in from HLA sequencing, new allelic variants are continuously being discovered which in part may be recognized as foreign. Variations also become evident when the donor and the recipient have a different sex. In summary, the severity of immune reactions such as GVHD depends on the type and degree of molecularly defined protein differences between the patient and the donor that are presented by the patient's cells.

The GVT activity has been best studied in CML patients, where the recognition and eradication of residual tumor cells by donor immune cells (CTLs) appears to be essential for inducing long-lasting molecular remission. Further insight into the mechanisms of immune regulation in CML has been gained by the observation that there is an increased risk of relapse following T-cell depletion of the grafts. The risk of relapse is also increased in the absence of GVHD (Goldman et al., Ann. Intem. Med. 1988, 108: 806-814; Horowitz et al., Blood 1990, 75: 555-562.). Moreover, syngeneic twin BMT is much less effective than matched sibling BMT. Together, these findings indicate that T-cell recognition of tumor cells is an essential prerequisite of the therapeutic effect.

When disease reappears after an apparently successful transplantation, complete remission can be achieved by withdrawal of immunosuppressive drugs or, more impressively, by additional donor T lymphocyte infusion. Thus, the GVT effect related to allo-BMT represents the most conclusive evidence that the immune system can cure cancer in humans and it has to be emphasized that the powerful anti-leukemia effect is generated by cytotoxic T cells transferred to the recipient.

Donor T lymphocytes destroy the recurrent leukemia cells by the GVT effect and, presumably, T cells of both the CD4⁺ and CD8⁺ subpopulations in the allograft contribute to this phenomenon. CD4⁺ T cells often have a helper function for antibody- or cell-mediated immune responses and are MHC class II-restricted. CD8⁺ T cells often have a cytotoxic function and are usually MHC class I-restricted. The relevant antigens (tumor-expressed antigens, the recipient's histocompatibility antigens, or both) have not been identified yet and it is the objective of the present invention to identify and define the antigens involved.

It is striking that T cell-depleted grafts are associated with an increased risk of relapse in CML (Goldman et al., Ann. Intem. Med. 1988, 108: 806-814; Horowitz et al., Blood 1990, 75: 555-562). As described above, anti-leukemia effects may be generated by allo-BMT, when donor lymphocyte infusion (DLI) is performed. In this setting, DLI can reinstate durable molecular remission in up to 70% of cases. However, DLI can as well be associated with significant toxicity caused by graft-versus-host responses, which frequently accompany a graft-versus-leukemia effect, with significant mortality from marrow aplasia and/or systemic GVHD occuring in 50-90% of cases (S. MacKinnon, Br. J. Haematol. 2000, 110: 12-17).

To overcome the shortcomings related to the toxicity of the "traditional" allo-BMT protocol, a conditioning model comprising immunosuppression with mycophenolate mofetil (Cellcept®) and cyclosporine in combination with minimally toxic low-dose total-body irradiation has been suggested. However, because of the less rigorous conditioning, a pronounced graft-versus-host response has been observed. Depleting T cells from the transplant prior to infusion may prevent GVHD in this situation. A modified type of transplantation procedure, sometimes called "minitransplant", is currently developed based on these observations. The hazards of graft rejection and a higher relapse rate can be avoided by maintaining only a portion of the T cells in the graft. The positive selection of CD34⁺ cells from peripheral blood preparations provides an approximately 1000-fold reduction of T-cells. These purified CD34⁺ cells containing committed and pluripotent stem cells are suitable for allogeneic transplantation. In CML, the administration of incrementally increasing T-cell doses has been used to partially circumvent the GVHD problem (MacKinnon et al., Blood 1995, 86: 1261-1268) and to increase the GVT effect at the same time.

In summary, the future of allograft approaches will comprise T cell-depleted minitransplants in combination with a moderate post-grafting immunosuppression to control graft rejection and GVHD. This is expected to dramatically reduce the acute toxicities of allografting and, thus, allo-BMT may be performed in previously ineligible patients, largely in an outpatient setting. This future development is expected to facilitate strategies based on allogeneic immunotherapy for the treatment of a variety of human malignancies.

Most research activities related to the immunologic discrimination between self and non-self have focused on the highly polymorphic MHC molecules, in particular HLA molecules in humans and H-2 antigens in mice. However, it has to be kept in mind that in most cases of BMT, the donor has been selected by the criterion that his or her HLA type is closely or perfectly matched to the HLA of the recipient. In the HLA-matched donors, the origin of GVHD and GVT has been supposed to be related to polymorphic molecules different from the HLA. Recent research trying to unpuzzle the molecular origin of GVHD and GVT immune responses in BMT has therefore taken advantage of the specific T lymphocyte-driven reactions, where the CTLs recognize antigen-derived peptides presented by the recipient's HLA class I. These T cells have been isolated and used to identify allo-antigens. It turns out that disparities in polymorphic antigens other than HLA between donor and recipient seem to be relevant for the development of T lymphocyte-driven GVT and GVHD. Thus, the key to understand the GVHD and GVT immune response is to understand which antigens are involved. Research in the field of allogeneic immune responses has considered the aforementioned aspects and is heading to the identification of other important molecules: the so-called "minor" histocompatibilty antigens (mHAgs). The large number of these highly diverse proteins in combination with the complicated and diverse biological function of the antigens has frustrated attempts of a full characterization so far.

By definition, mHAgs are capable of eliciting an immune response (Lewalle et al, Br. J. Haematol. 1996, 92: 587-594) and they are presented to the T-cell immune system as peptides bound to specific HLA molecules. Thus, only T cells can readily recognize them, and it has been suspected that mHAgs play an important role in the induction of CTL reactivity against leukemia and self-antigens after allogeneic BMT. Unfortunately, most of the few mHAgs that have been identified so far are not leukemia-specific and are as well expressed by normal tissues. The relative tissue expression of the known mHAgs has not been determined, due to the lack of available reagents. However, functional analyses using CTLs suggest that many mHAgs have a tissue-restricted distribution and, thus, only certain tissues may be at risk for rejection. Also of interest is the observation that, in BMT, the clinical picture of mHAg-induced GVHD resembles several autoimmune diseases, such as systemic lupus erythematosis and scleroderma, suggesting that the symptoms of chronic GVHD are autoimmune-like.

By definition, mHAgs are encoded outside the HLA region of the human chromosome 6, but are nevertheless capable of eliciting a remarkable immune response. Despite the fact that the mechanism of GVHD is not yet fully elucidated, it is well recognized that donor-derived CTLs specific for patients' mHAgs play an important role in the T lymphocyte-driven cytotoxic reaction against major target organs (including skin, gut, liver, lung and joints) and the resulting manifestation of GVHD which in severe cases may be fatal. While GVHD mechanism seem to be reasonably well investigated, the role of mHAgs in the induction of GVT is less defined. This could be due to the fact that only very few antigens of the complete mHAg spectrum have been identified and analyzed and that the technologies available today lack an effective method to recognize the antigens in a comprehensive manner. number. On the other hand the antigens are prerequisite for isolating and characterizing the CTL clones responsible for mediating a curative or an adverse effect. Thus the curative BMT approach remains an empirical discipline with regard to the specificity of the immune response involved. Patient and transplantor are normally exclusively focused on the outcome of the therapy, which works reasonably very well for a majority of the patients, as outlined above.

In humans, although cumbersome to identify, mHAgs related to the induction of GVHD have been suggested, but their overall number and complexity remains uncertain. Genetic experiments performed in mice indicated many mHAgs, but only a few genes have been identified. In humans, T-lymphocyte clones reactive with specific mHAgs, combined with genetic linkage analysis, have been applied to identify two distinct loci in a single patient, each locus encoding an antigen presented to a T-cell clone by HLA-B7. The technique has been suggested for a rough enumeration of the number of mHAgs in humans that are capable of eliciting T-cell responses in vivo. Whether these T-cell responses correlate with clinical GVHD is not yet clear. (Gubarev et al., Exp. Hematol. 1998, 10: 976-81).

To get a more complete picture of what characteristics qualify a protein to be nominated as a human mHAg, it is helpful to complement information available from the human system with data collected from the mouse system where additional mHAgs have been identified in the past. The human homologues of these proteins turned out to be recognized by human allo-reactive CTLs and the same is true for mice. More and more mHAgs (Table 1 A and B) have therefore been identified as targets of a response, e.g. by using isolated CTL clones that have been derived from patients suffering from GVHD. With the help of the clones it has been possible to analyze the peptide components (HLA-binding peptides) derived from the corresponding mHAgs and the specific T-cell clones involved in their recognition.

The human skin explant model is an approach that has been suggested as an accurate indicator of acute GVHD and might be useful to detect additional mHAg disparities. The model has been used to predict GVHD outcome in 77% of the cases. Other analyses, such as host-reactive T helper cell and CTL precursor frequency analysis, helped to predict the occurrence of acute GVHD after HLA-identical sibling BMT (Dickinson, Transplantation 1998, 66: 857-63).

Analysis of T-cell receptor alpha-chain variable region and T-cell receptor beta-chain variable region repertoires revealed that T-cell receptor usage was skewed at an early period (6-7 weeks) after BMT, suggesting that T cells may have expanded in response to allogeneic antigens, such as mHAgs, and that altered repertoires are eventually normalized by T-cell regeneration via a thymus-dependent pathway in children (Matsutani et al., Br. J. Haematol. 2000, 109: 759-769).

One of the earliest identified mHAgs was the H-Y antigen encoded by the SMCY gene (Meadows et al., Immunity 1997, 6: 273-281; Wang et al., Science 1995, 269: 1588-1590) which may play a role in spermatogenesis. H-Y antigens can lead to rejection of HLA-matched male organ and bone marrow grafts by female recipients, and to a higher incidence of GVHD in female-to-male grafts, particularly if the female donor had been previously pregnant. Meanwhile, DFFRY (Vogt et al., Blood 2000, 95: 1100-1105) and UTY genes have been identified as sources of further H-Y antigens (WO 97/05168, WO0077046).

Additional GVHD-inducing antigens, namely the HA-1, HA-2, H-4, H-5 and H-8 family of proteins have been identified through a retrospective study in recipients with severe GVHD (Mutis et al., Nat. Med. 1999, 5: 839-842).

The HA-1 antigen was identified with the help of HLA-A*0201-restricted CTLs and chemically characterized as a nonapeptide derived from an allele of the KIAA0223 gene. On the cDNA level, the HA-1 locus has two alleles, HA-1H and HA-1R, which differ in two nucleotides, resulting in a single amino-acid substitution (den Haan et al., Science 1998, 279: 1054-1057; Arostequi et al., Tissue Antigens 2000, 56: 69-76). Isolation and sequencing of cosmid DNA encoding the HA-1 peptide sequence revealed that the HA-1 alleles are encoded by two exons and that both sets contain intronic sequences. Genomic DNA-typing with two different primer sets, consisting of allele-specific primers and a common primer, revealed three families consisting of 24 HLA-A*0201-positive individuals that correlated in all cases with the mHAg classification by CTLs and by RT-PCR. In the future, prospective genomic typing for the HA-1 alleles might help to improve donor selection and identify HLA-A*0201-positive recipients with a high risk for HA-1-induced GVHD (Wilke et al., Tissue-Antigens 1998, 52: 312-317; W09905313). The human HA-2 antigen is a nonamer HLA-binding peptide derived from a class I myosin (Goulmy et al., US 5770201).

It has been noted that the expression of mHAgs HA-1 and HA-2 is primarily restricted to haemopoietic tissues, including leukemia cells and leukemia cell precursors (Mutis et al., Blood 1999, 93, 2336-2341). They are not expressed on fibroblasts, keratinocytes or liver cells. This may explain why CTLs specific for mHAgs HA-1 and HA-2 mediate HLA-A*0201-restricted killing of donor-derived haemopoietic cells.

HB-1 was described as another mHAg that elicited donor-derived CTL reactivity in a B cell ALL (B-ALL) patient treated by HLA-matched BMT. The HB-1 gene-encoded peptide EEKRGSLHVW was recognized by the CTL in association with HLA-B44 (Dolstra et al., J. Exp. Med. 1999, 189: 301-308). Further analysis revealed that a polymorphism in the HB-1 gene generates a single amino-acid exchange from His to Tyr at position 8 within this peptide. This amino-acid substitution was critical for recognition by HB-1-specific CTLs. It has been proposed that the restricted expression of the polymorphic HB-1 antigen by B-ALL cells and the ability to generate HB-1-specific CTLs in vitro using peptide-loaded dendritic cells may provide the opportunity to specifically target the immune system to B-ALL cells without the risk of evoking GVHD.

Another antigen that has been correlated with GVHD is CD31. Direct sequencing of various CD31 cDNAs revealed the presence of a single amino acid change in position 125 of the protein. No other polymorphism has been shown besides these two alleles (Behar at al., N. Engl. J. Med. 1996, 334: 286-291). The corresponding HLA-presented epitopes correlated well with single amino-acid changes recognized by the CTLs.

The findings described above with HA-1, HA-2 and the other mHAgs suggest that specific T cells could selectively attack tumor cells in vivo and discriminate between mHAgs expressed by haemopoietic stem cells and fibroblasts, killing only the former. Interestingly, complete remission could be induced in a patient treated with donor T cells that had been rendered 'leukemia-reactive' in vitro (Falkenburg et al., Blood 1999, 94: 1201-1208). However, the molecular basis for the discrimination between GVHD- and GVT-inducing antigens remains unknown and there is currently no straightforward approach which allows the identification of mHAg-derived HLA-binding peptides and their correlation with proteins for which the biochemical structure is known. Furthermore, little is known about the function of the proteins or the number of proteins that might have to be considered as mHAgs. By studying the frequency of mHAg gene mutations and the number of mHAg differences between strains of mice it has been estimated that the total number of mHAgs might be in the range of 430 to 720 genes. However, it has to be acknowledged that some of these studies have been performed with skin-graft rejection models which are extremely sensitive due to the presentation of mHAg peptides by skin dendritic cells. Dendritic cells derived from other organs such as the hematopoetic system might present antigens differently, hence a much lower number of mHAgs would result. Estimations based on this type of approach have given numbers in the range of 80 different proteins.

In a study aimed at the identification of target antigens for the GVT response to leukemia cells, Clave et al. (J. Immunother. 1999, 22: 1-6) detected polymorphism of proteinase 3, a primary granule protein over-expressed in myeloid leukemia. The study was carried out in 10 patients with hematological diseases and their HLA-identical marrow donors. The enzyme is expressed in cells of the myeloid lineage but is over-expressed in myeloid leukemia, including CML (Molldrem et al., Blood 1997, 90: 2529-2534; Dengler et al., Brit. J. Haematol. 1995, 89: 250-257), and CTLs specific for PR1, a proteinase 3-derived peptide, efficiently lyse CML cells (Molldrem et al., Blood 1997 90: 2529-2534). Circulating PR1-specific CTLs have been detected in a number of CML patients, including those having been treated by allo-BMT, and their presence is correlated with good prognosis (Molldrem et al., Nat. Med. 2000, 6: 1018-1023). By polymerase chain reaction (PCR) single strand conformation polymorphism assay, followed by direct sequencing of the PCR products, seven single nucleotide polymorphisms have been found. One of them encodes for either an isoleucine or a valine at position 119 of the amino acid sequence. Peptides that span the polymorphic site, at amino acids 115-124, were shown to bind in vitro to the HLA-A2 molecule. 23 HLA-A2 patients with myeloid leukemia and their HLA-identical donors have been screened for the polymorphism. No relapse was found in the group of 4 evaluable patients who possessed at least one allele that was absent in their donor, whereas 7 of the 15 remaining evaluable patients relapsed. These data support the possibility that T-cell responses to allelic differences of proteinase 3 could be used as a basis for designing leukemia-specific adoptive T-cell therapy in acute and chronic myeloid leukemia.

In summary, the current approaches for the identification of new mHAg candidate proteins are mainly based on the identification of isolated CTLs and/or HLA-eluted peptides mainly related to GVHD and only occasionally these antigens have been defined through the corresponding proteins.

A list of mHAgs collected from the literature for mice and men is given in Table 1A. No clear-cut strategy has been brought up so far to make mHAg identification more predictable and faster, hence candidate genes of new human mHAgs characterized via available approaches appear only slowly. There is no technique in the known art that would allow an easier access to additional new mHAgs or candidate proteins. They are probably a diverse and elusive group of fragments of molecules which are derived from proteins participating in various cellular housekeeping functions and, in general, the locations of the encoding loci are unknown. Some of the mHAgs appear to be widely expressed in various tissues throughout the body, whereas others show limited tissue distribution. Their analysis is so far not related to anti-leukemia reactivity but almost exclusively associated with life-threatening GVHD. This is because the currently available technologies are strongly biased to identify mHAgs related to GVHD and it is only sporadically that an mHAg has been suggested for prevention of disease, e.g., induction of GVT responses.

Interestingly, all studies for characterization of the GVHD and GVT immune responses resulted in the surprising observation that the antigens involved are mHAgs with a limited polymorphism that arises through rare DNA mutations leading to single amino-acid changes in the corresponding protein sequence. It is furthermore apparent that these amino-acid changes have to be presented via HLA class I in order to cause GVT responses or GVHD. Several allogeneic T-cell clones being involved in GVHD have been isolated and have been shown to specifically recognize single amino-acid exchanges of the HLA-presented peptides.

Leukemia, lymphoma and myeloma are cancers that originate in the bone marrow and lymphatic tissues. The diseases result from an acquired (not inherited) genetic injury to the DNA of a single cell derived from the hematopoetic system, which converts into the leukemic clone and then multiplies continuously. This unrestricted proliferation interferes with the body's production of healthy blood cells and makes the body unable to perform the essential physiological functions and protect itself against infections.

In the United States, an estimated 107,900 people have been diagnosed with leukemia, lymphoma and myeloma in 1999 and this accounts for 11 percent of cancer cases diagnosed in the U.S. each year. An estimated total of 632,000 Americans are presently living with leukemia, lymphoma and myeloma. The numbers available for Europe are very similar to what is seen in the U.S., where leukemia, lymphoma and myeloma will kill an estimated 60,500 persons each year. Leukemia and lymphomas are the leading fatal cancers in young women and young men under 35.

In the early chronic phase, CML is characterized by a t(9;22) chromosomal translocation (Philadelphia Chromosome, Ph) that creates the *bcr-abl* oncogene. The product of the chimeric gene is a constitutively active tyrosine kinase which is the target for synthetic inhibitors such as STI571. Compared with patients affected by other tumor types, CML patients still possess a relatively intact immune systems in the chronic phase, and it is now increasingly apparent that bcr-abl peptides, along with other unknown disease-associated antigens, can be presented by HLA molecules and recognized by T cells. Direct immunization of patients with fusion proteins has been used to explore the potential of allo-BMT in the clinical setting to boost naturally occurring or transplant-induced immunity. An initial trial has shown such vaccination to be safe; some patients exhibited specific T-cell responses to the immunizing antigen (Pinilla-Ibarz et al., Blood 2000, 95: 1781-1787).

Acute leukemia continues to present a formidable challenge for which the treatments (chemotherapy, BMT and radiation) are tailored according to the risk profiles that are deducted from the cytogenetic profile of the patients. BMT is reserved to patients which do poorly with chemotherapy alone. In order to make progress in terms of curing these devastating diseases, the understanding of the leukemia biology at the clinical, cellular and molecular levels, and especially the molecular definition of disease related antigens for the design of immunotherapeutic strategies, is a primary aim to allow eradication of the leukemic clone involved.

Renal cell carcinoma (RCC) represents approximately 5% of all cancer deaths. At the time of presentation, over 50% of the patients have already developed locally advanced metastatic disease with 5-year survival rates of less than 20%. Numerous studies with many different treatment modalities have resulted in only minor advances. No single agent or combination therapy has consistently shown a response proportion of 20% or higher. Interleukin-2 and interferon-alpha-based therapies are most commonly used to treat advanced disease, demonstrating low but reproducible response proportions in the 10% to 20% range, with durable responses of 5% or less (Nanus, Curr. Oncol. Rep. 2000, 2: 417-22).

Since RCC is susceptible to cytokine or interferon-based immunotherapy, there are good reasons to believe that specific T cells are involved in eliminating autologous tumor cells (Schendel et al., J. Mol. Med. 1997, 75: 400-413). Recently, tumor-specific T cells have been isolated from lymphocytes infiltrating human RCC by the IFN-gamma capture assay (Becker et al., Nat. Med. 2001, 7: 1159-1162). However, due to the incomplete knowledge of RCC antigens and their corresponding class I-presented peptides, the role of T cells in interferon-alpha immunotherapies of RCC is poorly understood.

The identification of new antigens that may be useful for the immunotherapy of RCC remains a high priority. Methods for the identification of antigens that are relevant in RCC have been described on the level of transcription as well as on the protein expression level. Comparison of the proteases of non-cancerous kidney and RCC by two-dimensional gel electrophoresis (2-DE) and silver staining revealed markedly different protein patterns (approximately 800 spots in RCCs versus approximately 1400 spots in normal kidney). 2-DE immunoblotting revealed five RCC-specific spots, reproducibly reactive with sera from RCC patients but not with those from healthy donors. Two of these antigens were isolated by preparative 2-DE and were identified as smooth muscle protein 22-alpha (SM22-alpha), an actin-binding protein of unknown function predominantly expressed in smooth muscle cells. In situ hybridization revealed that SM22-alpha is not expressed in the malignant cells but in mesenchymal cells of the tumor stroma. The second antigen represents carbonic anhydrase I, an isoform usually not expressed in kidney. Interestingly, a different isoform (CAXII) has previously been identified by serological expression cloning as an antigen over-expressed in some RCCs. Antibodies to recombinant CAI or SM22-alpha were detected in sera from 3 of 11 or 5 of 11 RCC patients, respectively, whereas sera from 13 healthy individuals did not react. In conclusion, serological methods may be a useful tool in proteome analysis and may contribute to the identification of renal tumor-associated antigens. However, there is still the need for identification of relevant RCC antigens and especially the relevance of these antigens for vaccination against cancer has to be shown.

This situation in RCC mirrors the status for various other solid tumor diseases, where meanwhile a total number of 60 different protein antigens corresponding to 178 epitopes have been defined. A great deal of these antigens and the corresponding T-cells epitopes have been used in diverse vaccination protocols, adjuvant formulations and cell-based presentation systems to improve the immune response. However independent of the protocol and antigen involved the results obtained have been quite comparable: T cell activation without clinical response. Thus vaccines may one day play an important role in therapy, however immune responses observed in clinical trials have not been translated into significant survival benefit so fare.

The immunotherapeutic potential of allo-BMT, as described above for leukemia, has meanwhile been explored with other diseases, such as enzyme-deficiency disorders, Fanconi's anemia, and thalassemia major. This has mainly been possible through the growing clinical experience and it became evident that the allo-BMT approach may as well be used for the immunotherapy of metastatic solid tumors such as RCC. In a recently published study (Childs et al., N. Engl. J. Med. 2000, 343: 750-758), nonmyeloablative allogeneic stem-cell transplantation has been applied to induce sustained regression of metastatic RCC in patients who had failed conventional cytokine therapy. Ten of 19 patients (53 percent) enrolled in the study had a measurable response, and 3 patients had complete, sustained responses. Although these results are promising and should encourage similar treatment strategies for use against other metastatic tumors, the procedure used by Childs et al. is not entirely satisfactory because the regression of the tumor in some patients was accompanied by severe GVHD. Two patients died after receiving this treatment. Although the procedure needs further refinement to minimize complications and improve its efficacy, the proof of principle is already at hand: allogeneic T cells can eradicate renal cancer cells, and donor lymphocytes can survive in the host after nonmyeloablative conditioning. The more general message of this study is, however, that allo-BMT is extensible to treatment of solid tumor. Future progress in the therapy of solid tumors, such as RCC, will depend on the establishment of safer and better-controlled anti-tumor immunotherapy that is free of GVHD responses.

The transplantation of bone marrow shares many aspects with the transplantation of solid organs, such as kidney, heart, liver and lung, where organ transfer also remains the treatment of choice for several disease states. Although recent progress with regard to the development of better immunosupressive drugs has improved the short-term survival of allografts, immunological rejection is still an obstacle to long-term survival. Substantial evidence has accumulated indicating that mis-matches in the donor organ and the patient, namely mHAg mis-matches, affect solid organ survival and promote GVHD. Thus, patients who experience long-term graft survival still have a poor prognosis with only 40% of kidneys surviving more than ten years. The role of mHAg mis-matches in the eventual loss of these grafts is unknown and subject of discussion. Thus, organ transplantation and especially renal transplantation is another application that might benefit from allo-BMT and the identification of allele-specific allo-antigens.

Cosimi et al. at the Massachusetts General Hospital have discussed that transplanted kidneys may be implanted after giving recipients bone marrow from the donor, thereby creating a state of T cell chimerism in the patients (N. Engl. J. Med. 2002, 346:2089-92). In theory, the donor lymphocytes will migrate to the thymus, along with antigen from the donor organ, and induce tolerance to the new kidney. In an even more future-oriented scenario, the process of tolerance induction may be supported by vaccination with the appropriate renal allo-antigens that will have been identified, prepared and delivered according to the present invention. An intermediate form of the future treatment may comprise allo-BMT transplantation in addition to renal transplantation, wherein both grafts are derived from the same donor.

Single nucleotide polymorphism (SNP) was defined as a mismatch between two DNA sequences (Stoneking, Nature 2001, 409: 821-822) and refers to a variation in the sequence of a gene in the genome of a population that arises as the result of a single base change, such as an insertion, deletion or, preferentially, as used herein, a change in a single base leading to an amino-acid change. SNPs are manifested as different mendelian alleles for a gene. A locus is the site at which divergence occurs.

The nucleotide base change, as understood in the present invention, relates to the coding portion of the genome and results in the incorporation of an alternative amino acid into the corresponding protein. The amino-acid exchange may affect posttranslational modifications of said amino acid, for example, glycosylation. Thus, SNP refers to the occurrence of two or more genetically determined alternative sequences or alleles in a population and can be manifested or detected as differences in nucleic acid sequences, gene expression (including, for example, transcription, processing, translation, transport, protein processing, trafficking), DNA synthesis, expressed proteins, other gene products or products of biochemical pathways or post-translational modifications manifested among members of a population.

SNPs as presented in the art have mainly been discussed in relation to altered protein function. However, finding functionally relevant SNPs among 3 billion DNA bases and distinguishing them from the few million SNPs with no known useful function is a big task and one of the major challenges of the post-genome research. While the generation of functionally relevant SNP information is progressing continuously but slowly, the overall information regarding the total number of human SNP and the assignment to individual genes is available from various databases such as dbSNP, CGAP, HGBASE, JST and Go!Poly etc. which collect and exploit data of SNPs established in the U.S., European countries, Japan and China. Companies like Celera are creating and selling tools to identify SNPs and will have a SNP-based linkage map of the human genome created by the end of the year 2002. The Celera-SNP database is based on DNA sequences from 40 or 50 individuals and uses that information to track down the SNPs. Access to these data will allow assignment to a specific gene and will allow prediction of the SNPs relevant to disease in the future.

The presentation of antigens is based on two distinct pathways, an exogenous HLA class II and an endogenous HLA class I pathway. The class I molecules are encoded by the HLA-A, B and C loci and are considered to activate primarily CD8⁺ cytotoxic T cells. The HLA class II molecules are encoded by the DR, DP and DQ loci and primarily activate CD4⁺ T cells, both helper cells and cytotoxic cells.

A "normal" individual has six HLA class I molecules, usually two from each of the three groups A, B and C. Correspondingly, all individuals have their own selection of HLA class II molecules, again two from each of the three groups DP, DQ and DR. Each of the groups A, B, C and DP, DQ and DR are again divided into several subgroups. All the gene products are highly polymorph. Different individuals thus express distinct HLA molecules that differ from those of other individuals. This is the reason for the difficulties in finding HLA-matched organ donors in transplantations. The significance of the genetic variation of the HLA molecules in immunobiology is reflected by their role as immune-response genes. Through their peptide binding capacity, the presence or absence of certain HLA molecules governs the capacity of an individual to respond to peptide epitopes. As a consequence, HLA molecules determine resistance or susceptibility to diseases.

HLA class II expression is restricted to APCs. This is consistent with the functions of helper T lymphocytes, which are locally activated wherever they encounter APCs (macrophages, dendritic cells, or B cells) that have internalized and processed antigens produced by pathogenic organisms.

MHC (HLA) class I molecules are expressed on every nucleated cell of the body and are part of the main immunological defense mechanism against viruses and other intracellular pathogens. They are assembled as heterodimers of a class I chain (HLA-A, -B, -C) and soluble β₂-microglobulin that bind peptides generated by antigen processing inside the cell and transport these peptides to the cell surface where they can be recognized by CTLs via the T-cell receptor.

The class I and class II pathways are not fully distinct. For example, it is known that dendritic cells, and to some extend macrophages, are capable of endocytosing (pinocytosing) extracellular proteins and subsequently present them in the context of MHC class I. It has been demonstrated that exogenous antigens are also capable of entering the class I pathway (Rock, et al., Immunol. Today, 1996, 17:131-137). This can be achieved by using specialized administration routes, e.g. by coupling the antigens to iron oxide beads, and seems to be a central mechanism, because of the importance of a concomitant expression of both MHC class I and class II on the same APC to elicit a three-cell type cluster. This three-cell type cluster of interaction has been proposed by Mitchison et al. (Eur. J. Immunol., 1987, 17:1579-83.) and later by other authors. They showed the importance of concomitant presentation of class I and class II epitopes on the same APC. According to the recently described mechanism for CTL activation (Lanzavecchia, Nature 1998, 393: 413-414, Matzinger, Nat. Med. 1999: 616-617), professional APCs presenting antigen via MHC class II are recognized by T helper cells. This results in an activation of the APC (mediated by interaction of CD40 ligand on the T helper cell and CD40 on the APC) and enables the APC to directly stimulate CTLs which are thereby activated.

It has previously been demonstrated that insertion of a foreign MHC class II-restricted T helper cell epitope into a self-antigen results in the generation of an antigen capable of inducing strong cross-reactive antibody responses directed against the non-modified self-antigen (cf. applicant's WO 95/05849). It was shown that the auto-antibody induction is caused by specific T cell help induced by the inserted foreign epitope and it is expected that modified self-antigens - with the aid of appropriate adjuvant - are capable to induce a strong CTL response against MHC class I-restricted self-epitopes. Hence the technology described in WO 95/05849 can be adapted to also provide vaccination strategies against intracellular and other cell-associated antigens which have epitopes presented in the context of MHC.

The HLA-binding motifs for the most frequently found class I alleles (HLA-A1, -A2, - A3, -A11, -A24, -B7) as well as those for several major class II molecules have been reported (Rammensee et al., Immunogenet. 1995; 41: 178-228; Ruppert et al., Cell 1993; 74: 929-937; Kubo et al., J. Immunol. 1994; 152: 3913-3924, Kondo et al., Immunogenet. 1997; 45: 249-258; Southwood et al., J. Immuno/. 1998, 160: 3363-3373; Geluk et al., J. Immunol. 1994; 152: 5742-5748). A binding motif is characterized by the requirement for amino acids of a certain type, for instance those carrying large and hydrophobic or positively charged side groups, in definite positions of the peptide, so that a narrow fit with the pockets of the HLA-binding groove is achieved. The result of this, taken together with the peptide-length restriction of 8 to 10 amino acids within the binding groove, is that it is quite unlikely that a peptide binding to one type of HLA class I molecules will also bind to another type. Thus, for example, it may very well be that the peptide-binding motif for the HLA-A1 and HLA-A2 subgroups, which both belong to the class I gender, are as different as the motifs for the HLA-A1 and HLA-B1 molecules.

For the same reasons, it is unlikely that exactly the same sequence of amino acids will be located in the binding groove of the different class II molecules. In the case of HLA class II molecules, the binding sequences of peptides may be longer, and it has been found that they usually contain 10 to 16 amino acids, some of which, at one or both terminals, are not a part of the binding motif for the HLA groove.

An overlap of the different peptide-binding motifs of several HLA class I and class II molecules may occur. Peptides that have an overlap in the binding sequences for at least two different HLA molecules are said to contain "nested T-cell epitopes". The various epitopes contained in a "nested epitope peptide" may be formed by processing of the peptide by APCs and, thereafter, may be presented to T cells via different HLA molecules. The individual variety of HLA molecules in humans makes peptides containing nested epitopes more useful as general vaccines than peptides that are only capable of binding to one type of HLA molecule.

Effective vaccination of an individual can only be achieved if at least one type of HLA class I and/or class II molecule in the patient can bind a vaccine peptide either in its full length or after processing by the patient's own APC.

The usefulness of a peptide as a general vaccine for the majority of the population increases with the number of different HLA molecules it can bind to, either in its full length or after processing by APCs. By identifying sets of antigen-associated peptides that bear these motifs and that bind to the various HLA molecules, one could offer coverage to the majority of the human population (>80%) for developing T-cell epitope-based immunotherapy of tumors.

In order to use peptides derived from a protein encoded by allelic versions of a gene as vaccines or anticancer agents to generate anti-tumor CD4⁺ and/or CD8⁺ T cells, it is necessary to investigate the mutant protein in question and identify peptides that are capable, possibly after processing to shorter peptides by the APC, to stimulate T cells.

In general, tumors are very heterogeneous with respect to genetic alterations found in the tumor cells. This implies that both the potential therapeutic effect and prophylactic strength of a cancer vaccine will increase with the number of targets that the vaccine is able to elicit T cell immunity against. A multiple target vaccine will also reduce the risk of new tumor formation by treatment escape variants from the primary tumor.

It is explicit that the presentation of T-cell epitopes (peptide fragments) on HLA class I molecules is not only a feature for recognition of cells but as well a prerequisite to survey and kill tumor cells and other cells carrying allo-antigens by specific T cells.

In more detail, for epitope generation the corresponding proteins must be cleaved by proteasomes into peptides with specific C-terminal amino acids. Cleaved fragments must be transported by so-called TAP molecules (transporters associated with antigen processing) into the endoplasmic reticulum where HLA binding occurs when fragments contain proper HLA-binding motifs. Thus, it is a prerequisite that candidate target peptides for immunotherapy, containing the proper motif for HLA binding, are cleaved by the proteasome at the right C-terminal amino acid. To determine appropriate proteasome cleavage of candidate proteins experimentally, in vitro cleavage assays (4-24 hrs) using purified cellular 20S proteasomes have been developed and combined with peptide analysis by mass spectrometry. The results of such experiments indicate that combining proteasome digestion with binding studies is useful to define candidate target peptides for immunotherapy and the expert might benefit from PAProC (hftp://www.paproc.de), a prediction algorithm developed to assess the general cleavability of disease-linked proteins.

Numerous CTL epitopes have been identified to date and, as pointed out earlier, they have common motifs with a preferred length and amino acid composition at certain positions. The predictable motifs have been used to design computer programs that translate the amino-acid sequence of a given protein into CTL epitopes. It is particularly useful for immunologists working on the prediction of MHC class I ligands and CTL epitopes to use SYFPEITHI at http://syfpeithi.bmi-heidelberg.com) or alternatively BIMAS at http://bimas.dcrt.nih.gov/molbio/hla bind/.

In-vivo investigations of T-cell responses may be limited by the difficulty of identifying antigen-specific T cells among a plethora of non-specific cells. This difficulty is largely due to the low affinity of interactions between the T-cell receptor (TCR) and its natural ligand, the HLA-peptide complex. Multimerization of HLA-peptide complexes known as tetramer technology can overcome these technical problems by increasing the overall affinity of the TCR-HLA interaction to an extent that such complexes can be used as reagents for epitope-specific detection of T cells.

The generation of soluble HLA class II-peptide complexes is not so well established, perhaps due to the more complex structure of the class II peptide-binding groove. In vivo expression and refolding in insect cells as well as the use of covalently linked peptide epitopes are promising approaches to overcome these technical problems.

Tetramer staining is highly epitope-specific, and even very small populations can be identified directly ex vivo with this technique. In addition to the precise frequency analysis, these reagents allow detailed phenotypic and functional characterization of epitope-specific T cell populations at the single cell level, e.g. the expression of surface markers, determination of cytokine profiles, and TCR repertoire analyses. The binding kinetics of tetrameric HLA-peptide complexes appears to be a useful tool to measure relative affinities of epitope-specific T cells for their ligand. In addition to the basic insights and quantification of T cell-mediated immune responses that have been made possible with tetramers, the technology may be used in allo-BMT for elimination of autoreactive T cells involved in GVHD.

The theoretical prediction of a CTL epitope with the help of the known prediction-programs can, however, only be performed for known antigens. Thus, the identification of proteins carrying the epitopes recognized by protective T cells is a central issue in vaccine development. If T-cell recognized peptide has been generated by eluted from class 1 molecules the further development towards a peptide-based vaccine can be a time consuming project. It is the objective of this invention to close said gap with respect to prediction of cancer- and GVHD-related protein antigens.

WO 00/42181 discloses minor histocompatibility antigens and their use in diagnosis and treatment of tumor including a kit for detecting a single amino acid mismatched allelic variant relating to a SNP comprising non-overlapping primers.

The special purpose of this patent application is to identify and characterize allo-antigens, such as mHAgs, and to determine their role in GVT, GVHD and rejection of solid organ grafts.

To this end, we develop novel approaches to identify and characterize allo-antigens and the immune response to them. Other relevant topics include, but are not limited to, the identification of the genetic loci and alleles that encode allo-antigens and mHAgs and the improvement of techniques to determine the total number of mHAg antigens/mHAg loci and alleles involved. The identification of immunodominant mHAg antigens/mHAgs includes the evaluation of antigenic peptides and their role in therapy and disease as well as their relevance with respect to relative abundance, affinity of peptide for HLAand induction of cytotoxic T-cell action. Also, in vivo correlates of in vitro peptide immune function could be studied to determine if the immunodominant peptides identified in vitro function similarly in vivo. The relative tissue expression of various allo-antigens or mHAgs and the impact of their differential tissue distribution on transplant rejection may be studied as well.

This comprehensive information will be used to identify approaches to enhance GVT responses as observed following BMT, to improve graft survival. The scope of research to support this patent application includes, but is not limited to, the following broad areas of interest and specific examples of investigations. The examples are not meant to be directive, but illustrative of areas that remain to be further explored.

### Summary of the invention

The invention involves the surprising discovery of various genes coding single nucleotide polymorphism (SNPs), some previously known and the remainder previously unknown, which are expressed in individuals that have cancer. A more general aspect of the present invention, describes for the first time a generalized method to define a group of functionally heterogeneous protein antigens related to the induction of GVHD and GVT immune responses, which have so far been summarized as mHAgs. Said mHAgs belong to the group of allo-antigens which had so far not been accessible to a generalized scheme of identification: According to the present disclosure a universal method has been established which helps to dissect the group of allo-antigens into those which are responsible for generation of GVHD and those that confer GVT. It is furthermore disclosed that single amino acid mismatched allelic variants arising from coding SNPs in genes are presented by cancer cells and that said antigens are recognized by (donor) allo-reactive T cells in the context of HLA molecules leading to the destruction of said cell.

Coding SNP in a gene stand for a single amino-acid exchange in a protein and are inherited and unique for a given individual. Transplanted patients or those that have been scheduled for bone marrow transplantion or stem cell transfer from a non-syngen donor for therapeutic reasons represent a chimeric status with respect to the gene products presenting a single amino-acid exchange coded by a SNP and the allo-reactive T cells specific for said T cell epitope. As a consequence, gene products are recognized by the donor-derived (host's) immune system and, as a result form a basis for diagnosis, monitoring and therapy.

Briefly stated, the present invention delivers a new concept with respect to the broad definition of allo-antigens that drive GVT and or GVHD and a main object of the invention is to obtain peptides corresponding to peptide fragments of proteins with a single amino acid exchange produced and presented by cancer cells which can be used to stimulate T cells. It is noteworthy to point out that the molecular basis of the single amino acid substitution in this invention is based on an allelic difference, involving a single conservative amino acid substitution in a normal protein, and it is part of the invention to determine whether an individual in need of BMT or already subjected to BMT carries said amino acid exchange in a given protein or not. It is furthermore an aspect of the present invention to determine whether said protein is mainly and or selectively expressed in cells representing cancerous tissues or normal tissues.

A further purpose of the disclosure is to determine and, when needed, isolate donor-derived T cells that recognize said single amino-acid differences presented on target cells. Said T cells may be found to be specific for cancer cells and used therapeutically, or may be found to be specific for omnipresent protein antigens and therefore may be identified as stimulators of GVHD.

Some of the peptides defined according to this disclosure are found to have a broad tissue distribution and are inducers of GVHD thus may be used to induce immunological tolerance, whereas those defined as exclusively expressed will stimulate GVT responses and are useful for specific immunotherapy of disease.

Another main object of the disclosure is to develop a therapy for cancers based on T-cell immunity, which may be induced in patients by allo-BMT and/or by stimulating their own T cells or donors T cells either in vivo or in vitro with the peptides according to the disclosure in order to induce a cytotoxic T cell response and overcome immunological tolerance.

A further main object of the disclosure is to develop a vaccine to prevent the onset of or to eradicate cancers based solely or partially on peptides corresponding to peptides of the present disclosure which can be done by generating and activating the T cells cytotoxic activity against cells harboring the single nucleotide-altered genes and the single amino acid-substituted peptides.

Another aspect of vaccination relates to the use of peptides corresponding to GVHD-inducing peptides of the present invention for induction of tolerance with regard to T cells recognizing cells harboring the single nucleotide-altered genes and the single amino acid-substituted peptides.

Another main object of the disclosure is to design an anticancer treatment or prophylaxis specifically adapted to a human individual in need of such treatment or prophylaxis, which comprises administering at least one peptide or more peptides according to this disclosure.

According to the disclosure, normal tissue expression of single amino acid-substituted proteins relates to prevention and treatment of GVHD, whereas selected organ- and/or cancer cell-specific or disease related expression relates to a therapeutic T-cell responses. According to this aspect of the disclosure, it is particularly useful to determine differences concerning the amino acid-substituted protein in a donor-derived specimen compared to a recipient-derived specimen. Donor-recipient differences analyzed according to this invention and correlated with normal tissue expression deliver a strong indicator for GVHD following BMT. On the other hand, donor-recipient differences analyzed according to this disclosure and correlated with disease related expression in cancerous tissues, organs and cells delivers a strong predictor for beneficial GVT reactivity following allo-BMT.

The group of peptides corresponding to fragments of single amino acid-substituted proteins arising from genes coding SNPs in cancer cells, identified according to the present disclosure, can not only be used to generate isolated T cells. Moreover, said peptides can be used for the induction of T cell reactivity in the patient killing cancer cells harboring a gene with a SNP as described above.

The amino acid mismatched peptides defined according to this disclosure are at least 8 amino acids long and correspond, either in their full length or after processing by APCs, to the SNP-correlated gene products, or fragments thereof, produced by a disease related cell in a human patient afflicted with cancer. A peptide according to this disclosure is characterized by that it a) is at least 8 amino acids long and is a fragment of a single amino acid-substituted protein arising from said coding SNP in a gene of a cancer cell; b) comprises the single amino-acid substitution as part of the protein sequence encoded by said gene; and c) induces, either in its full length or after processing by APCs, T-cell responses.

The peptides of this invention contain preferably 8 to 25, 9 to 20, 9 to 16, 8 to 12 or 20 to 25 amino acids. They may for instance contain 9, 12, 13, 16 or 21 amino acids.

It is most preferred that the peptides of the present disclosure are at least 9 amino acids long, for instance 9 to 18 amino acids long, but, due to the intrinsic processing possibility of the APC, longer peptides are as well suited to create HLA-complexed peptides. Thus, the whole amino-acid sequence of a protein carrying one or more single amino-acid substitutions may be used as peptide or protein according to the present disclosure, if it comprises 8 amino acids or more. It is important to mention that a DNA molecule coding such a polypeptide could as well be used to express and present the peptide.

In order to determine whether peptides identified according to the present disclosure are usable in the compositions and methods according to the present disclosure the following steps should be performed:
1) Identify genes specific for cancer cell which are selectively expressed or over expressed in said cell. Analyze whether said genes are polymorph with respect to allele-specific single nucleotide coding for an amino acid exchange in the gene product.
   Determine according to the selective or broad expression profile of a gene whether the peptides thus identified are usful in the treatment or prophylaxis of cancer or the prevention of GVHD.
2) Determining whether the polymorph part of the peptid representing the single amino acid mismatched, either in their full length or as shorter fragments fit with the definition of a HLA class 1 T-cell epitopes, required to stimulate T cells.
   Optionally, a further step may be added as follows:
3) Determine peptides containing nested epitopes for different major HLA class I and/or HLA class II molecules and using these peptides for stimulation or inhibition of T cells.

In summary, it is an essential aspect of the disclosure to identify SNP-encoded peptides which are fragments of proteins and which are T-cell epitopes, or derivatives thereof, having functional or immunological properties intrinsically related to allo-reactivity, wherein the immunological difference is determined by one or more single amino-acid changes coded by a SNP within said T-cell epitope. It is furthermore an aspect of the present disclosure that with established standard methods known to the expert it is now possible to identify new relevant allo-antigens responsible for induction of allo-immune response as associated with mHAgs, GVHD antigens, GVT antigens and host-versus-graft antigens. On the basis of the methods and peptides described herein, genetic probes or primers may be produced which can be used to screen for the allo-antigens, especially the SNPs, in the gene encoding the single amino acid allelic version of the protein. Furthermore, the disclosure provides a method for determining a subjects allelic status with respect to a polymorph gene through (a) obtaining an appropriate nucleic acid sample from the subject and (b) determining whether the nucleic acid sample from step (a) is, or is derived from, a nucleic acid which encodes a SNP-defined allo-antigen so as to thereby determine whether a subject carries one or the other allelic version of the SNP-defined allo-antigen gene.

This disclosure also provides oligonucleotides of at least 15 nucleotides capable of specifically hybridizing with a sequence of nucleotides present within a nucleic acid which encodes one allelic version of the SNP leading to the single amino-acid exchange, and oligonucleotides of at least 15 nucleotides capable of specifically hybridizing with a sequence of nucleotides present within the nucleic acid which (the other allelic version) encodes the other amino acid exchange without hybridizing to a nucleic acid which encodes the other allele.

The disclosure further provides a method for determining whether a subject might benefit from allo-BMT for cancer therapy which comprises (a) obtaining an appropriate nucleic acid sample from the subject; and (b) determining whether the nucleic acid sample from step (a) is, or is derived from, a nucleic acid which encodes one and/or other allelic protein version so as to thereby determine whether a subject has a predisposition for GVHD or GVT response.

A specific aspect of the disclosure relates to a selective tissue expression and distribution of said SNP-encoded allelic protein variant and the peptides described herein can be used to produce selective therapeutic agents as required to combat diseases such as cancer and/or GVHD. Enhancing the immune response of patient, or indeed donor, with allo-antigen identified according to this invention might contribute significantly to improved immunotherapies. To provide protective immunity in a patient, it might be necessary to administer an effective amount of one or more of the polypeptides described in the invention together with an adjuvant. The expert in the field will select this immune response amplifier according to the immunization protocol chosen.

In addition, the present disclosure provides pharmaceutical compositions that comprise at least one of the inventive polypeptides, or a DNA molecule encoding such a polypeptide, and a physiologically acceptable carrier. The disclosure also provides vaccines comprising at least one of the invention's polypeptides and a non-specific immune response amplifier, together with vaccines comprising at least one DNA sequence encoding such polypeptides and a non-specific immune response amplifier.

The disclosure further provides a method for treating a subject who has a predisposition to GVHD or GVT responses by either introducing the isolated nucleic acid encoding one and/or the other allelic protein version or an effective amount of one and/or other allelic protein version and a pharmaceutically acceptable carrier, so as to thereby treat the subject who is susceptible to GVHD and or cancer.

This disclosure also provides a method for determining whether a subject has residual cancer after a previous allogeneic stem cell or bone marrow transfer, which comprises (a) obtaining an appropriate nucleic acid sample from blood cells of the diseased subject, and (b) determining whether the nucleic acid sample from step (a) is, or is derived from, a nucleic acid which encodes the patient inherited own allelic protein version or the donors allelic variant so as to thereby determine whether a subject has residual cancer.

This disclosure further provides a method of treating a subject who has cancer by either introducing the isolated nucleic acid encoding one and/or the other allelic gene version, or the protein itself, in an effective amount.

This disclosure further provides a method for identifying a chemical compound which is capable of suppressing cells unable to regulate themselves in a subject which comprises (a) contacting one and/or the other allelic variant of the protein version with the chemical compound under conditions permitting binding between one and/or the other allelic variant of the protein and the chemical compound, (b) detecting specific binding of the chemical compound to one and/or the other allelic variant of the protein, and (c) determining whether the chemical compound inhibits one and/or the other allelic variant of the protein so as to identify a chemical compound which is capable of suppressing cells unable to regulate themselves.

This disclosure further provides pharmaceutical compositions comprising a chemical compound capable of inhibiting cancer, an antisense molecule capable of inhibiting an isolated nucleic acid encoding one and/or the other allelic variant of the protein, or a purified allelic variant of the protein in an amount effective to treat cancer and if necessary together with an effective pharmaceutical carrier.

This disclosure further provides a method of treating a subject who has cancer, comprising administration of an effective amount of the above-identified pharmaceutical composition.

This disclosure further provides transgenic, non-human mammals, comprising an isolated nucleic acid encoding one and/or the other human allelic version of the protein.

In further aspects of this disclosure, methods and diagnostic kits are provided for detecting one or the other allelic version of the SNP-defined allo-antigen gene in a patient or in a donor. In a first embodiment, the method comprises contacting dermal cells or lymphocytes of a patient or donor with one or more of the above polypeptides and detecting an immune response within the patient's or donor's skin. Indirect methods used to assess lymphocyte activation such as the analysis of proliferation or cytotoxic responses may be used as well. In a second embodiment, the method comprises contacting a biological sample with at least one of the above polypeptides and detecting in the sample the presence of antibodies that bind to the polypeptide(s), thereby detecting allo-reactivity in the biological sample. Suitable biological samples include whole blood, sputum, serum, plasma, saliva, cerebrospinal fluid and urine.

Diagnostic kits comprising one or more of the above polypeptides in combination with an apparatus sufficient to contact the polypeptide with the selected cells of a patient or donor are provided. The present disclosure also provides diagnostic kits comprising one or more of the inventive polypeptides in combination with a detection reagent.

In yet another aspect, the present disclosure provides antibodies, both polyclonal and monoclonal, that bind to the individual allelic versions of the SNP-defined polypeptides, as well as methods for their use in the detection of allo-antigens in diseased individuals or healthy donors.

These and other aspects of the present disclosure will become apparent upon reference to the following detailed description

In summary the disclosure relates to the following items:
(i) A method for providing epitopes of allelic variants of antigenic peptides or proteins from a specific species based upon one or more amino acid exchanges related to coding single nucleotide polymorphism, wherein said method comprises the steps:
   (i) defining a protein or peptide of interest or a subset thereof;
   (ii) screening a database representing at least two or more DNA libraries of said species for said defined peptide or protein or a subset thereof,
   (iii) identifying and selecting amino acid exchanges of allelic peptide/protein variants, an expression product or a fragment thereof which is encoded by a DNA sequence containing at least one single nucleic polymorphism in the coding region,
   (iv) creating 8mer to 25mer epitopes comprising the amino acid residue containing said polymorphism, and
   (v) selecting such epitopes that bind to MHC protein.
(ii) A method of item (i), wherein a chimeric status with respect to the allelic variants was produced or caused by allo-transplanted cells, and recipients cells express and present allelic variants different from the donor.
(iii) A method of item (i),(ii), wherein the antigenic peptide or protein is related to a disease or disorder and said disease or disorder requires allo-transplantation.
(iv) A method of item (iii), wherein said antigenic peptide or protein is expressed in diseased tissue or organ as compared to cells of normal tissue or organ.
(v) A method of item (iv), wherein said disease or disorder is cancer and said diseased tissue or organ are cancerous.
(vi) A method of item (v), wherein the recipient's cancer cells were expressing the allelic version which was different from that of a donor's allo-transplanted cells.
(vii) A method of item (i) wherein the MHC protein is a HLA molecule.
(viii) A method of any of the items (i) - (vi), wherein the epitopes created by step (iv) of claim 1 are 9mer to 16mer peptides.
(ix) A method of any of the items (i) - (viii), wherein the epitopes induce a graft-versus-host disease (GVHD) immune response.
(x) A method of any of the items (i) - (viii), wherein the epitopes induce a graft-versus-tumor (GVT) immune response.
(xi) A method of any of the items (i) - (x), wherein said protein or polypeptide of interest is selected from the antigens listed in Tables 1-6.
(xii) A synthetic peptide or polypeptide comprising a sequence that (i) derives from an allelic variant of an antigenic peptide or protein from an individual of a specific species, (ii) is encoded by a DNA sequence containing at least one single nucleotide polymorphism (SNP) in the coding region, (iii) binds to the MHC-protein complex to induce an immunogenic response, and (iv) is not identical to a sequence which induces a GVHD.
(xiii) A synthetic peptide or polypeptide of item (xii), wherein said GVHD sequence is a sequence, or a portion thereof, as given in Table 1-6.
(xiv) A synthetic antigenic peptide or polypeptide of item (xii) or (xiii), wherein said peptide or polypeptide induces GVT activity.
(xv) A synthetic peptide or polypeptide of item (xiv), wherein said GVT activity is induced by a cancer-related allelic variant.
(xvi) A synthetic peptide or polypeptide of any of the items (xii) - (xv), wherein said SNP-encoded antigen was presented by cancer cells.
(xvii) A synthetic peptide or a polypeptide of any of the items (xii) - (xvi) as a medicament.
(xviii) A synthetic peptide or polypeptide of any of the items (xii) - (xvii), obtained by the method of item (i) or (ii).
(xix) A cell expressing a peptide, polypeptide or protein, or mixtures of different such molecules, comprising the sequence of any of the synthetic peptides as defined in items (xii) - (xviii).
(xx) A cell of item (xix) wherein said cell expresses a MHC molecule that binds to said amino acid exchange carrying peptides.
(xxi) A pharmaceutical composition comprising an antigenic peptide or polypeptide, or a mixture of different peptides or polypeptides, as defined in any of the items (xii) - (xviii), or, alternatively, a cell as defined in items (xix) or (xx), optionally together with a pharmaceutically effective carrier, diluent or excipient.
(xxii)A pharmaceutical composition of item (xxi), further comprising an adjuvant.
(xxiii) Use of peptide/polypeptide, a cell or a pharmaceutical composition according to items (xxi) or (xxii) for the manufacture of a vaccine.
(xxiv) A use according to item (xxiii), wherein said vaccine is directed to antigen comprising SNP-encoded peptide presented by disease-related cells, preferably cancer cells.
(xxv)A method of treating a SNP-related disorder or disease in an individual, comprising administering to said individual an antigenic peptide or polypeptide of any of the items (xii) - (xviii) and/or a nucleic acid molecule coding for said peptides or polypeptides, wherein said peptide/polypeptide or nucleic acid enriches selectively the presentation with an HLA molecule.
(xxvi) An ex -vivo method for determining and selecting different allelic variants of a specific protein from a first individual compared with that from a second individual of the same species, wherein said difference is deduced to a specific pathogenic condition or disease of one of said individuals, the method comprising the following steps:
   (i) taking a sample of selected tissue or organ of the first individual as well as of the second individual,
   (ii) screening each sample of at least one single amino-acid mismatch of allelic variants of said protein, which binds to HLA protein and is encoded by a single SNP, or expression product thereof, or a fragment of this expression product, and
   (iii) selecting such mismatches which occur only in one of the individuals.
(xxvii) A method of item (xxvi), wherein the sample derives from diseased tissue and the amino-acid mismatch occurs in the individual being in a pathogenic condition.
(xxviii) A methodof item (xxvii), wherein a peptide or polypeptide representing said single amino-acid mismatch binds only or preferably to the antigen presenting cell or HLA protein of the diseased individual.
(xxix) A method of item (xxviii), wherein said peptide or polypeptide is recognized by cytotoxic T lymphocytes (CTLs).
(xxx)A method of transplanting hematopoetic stem cells from a donor to a recipient comprising the method of item (xxvi) - (xxix).
(xxxi) A method of treating cancer by means of hematopoetic stem cells transplantation comprising the method of item (xxx).
(xxxii) A method of item (xxxi), wherein said cancer is leukemia.
(xxxiii)An ex-vivo method for determining the progression, regression or onset of a disease treated by a method of treatment of any of items (xxx) - (xxxii), comprising (i) taking a sample from the diseased individual before and/or after allo-transplantation, and (ii) monitoring the sample for various parameters selected from the group consisting of (a) a protein, polypeptide or peptide representing the single amino-acid mismatch obtained by the method of item (xxvi), (b) an antibody which selectively binds to said protein, polypeptide or peptide, and (c) cytolytic T cells that specifically recognize that peptide, polypeptide or protein bound to the HLA molecule complex.
(xxxiv)A kit suitable for detecting the presence of the expression of a single amino acid-mismatched allelic variant related to a SNP comprising a first and a second package, each containing an isolated or synthetic nucleic acid sequence of 12 - 32 nucleotides which is a segment of the nucleotide sequence that codes for said amino acid-mismatched variant, and co-nucleotide complements, wherein the segment of the first package does not overlap with that of the second package.

Item (XXXiii) relates to the method of the invention, which is defined in claim 1.

### Detailed description of the invention

This invention introduces a new concept for the use of alloantigens in cancer therapy and transplantation in general. The definition of allo-antigens and their T-cell recognition is given through T-cell epitopes derived from common self-proteins as peptides carrying a single amino acid exchange and said exchange is defined by a coding SNP, provided that the specific allelic variant of the SNP-defined T cell epitope is not expressed in the donor however is expressed and presented in the recipient via a HLA class I molecule in a disease related cell.

T cell immunity against tumors, as pointed out earlier occurs naturally as it is not unusual that tumor associated CTLs have been found that recognize self-antigens on cancer cells. However while more and more antigens and T cell epitopes are declared to be targets in all categories of tumors, the therapeutic T cell response usually falls short of the maximally possible and required response and this is due to the fact that the functional T cell repertoire that is available to respond to infection, immunization and to tumor antigens is shaped by mechanisms that establish and maintain immunologic tolerance towards self-antigens and one way to overcome this status is by means of using the T cell repertoire of an HLA-matched individium as proposed in this application.

It is common knowledge that the prevention of autoimmune attack against normal tissues requires the deletion of T cells expressing high affinity T cell receptors for self-antigens during the process of thymus dependent selection of the T cell repertoire. In addition, other mechanisms for establishing unresponsiveness to self-antigens found exclusively outside the thymus have been described and both systems together are considered to induction tolerance to self. Numerous experiments demonstrate that encounter of tumor antigens by mature T-cells may often result in the induction of tolerance because of either immunological ignorance, anergy or physical deletion (Pardoll, 1998, Nature Med., 4:525-531, (Staveley et al., 1998). Tolerance induction may even be responsible for tumor immune evasion and finally bear the reason why currently employed methodologies for generating therapeutic tumor specific T cells response in vitro and in vivo seem unreliable. It is therefore not astonishing that most of the more then sixty tumor antigens corresponding to several hundred T cell epitopes known in the art have not been useful for inducing a long-lasting curative anti-tumor immune response in men.

To overcome the general limitation innate to auto antigens requires to alter the tolerance status of a patient, which is currently not possible due to the insufficient knowledge of the mechanisms that drive tolerance.

In the very invention this general weakness of current cancer vaccination strategies is overcome by using cytotoxic T cell populations that have not been deleted or tolarized by previous exposure to the patients self antigens. Such T cells are available through HLA-matched donors and are already present in a recipients that previously have experienced an allo-BMT or a similar treatment based on cord blood or mobilized blood stem cells.

While allo-BMT is an established method that allows to override immunological tolerance it is another finding that allo-BMT and or allogeneic stem cell transplantation is the basis for a curative T-cell based immunotherapy therapy, which help them to clear residual disease. The molecular background of the allo-response is partially uncovered, and one of the important findings is , that patients which experience allo-BMT are much better off then those which have no access to this therapy. The results and the cure rates achieved with this T cell driven method are among the most impressive results seen in immunotherapy of cancer and the current application build on this powerful mechanism.

Notice that under certain conditions related to organ transplantation the donor presents the polymorph, amino-acid mismatched T-cell epitopes to his own immune cells and this is, without a previous exchange of the blood stem cell system.

A general method for identifying alloantigens as allelic variants of antigenic petides or proteins of a species according to this disclosure requires detection of single amino acid exchanges, and comprises the steps:
(i) defining a protein or peptide exclusively expressed or over expressed in a tissue, organ or a subset thereof which relates to a disorder;
(ii) screening a data base containing one or more DNA libraries of said species for said defined peptide or protein or subset thereof, and
(iii) identifying and selecting amino acid exchanges of allelic peptide / protein variants, an expression product or a fragment thereof which is encoded by a DNA sequence containing at least one single nucleic polymorphism in the coding region,
(iv) creating T-cells epitopes (9mer - 16mer) epitopes comprising the amino acid residue containing said polymorphism, and
(v) identifying said epitopes which bind to MHC protein complex

The polymorph amino-acid mismatched peptides exclusively expressed or over expressed in a tissue; organ or cell representing the diseased tissue is characterized by the expression of one or a plurality of amino acid mismatches representing the allelic variants of proteins each of which is specific for a different, disease associated protein, and wherein said plurality is at at least 2, at least 3, at least 4, at least 4, at least 6, at least 7, or at least 8, at least 9 or at least 10 such agents.

The amino acid mismatched allo-antigens according to this disclosure are specific for a plurality of human diseases such as AML, ALL, CML, Hodgkin's disease, lymphoma, myelodysplasia, aplastic anemia, renal cell carcinoma. GVHD and host versus graft disease.

Single amino acid mismatches of allelic variants of proteins expressed in disease unrelated tissues and complexed with HLA protein are considered as GVHD inducing antigens.

Introducing the peptides into patients as a vaccine leads to a status wherein the recipients HLA molecule as well as APCs derived from the donor present the antigen to donor derived T cells (originating from a previous allo-BMT). Donor T cells may as well be expanded and differentiated from donor blood stem cells or may be taken directly from the donor and transferred to the recipient; a procedure generally known as donor lymphocyte infusion (DLI). These T cells are readily activated in the patient upon encounter of antigen presented in the context of HLA. Alternatively ex vivo stimulation of T cells and transfer into the patient may be an alternative way another way to generate activated cytotoxic T cells. On the other hand, disease conditions related for instance to induce tolerance require the appropriate activation of the patients autologous T cells.

The disclosure delivers the molecular basis of allo-BMT based therapeutic protocols and defines antigenic peptides and immunotherapeutic protocols that will induce a curative immune response similar or better to that seen with the currently applied protocols, however performed in a more predictable and less toxic manner as today. It is an essential element of this disclosure that immunological tolerance is circumvented by exploiting the T-cell repertoire of HLA-matched donors. As already shown with allogeneic BMT, T cell responses are directed against antigens other than major HLA type mismatches which an form part of the GVHD and GVT response.

Analysis of single conservative amino acid exchange, in a disease related protein, is based on the general principle of genetically inherited polymorphisms which requires the testing of patients and donors with respect to the status of the polymorph proteins. The coding SNP allo-antigen definition according to this invention implies that each individual carries according to mendelian rules either one or the other or both coding SNP variants of a gene and can either express one or the other or both polymorph amino-acid mismatch protein variants characterized by one or the other amino acid in a given position of a protein.

Diagnosing allelic versions of amino acid mismatched allo-antigens can be done by: analysis of an expression product with an altered amino acid, or a fragment of an expression product complexed with an HLA and carrying the single amino acid substitution, or by contacting biological samples isolated from a subject with an agent that specifically binds to the SNP modified nucleic acid portion, wherein the SNP carrying nucleic acid molecule is expressed preferentially or solely in cells of the diseased tissue or organ. To perform the analysis the expert tailors agents consisting of a nucleic acid molecule comprising the coding single nucleic acid molecule, a complementary nucleic acid, or a fragment thereof and performs hybridization studies with the target gene. Alternatively an antibody that binds to a single amino acid substituted allelic variant in the expression product can do as well. Antibody agents that bind to a complex of an HLA molecule and a fragment of the amino acid mismatched allelic variant can be used as well. According to the method chosen it is especially useful to perform the analysis of the SNP modified nucleic acid molecule with samples derived from disease related tissues or organs. or cells. Diagnosing and selecting appropriate therapy related single amino acid mismatches in proteins is preferentially done simultaneously for a donor and a recipient and can easily be combined with other molecular diagnosis performed such as HLA-typing. A powerful tool for testing mismatches may generated by arranging the assay in the format of a DNA array.

Furthermore according to this disclosure, specific donor derived immune cells namely specific CTLs from donors mismatched in said amino acid are enabled to recognize in the recipient the amino acid variant in a given peptide position and are capable of inducing a cytotoxic response towards said cellular target presenting said peptide.

According to the present disclosure a number of new antigens are under investigation and are described and cannot only be used to refine allo-BMT and GVT further, but also to treat patients. As recipients own T cell repertoire is found to be tolerant respectively deleted with respect to the homologous expressed allelic version representing the SNP coded amino acid, the repertoire of the HLA matched donor does unusually contain CTLs which specifically recognize the T cell epitope in the context of HLA class I molecules and kill the presenting cells. These so called allo-restricted CTL selectively kill tumor cells expressing the mis-matched allelic version of a specific SNP defined peptide. Provided that the SNP defined T-cell epitope represent proteins with a restricted tissue, organ or tumor specific expression pattern it is envisible to apply the disclosure for tumor therapy and treatment of other conditions in general. Since numerous selectively expressed tumor antigens have been described in literature, it is obvious to those skilled to apply the teaching given in this disclosure to any protein or DNA sequence described as being expressed tumor or tissue or organ or cell specific.

The hematopoetic origin of leukemia, lymphoma and myeloma, the clonal origin of the diseased cells involved and the restricted expression pattern of CD-clustered proteins are especially advantageous to demonstrate the new concept. Both the leukemic blood cells as well as the proteins known as CD-clustered proteins originate from the blood forming system e.g. blood stem cells. This makes leukemia, lymphoma and myeloma particularly suited diseases to be assessed and cured with the help of the current disclosure. Another aspect that makes leukemia, lymphoma and myeloma treatment especially promising is given by the fact that throughout allogeneic BMT the patients complete marrow stem cell-born hematopoetic system is going to by replaced by the donors blood derived stem cells or bone marrow stem cells which are then going to built-up all the future blood cells including the lymphocytes involved.

Among the diseases with a cancer origin leukemia is a preferred group of diseases to be treated according to this disclosure, and within this group CML patients are expected to have the best benefit when treated, while therapy of AML and ALL are the second choice, however preferred over lymphoma. Among the none hematological diseases RCC is the prime target for therapy and melanoma is another disease to be treated according to this disclosure.

According to this disclosure we use so far unexplored intrinsic molecular variability of antigens to give teaching for a new generalized characterization scheme for allo-antigens, define new allo-antigens and dissect the immune response related to allo-antigens with respect to circumvention of GVHD and at the same time reinforcement of GVT response, as well. This disclosure satisfies need and is prerequisite to develop effective anti-cancer vaccines and provides related advantages such as long-term graft survival of bone marrow and solid organ transplant in recipients and diagnosis of cancer.

Specific embodiments of the present disclosure relate to identification of allelic variants of genes encoding single amino acid exchanges and more general allo-antigens, with said method comprising:
1. Screening for relevant genes and the corresponding proteins, which are selectively expressed or over-expressed in a given tissue, organ or disease causing cell type and are typically expressed to a lesser extent or not at all in normal disease unrelated tissues or organs.
2. Genes preselected according to a) are screened for previously known SNPs, wherein the coding single nucleotide changes of individual genes may be obtained directly via one or more SNP-data base which are presently accessible in the public domain or are commercially available.
3. Genes preselected according to a) with no previously known SNPs correlation in DNA-databases are screened by comparing homologous DNA sequences and detecting the variable positions representative for gene and protein polymorphism with an alignment program such as BLAST.
4. Identification of polymorphic genes carrying unknown SNPs by performing indirect methods such as alignment of EST-sequences/protein-sequences covering desired genes, wherein various EST-databases deliver redundancy of sequence information with regard to an identical sequence stretch, overlapping or partially identical stretches of DNA sequences allowing for identification of SNPs via sequence alignment techniques and annotation of the corresponding gene.
5. Validation of disease relevant SNPs carrying genes and or proteins pre-screened according to methods 1-4 by measuring their organ and or tissue distribution.
6. Determining whether the identified peptides, either in their full length or as shorter fragments of the peptides, are able to stimulate T cells, locate peptides containing T-cell epitopes or nested epitopes for different major HLA class I and or HLA class II molecules and use corresponding peptides for stimulation or inhibition of T cells.

Diagnostic applications envisaged in this disclosure include, but are not limited to cancer and or BMT, stem cell and organ transplantation.

Various techniques, to allow detection of suitable donors or recipients, may be used, based on amplification of the specific nucleic acid sequences or on the protein or peptides as set out further.

According to one embodiment, the present disclosure relates to a method for typing alleles of the CD-cluster antigens in a sample comprising the detection of polymorphic nucleotides in the cDNA or genomic nucleic acids of said alleles, more particularly the alleles of the individual gene.

In a preferential embodiment said typing method will be a method of genomic DNA typing. Alternatively said method may also be a method of cDNA typing.
(a) Contacting the genomic polynucleic acids in the sample with at least one pair of primers, whereby said pair of primers specifically hybridize to the flanking regions comprising the polymorphic nucleotide in said alleles, and performing an amplification reaction;
(b) For each of said at least one pair of primers detecting whether or not in step a) an amplification product is formed;
(c) Inferring from the result of step b) which SNP- allele is present in said sample.

According to a preferred embodiment, the present disclosure relates to a method as described above, further characterized in that said alleles of the SNP-defined allo-antigens are different in the donor allele and the recipient allele.

According to the SNP variability of the human genome a given individual carries two copies of a given gene inherited from his parents. The copies may represent a different or a similar pair of alleles as inherited, thus the expert understands, that RT-PCR using mRNA as a template or standard PCR using genomic DNA as a template may be used routinely to diagnose for SNP encoded allelic amino acid variants of the present disclosure.

Blood cells with a normal genetic program or with an aberrant expression pattern such as shown for blood cancer cells all carry the normal leukocyte expressed typical assortments of molecules on their cell surfaces. The lineage markers and additional differentiation marker on the leukocyte cell surface are routinely detected with anti-leukocyte monoclonal antibodies and the antigens are named systematically by assigning them a cluster of differentiation (CD) antigen. Several sources for information regarding this system are available through the Internet and a preferred web side for the interested would be:
http://www.vetmed.wsu.edu/tkp/Search.asp

The widely accepted norm for formal designation of leukocyte surface molecules and the fact that the blood stem cells form a common origin for all blood cells including blood cancer cells makes the CD-antigens ideal candidate antigens according to this invention. Furthermore in patients in need for allogeneic stem cell transplantation all cells representing the pathologic blood cells system are finally going to be replaced by a donor derived stem cell system throughout the course of rebuilding a new hematopoetic system with the consequence, that the complete original immune and blood cell system of the recipient needs to be eliminated. In this respect the CD-antigens are representing a single "organ" and are extremely helpful to reduce the inventive concept in this invention into practice.

The present disclosure teaches how to select among several hundred known leukocyte proteins available under the current CD-system, those, which are most representative for certain types of blood born cancers. Specific CD-antigens and their correlation with disease are given in the examples and are acknowledged and used by the expert in the field. The analysis of predefined CD-proteins forms the current basis for diagnosing and staging of leukemia and lymphomas. It is however important to understand that principally the invention is not limited to the preselection of leukocyte antigens given in this application, moreover any tissue or otherwise selectively expressed protein or groups of proteins may be envisioned as well.

In this respect the disclosure provides CD-proteins comprising an immunogenic amino acid exchange which characterizes the polymorph portion of a soluble allo-antigen and which is defined on a molecular level by a coding SNP. Said CD-proteins representative for disease and qualified as allo-antigens carrying an amino acid exchange have been summarized in Table 2. The general teaching given in this application allows to those skilled in the art to define alloantigen already known in the art or additional new allo-antigens not yet disclosed in the art or mentioned herein, thus the invention is not limited to the selection of allo-antigens and T cell epitopes given in Table 2-5.

In one embodiment, the soluble allo-antigen defined by the invention induces an immune response in patients previously allo-transplanted. In a second embodiment, the antigens induce cytolytic activity upon their presentation in the context of HLA-molecule. Amino acid sequences especially useful for immunization and for induction of cytolysis may be selected from the group consisting of sequences recited in Table 3-5, and variants thereof. However as cited earlier the processing of allo-antigens might be quite variable in vivo and the N- and or C-terminal extension of the amino acid representing the exchange might vary considerable from the sequences listed. The expert in the field knows how to analyze the HLA variability and correlate the variability on an HLA-binding peptide level as well as on an HLA-binding peptide prediction level.

In a further embodiment, the soluble allo-antigen defined by the disclosure induces an immune response in patients essential for induction of immunological tolerance. Induction of immunological tolerance is particularly useful for antigen related to GVHD, which have been defined as being polymorph and broadly expressed in various tissues and organs such as lung, liver, gut, joints etc. Amino acid sequences especially useful for immunization and for induction of tolerance may be selected from the group consisting of mHAg sequences recited in Table 1 A, B and HLA sequences listed in Table 6, and variants thereof. The HLA-antigen are known in the art and defined as major antigen that drive an allo-immune reaction.. Furthermore the antigenic stretches within the molecules are well documented and various kind of detection agents are available. Nevertheless the HLA molecules have been included in this invention because the epitopes listed in Table 6 have been selected from protein regions outside the hypervariable antigenic regions, which are typically used to match HLA molecule. Thus this antigens have to be considered as SNP encoded amino acid exchanges as defined with this invention. The T cell epitopes would typically be used to prevent GVHD related to stem cell and organ transplantation.

In another embodiment, the allo-antigen comprising an immunogenic amino acid exchange is characterized by the DNA sequences encoding the inventive polypeptides, inter alia, isolated nucleic acid molecules, expression vectors containing those molecules and host cells transformed or transfected with those molecules.

The disclosure also provides isolated proteins, peptides and antibodies to those proteins and peptides and CTLs, which recognize the proteins and peptides. Fragments including functional fragments and variants of the foregoing also are provided. Kits containing the foregoing molecules additionally are provided. The foregoing can be used in the diagnosis, monitoring, research, or treatment of conditions characterized by the expression of one or more cancer associated allo-antigens. Prior to the present invention, only a handful of allo-antigens genes such as the mHAgs associated genes had been identified so far.

In another aspect, the present disclosure provides fusion proteins comprising a first and or a second disclosed T cell epitope or, alternatively, a disclosed polypeptide and a known tumor antigen, or a foreign epitope that renders the disclosed epitope immunogenic.

The disclosure involves the use of a single material, a plurality of different materials and even large panels and combinations of materials. For example, a single gene carrying the SNP, a single protein encoded by said gene, a single functional fragment thereof, a single antibody thereto, etc. can be used in methods and products of the disclosure. Likewise, pairs, groups and even panels of these materials and optionally other cancer associated allo-antigen genes and or gene products or conventional tumor antigens can be used for diagnosis, monitoring and therapy. The pairs, groups or panels can involve 2, 3, 4, 5 or more genes, gene products, fragments thereof or agents that recognize such materials. A plurality of such materials are not only useful in monitoring, typing, characterizing and diagnosing cells expressing SNP encoded modified gene products, but a plurality of such materials can be used therapeutically.

An example of this is the use of a plurality of such materials prophylactic or acutely for the prevention, delay of onset, amelioration, etc. of cancer in cells, which express or will express such genes. Any and all combinations of the genes, gene products, and materials, which recognize the genes and gene products can be tested and identified for use according to the disclosure. It would be far too lengthy to recite all such combinations; those skilled in the art, particularly in view of the teaching contained herein, will readily be able to determine which combinations are most appropriate for which circumstances.

As will be clear from the following discussion, the disclosure has in vivo and in vitro uses, including for therapeutic, diagnostic, monitoring and research purposes. One aspect of the disclosure is the ability to fingerprint a cell expressing a number of the genes identified according to the invention by, for example, quantifying the expression of such gene products. Such fingerprints will be characteristic, for example for predicting the GVT and GVHD effect in animal models for a therapy of a cancer. Cells may as well be screened to determine whether such cells express the SNP modified genes identified according to the invention.

The disclosure, in one aspect, is a method for diagnosing a therapeutically relevant allo- and cancer-associated antigen coded for by a nucleic acid molecule carrying a coding SNP. The method involves the steps of contacting a biological sample isolated from a diseased subject with an agent that specifically binds to the nucleic acid molecule carrying a coding SNP, an expression product thereof, or a fragment of an expression product thereof complexed with an MHC, preferably an HLA, molecule, wherein the molecule defined by a coding SNP may be selected from a listed allo-antigen according to Table 1-6 in form of the nucleic acid molecule, and used for determining the interaction between the agent and the nucleic acid molecule carrying a coding SNP, the expression product or fragment of the expression product thereof.

Another aspect is a method of diagnosing a therapeutically relevant and cancer associated allo-antigen coded for by nucleic acid molecule carrying a coding SNP. The method involves the steps of contacting a biological sample isolated from a subject considered as a suitable donor for BMT with an agent that specifically binds to the nucleic acid molecule carrying a coding SNP, an expression product thereof, or a fragment of an expression product thereof complexed with an MHC, preferably an HLA, molecule, wherein the nucleic acid molecule carrying a coding SNP is selected from a listed allo-antigen according to Table 1-6 in form of the nucleic acid molecule, and determining the interaction between the agent and the nucleic acid molecule carrying a coding SNP, the expression product or fragment of the expression product. In another embodiment the nucleic acid molecule carrying a coding SNP or a peptide fragment thereof may be detected with an antibody. A fragment of the single amino acid modified expression product complexed with an MHC, preferably HLA, molecule may be detected with an antibody or alternatively the HLA-complexed peptide may be used directly for diagnosis and activation or inhibition of cells.

Disorders may be characterized by expression of a plurality of cancer associated antigen precursors carrying coding-SNP modified genes. Thus the methods of diagnosis may include use of a plurality of agents, each of which is specific for a different human cancer associated SNP carrying antigen precursor (including at least one of the cancer associated antigen precursors disclosed herein), and wherein said plurality of agents is at least 2, at least 3, at least 4, at least 5, at least 6, at least 7, at least 8, at least 9 or at least 10 such agents. In each of the above embodiments the agent may be specific for a human disease preferentially cancer associated antigen precursor, including renal and blood born cancer associated antigen precursors disclosed herein.

According to the invention is a method for determining regression, progression or onset of a condition characterized by expression of a protein encoded by an SNP carrying nucleic acid molecule that is selected from a listed allo-antigen according to Table 1-6. The method involves the steps of monitoring a sample, from a subject who has or is suspected of having the condition, for a parameter selected from the group consisting of (i) the protein, (ii) a peptide derived from the protein, (iii) an antibody which selectively binds the protein or peptide, and (iv) cytolytic T cells specific for a complex of the peptide derived from the protein and an MHC/HLA molecule, as a determination of regression, progression or onset of said condition. In one embodiment the sample is a body fluid, a body effusion or a tissue. In another embodiment the step of monitoring comprises contacting the sample with a detectable agent selected from the group consisting of (a) an antibody which selectively binds the protein of (i), or the peptide of (ii), (b) a protein or peptide which binds the antibody of (iii), and (c) a cell which presents the complex of the peptide and MHC molecule of (iv). In a preferred embodiment the antibody, the protein, the peptide or the cell is labeled with a detectable molecule, such as a radioactive label or an enzyme. The sample in a preferred embodiment is assayed for the peptide.

In yet another embodiment the protein is a plurality of proteins, the parameter is a plurality of parameters, each of the plurality of parameters being specific for a different of the plurality of proteins.

The disclosure in another aspect is a pharmaceutical preparation for a human subject. The pharmaceutical preparation includes an agent which when administered to the subject enriches selectively the presence of complexes of an HLA molecule and a coding SNP modified human cancer associated allo-antigen, and a pharmaceutically acceptable carrier, wherein the human cancer associated allo-antigen is a fragment of a human cancer associated allo-antigen precursor encoded by a nucleic acid molecule which comprises nucleic acid molecules carrying a coding SNP selected from a listed allo-antigen according to Table 1-6.

In one embodiment the nucleic acid molecule is a nucleic acid molecule carrying an inventive listed allo-antigen according to Table 1-6. The agent in one embodiment comprises a plurality of agents, each of which enriches selectively in the subject complexes of an HLA molecule and a different SNP encoded human cancer associated allo-antigen. Preferably the plurality is at least two, at least three, at least four or at least five different such agents. In another embodiment the agent is selected from the group consisting of (1) an isolated polypeptide comprising the human cancer associated allo-antigen, or a functional variant thereof, (2) an isolated nucleic acid operable linked to a promoter for expressing the isolated polypeptide, or functional variant thereof, (3) a host cell expressing the isolated polypeptide, or functional variant thereof, and (4) isolated complexes of the polypeptide, or functional variants thereof, and an HLA molecule. The agent may be a cell expressing an isolated polypeptide.

In one embodiment the agent is a cell expressing an isolated polypeptide comprising the human cancer associated allo-antigen or a functional variant thereof. In another embodiment the agent is a cell expressing an isolated polypeptide comprising the human cancer associated allo-antigen or a functional variant thereof, wherein the cell expresses an HLA molecule that binds the polypeptide. The cell can express one or both of the polypeptide and HLA molecules as recombinant variants. In preferred embodiments the cell is non-proliferative.

In yet another embodiment the agent is at least two, at least three, at least four or at least five different polypeptides, each representing a different human cancer associated allo-antigen or functional variant thereof.

In other embodiments, the agent is a plurality of different agents that bind selectively at least two, at least three, at least four, or at least five different such polypeptides representing the amino acid change. In each of the above-described embodiments the agent may be an antibody. In another aspect the invention is a composition of matter composed of a conjugate of the agent of the above-described compositions of the invention and a therapeutic or diagnostic agent. Preferably the conjugate is of the agent and a therapeutic or diagnostic that is an anti-neoplastic.

The disclosure in another aspect is a pharmaceutical composition, which includes an isolated nucleic acid molecule selected from Table 2 to 6, and a pharmaceutically acceptable carrier. In one embodiment the isolated nucleic acid molecule represent one molecule selected from Table 3 and or 4. In another embodiment the isolated nucleic acid molecule comprises at least two isolated nucleic acid molecules coding for two different polypeptides, each polypeptide comprising a different cancer associated allo-antigen. Preferably the pharmaceutical composition also includes an expression vector with a promoter operable linked to the isolated nucleic acid molecule. In another embodiment the pharmaceutical composition also includes a host cell expressing the recombinant isolated nucleic acid molecule. According to another aspect of the disclosure a pharmaceutical composition is provided. The pharmaceutical composition includes an isolated polypeptide selected from Table 2 to 6 representing a single allo-antigen, and a pharmaceutically acceptable carrier. In one embodiment the isolated polypeptides represents an allo-antigen. In another embodiment the isolated polypeptides represent at least two different proteins, each comprising a different cancer associated allo-antigen encoded by a SNP modified gene as disclosed herein. In an embodiment each of the pharmaceutical compositions described herein also includes an adjuvant.

In another embodiment the fragment selected from Table 2 to 6 has a size of at least: 8 nucleotides, 10 nucleotides, 12 nucleotides, 14 nucleotides, 16 nucleotides, 18 nucleotides, 20, nucleotides, 22 nucleotides, 24 nucleotides, 26 nucleotides, 28 nucleotides, nucleotides, 50 nucleotides, 75 nucleotides, 100 nucleotides, 200 nucleotides, 1000 nucleotides and every integer length there between. In yet another embodiment the molecule encodes a polypeptide or a fragment of which, binds a human HLA receptor or a human antibody. Another aspect of the disclosure is an expression vector comprising an isolated nucleic acid molecule of the disclosure described above operable linked to a promoter. According to one aspect the invention is an expression vector comprising a nucleic acid operable linked to a promoter, wherein the nucleic acid is a SNP modified molecule. In another aspect the disclosure is an expression vector comprising a SNP modified molecule and a nucleic acid encoding an MHC, preferably HLA, molecule. In yet another aspect the disclosure is a host cell transformed or transfected with an expression vector of the disclosure described above. In another aspect the disclosure is a host cell transformed or transfected with an expression vector comprising an isolated nucleic acid molecule of the invention described above operable linked to a promoter, or an expression vector comprising a nucleic acid operable linked to a promoter, wherein the nucleic acid is a SNP modified molecule and further comprising a nucleic acid encoding HLA.

In another aspect, methods for making the nucleic acids described herein and polypeptides encoded thereby are provided. In some embodiments, the methods include culturing the host cells and isolating the nucleic acid or polypeptide from the host cells or culture medium. In other embodiments, the methods include providing a non-cell system for transcription and or translation of a nucleic acid, such a cell-free transcription and or translation lysate of rabbit reticulocytes or wheat germ extract. The most advanced system available for protein production according to this invention would however take advantage of the "cell factory" system supplied by Roche Diagnostics. In another aspect of the disclosure the methods also include introducing the nucleic acid or expression vector into the non-cell system, incubating the system under conditions sufficient for transcription or translation of the nucleic acid and isolating the transcribed nucleic acid or translated polypeptide from the non-cell system. According to another aspect of the disclosure isolated polypeptides encoded by the isolated nucleic acid molecules of the disclosure, described above, are provided. The disclosure also includes a fragment of the polypeptides selected from listed molecules in Table 1-6, which is immunogenic. In one embodiment the fragment, or a portion of the fragment, binds HLA or a human antibody. The disclosure includes in another aspect an isolated fragment of a human cancer associated antigen precursor which, or portion of which, binds HLA or a human antibody, wherein the precursor is encoded by nucleic acid molecule carrying a coding SNP that is a selected from a listed molecule summarized in Table 1-6. In one embodiment the fragment is part of a complex with HLA. In another embodiment the fragment is between 8 and 12 amino acids in length. In another embodiment the disclosure includes an isolated polypeptide comprising a fragment of the polypeptide of sufficient length to represent a sequence unique within the human genome and identifying a polypeptide that is a human cancer associated antigen precursor. According to another aspect of the disclosure a kit for detecting the presence of the expression of a cancer associated allo-antigen precursor is provided. The kit includes a pair of isolated nucleic acid molecules representative for both allelic versions of the gene.

In another approach invisible through the disclosure immune cells can be generated in vitro by culture of lymphocytes with peptides selected from listed molecules selected from Tables 2 to 5 representing the immunogenic amino acid exchange of the allo-antigen and wherein the allelic version corresponds to the version expressed by the patient's tumor cells. By using the allo-antigens according to such a procedure the donor cytotoxic and helper T cells recognizing single-antigen with single amino acid changes can be generated in vitro by a method that prevents the reactivity of the T cells to the prospective host's histocompatibility antigens, leaving a population of allo-reactive tumor-specific T cells. Another approach, which could be used in patients with or without a histocompatible sibling, comprises well-tolerated conditioning regimens that causes immunosuppression with regard to GVHD inducing allo-antigens defined according to this invention without ablating the bone marrow.

These procedures could significantly improve recently reported clinical studies that have used non-myeloablative stem-cell transplantation for other indications, including solid tumors and solid organ transplantation. When the approach is used for instance for treatment of cancer, the recipient receives T-cell-depleted hematopoetic stem cells, which the recipient will not reject, followed by the administration of progressively larger numbers of donor T cells (or tumor-specific T cells), which have been carefully stimulated regarding allo-antigen recognition. Throughout this approach it is especially useful to expand donor T cells with the help of HLA binding peptides representing the immunogenic amino acid exchange of the allo-antigen with peptides from taken from Table 2 to 5 either ex vivo or in situ in order to promote anti-tumor reactivity of the T cells. Sufficient numbers of the CTLs can be obtained for the adoptive immunotherapy purposes and in conclusion this enables a novel therapy for the treatment for relapsed leukemia after BMT with a minimal risk of inducing GVHD.

It is also possible to manipulate the allo-antigen (recognizing a single amino acid modification) specific donor lymphocytes in vitro by inserting a suicide gene known in the art, such as the herpes simplex virus thymidine kinase gene. This provides the physician with the possibility of destroying the infused lymphocytes in patients with uncontrolled graft-versus-host disease.

Another approach comprises pre-transplant immunization of allogeneic BMT donors with a recipient-defined single amino acid modified cancer allo-antigen vaccine which increases GVT activity without exacerbating GVHD because of the priming of donor T cells against putative single amino acid modified antigens on the tumor cells only. In summary the invention helps to avoid toxicity and mortality related to BMT and other transplantation related procedures.

Situations that call for vaccination with allo-antigens are preferentially characterized by low tumor burden and adoptive transfer with tumor specific T cells in cases of higher tumor burden. However the outcome of immunization with vaccines containing tumor CTL epitopes strongly depends on the mode of epitope delivery. Surprisingly, vaccination with MHC class I binding peptides may cause CTL tolerance associated with enhanced tumor outgrowth rather than immunity. These results point to the possibility of using vaccination to induce tolerance with respect to the GVHD inducing allo-antigens. On the other hand to prevent induction of tolerance the modulation of APCs is a promising strategy for enhancing responsiveness to immunization (Sotomayor, 1999). Detailed description of immunization protocols useful with allo-antigens are disclosed in the experimental part of this invention.

With respect to recipients of HLA genotype-identical transplants, disparities in allo-antigens are according to this invention defined by carrying a SNP encoded amino acid exchange. Genomic identification of the SNP defined allo-antigen locus may be performed by allele-specific PCR methods. The differences between donor and recipient are generally analyzed by two different primer sets. Each primer set consists of allele-specific primers and common primers, and both primer sets may contain intronic sequences.

Predicted allele-specific products may be correlated in all cases with the SNP encoded amino acid exchange detected by biochemical methods, by antibodies by CTLs or by RT-PCR. As has been demonstrated throughout this invention the identification of new additional allo-antigens encoded by SNP may be used for prospective genomic typing for the SNP encoded allo-antigen alleles and will improve donor selection and identify BMT recipients with respect to low or high risk of GVHD and improved GVT. SNPs according to this purpose may be detected in a sequence-specific way, by using a hybridization, primer extension or DNA ligation approach. Those skilled in the art will select a suitable approach as listed below and perform antigen analysis according to standard operation procedures specific for the individual approach.
1. The hybridization approach is based on two allele-specific probes that hybridize to the target sequence only when they match perfectly. Under optimized assay conditions, the one-base mismatch sufficiently destabilizes the hybridization to prevent the allelic probe from annealing to the target sequence. When the allele-specific probes are immobilized on a solid support, labeled target DNA samples are captured, and the hybridization event is visualized by detecting the label after the unbound targets are washed away.
2. Primer extension is another very robust allelic discrimination mechanism. It is highly flexible and requires the smallest number of primers/probes. Probe design and optimization of the assay are usually very straightforward. There are numerous variations in the primer extension approach that are based on the ability of DNA polymerase to incorporate specific deoxyribonucleosides complementary to the sequence of the template DNA however, they can be grouped into two categories.

In more detail the identity of the polymorphic base in the target DNA is determined by allele-specific nucleotide incorporation followed by sequencing. Using an allele-specific PCR approach, the DNA polymerase is used to amplify the target DNA only if the PCR primers are perfectly complementary to the target DNA sequence. A number of ingenious ways have been devised for primer extension product analysis in homogeneous assays. Most of these approaches combine novel nucleic acid analogous and monitoring of interesting differences in physical properties between starting reagents and primer extension products. In the allele-specific PCR approach, one relies on the DNA polymerase to extend a primer only when its 3' end is perfectly complementary to the template. When this condition is met, a PCR product is produced. By determining whether a PCR product is produced or not, one can infer the allele found on the target DNA. Several innovative approaches have been utilized to detect the formation of specific PCR products in homogeneous assays. Some are based on melting curve analysis, and some are based on hybridization of target specific probes. A variation of this approach is the allele-specific primer extension. Here, the PCR product containing the polymorphic site serves as template, and the 3' end of the primer extension probe consists of the allelic base. The primer is extended only if the 3' base complements the allele present in the target DNA. Monitoring the primer extension event, therefore, allows one to infer the allele(s) found in the DNA sample.

DNA ligase is highly specific in repairing nicks in the DNA molecule. When two adjacent oligonucleotides are annealed to a DNA template, they are ligated together only if the oligonucleotides perfectly match the template at the junction. Allele-specific oligonucleotides can, therefore, interrogate the nature of the base at the polymorphic site. One can infer the allele(s) present in the target DNA by determining whether ligation has occurred. Although ligation has the highest level of specificity and is easiest to optimize among all allelic discrimination mechanisms, it is the slowest reaction and requires the largest number of modified probes. However, ligation as a mechanism has the potential of genotyping without prior target amplification by PCR.

Detection of a positive allelic reaction product is done by monitoring the light emitted, or measuring the mass of the products, or detecting a change in the electrical property when the products are formed.

### Glossary

The following definitions are provided to facilitate understanding of certain terms used frequently hereinbefore.

Allo-reactive is the term used to describe polymorph T cell epitopes different among individuals which are specifically recognize by T cells.

"Antibodies" as used herein includes polyclonal and monoclonal antibodies, chimeric, single chain, and humanized antibodies, as well as Fab fragments, including the products of an Fab or other immunoglobulin expression library.

"CD-proteins" are lineage-specific cell surface markers, which are produced by the normal genetic program of the cells or by aberrant expression patterns that are pathologic. CD-proteins are typically assigned to cells derived from hematopoetic origin. The cell markers are designated according to a standard nomenclature that defines Clusters of Differentiation (CD) by scientific consensus. CD-proteins are detected by a process that combines fluorescent-labeled, monospecific immunological reagents and a flow cytometer to count and analyze the cell populations. The cells are then classified by size, marker reactivity, clonality, and proportion. The procedure is widely used clinically in diagnosis, prognosis, residual disease assessment, therapeutic monitoring, and case management of leukemia, lymphomas, and related conditions and is well known to those skilled in the art. A variety of tissues and body fluids may be analyzed. To ensure the quality and clinical utility of the interpretations, all cytometric data are interpreted in the context of a microscopic review of the specimen.

"Immunophenotyping" of cancer markers normally includes a two-step staining procedure. In the first, step antigen-specific murine mAbs as listed earlier are added to the cells. Binding of the mAbs is assessed by an immunofluorescence technique using FITC-conjugated anti-mouse Ig antisera. Distribution of antigens is analyzed by flow cytometry and or light microscopy. Results of immunotyping are typically available within 24 hours of sample receipt; and provide cytometric marker percentages. Morphologic and marker expression levels (intensity) may be also evaluated and described when relevant. Intensity of antigen expression may vary between passages and may be influenced by cell culture conditions.

"Isolated" means altered "by the hand of man" from its natural state, i.e., if it occurs in nature, it has been changed or removed from its original environment, or both. For example, a polynucleotide or a polypeptide naturally present in a living organism is not "isolated," but the same polynucleotide or polypeptide separated from the coexisting materials of its natural state is "isolated", as the term is employed herein. Moreover, a polynucleotide or polypeptide that is introduced into an organism by transformation, genetic manipulation or by any other recombinant method is "isolated" even if it is still present in said organism, which organism may be living or non-living.

"Polynucleotide" generally refers to any polyribonucleotide (RNA) or polydeoxribonucleotide (DNA), which may be unmodified or modified RNA or DNA. "Polynucleotides" include, without limitation, single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term "polynucleotide" also includes DNAs or RNAs containing one or more modified bases and DNAs or RNAs with backbones modified for stability or for other reasons. "Modified" bases include, for example, tritylated bases and unusual bases such as inosine. A variety of modifications may be made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

"Polypeptide" refers to any polypeptide comprising two or more amino acids joined to each other by peptide bonds or modified peptide bonds, i.e., peptide isoesteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as post-translational processing, or by chemical modification techniques that are well known in the art. Such modifications are well described in basic texts and in more detailed monographs, as well as in a voluminous research literature. Modifications may occur anywhere in a polypeptide, including the peptide backbone, the amino acid side-chains and the amino or carboxyl termini. It will be appreciated that the same type of modification may be present to the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from post-translation natural processes or may be made by synthetic methods. Modifications include acetylation, acylation, ADP-ribosylation, amidation, biotinylation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination (see, for instance, Proteins - Structure and Molecular Properties, 2nd Ed., T. E. Creighton, W. H. Freeman and Company, New York, 1993; Wold, F., Post-translational Protein Modifications: Perspectives and Prospects, 1-12, in Post-translational Covalent Modification of Proteins, B. C. Johnson, Ed., Academic Press, New York, 1983; Seifter et al., "Analysis for protein modifications and no protein cofactors", Meth Enzymol, 182, 626-646, 1990, and Rattan et al., "Protein Synthesis: Post-translational Modifications and Aging", Ann NY Acad Sci, 663, 48-62, 1992).

"Fragment" of a polypeptide sequence refers to a polypeptide sequence that is shorter than the reference sequence but that retains essentially the same biological function or activity as the reference polypeptide. "Fragment" of a polynucleotide sequence refers to a polynucleotide sequence that is shorter than the reference gene sequence.

"Variant" refers to a polynucleotide or polypeptide that differs from a reference polynucleotide or polypeptide, but retains the essential properties thereof. A typical variant of a polynucleotide differs in nucleotide sequence from the reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from the reference polypeptide. Generally, alterations are limited so that the sequences of the reference polypeptide and the variant are closely similar overall and, in many regions, identical. A variant and reference polypeptide may differ in amino acid sequence by one or more substitutions, insertions, and deletions in any combination. A substituted or inserted amino acid residue may or may not be one encoded by the genetic code. Typical conservative substitutions include Gly, Ala; Val, Ile, Leu; Asp, Glu; Asn, Gln; Ser, Thr; Lys, Arg; and Phe and Tyr. A variant of a polynucleotide or polypeptide may be naturally occurring such as an allele, or it may be a variant that is not known to occur naturally. Non-naturally occurring variants of polynucleotides and polypeptides may be made by mutagenesis techniques or by direct synthesis. Also included, as variants are polypeptides having one or more post-translational modifications, for instance glycosylation, phosphorylation, methylation, ADP ribosylation and the like. Embodiments include methylation of the N-terminal amino acid, phosphorylations of serines and threonines and modification of C-terminal glycines.

"Allele" refers to one of two or more alternative forms of a gene occurring at a given locus in the genome. Since mammals are diploid organisms the gene is represented twice and we have pairs of chromosomes. Two genes a particular locus, on matched sister chromosomes, control one particular trait for characteristic and are called alleles. In humans paired chromosomes may carry different alleles. If each gene is expressed, in a heterozygous situation, they are said to be co dominant and two different gene products, or allo-antigens, are produced. If each gene is expressed, in a homozygous situation, there is only one gene products, that is produced.

Two or more individuals (or strains) are stated to be allogeneic to one another when the genes at one or more loci are not identical in sequence in each organism. Allogeneic is usually specified with reference to the locus or loci involved.

Individuals of a species considered allogeneic represent an antigenic difference which will cause an immune response to allograft. The antigens concerned are often referred to as alloantigens

"Allo-antigens" are represented by two groups of histocompatibility gene products. These were seen as major and minor histocompatibility antigens. Major histocompatibility antigens stimulate acute, rapid, intense forms of graft rejection and are represented by the HLA class I and II proteins. Minor histocompatibility antigens stimulate chronic, slow, less intense reactions and represent a functionally heterogeneous group of proteins. Monoclonal antibodies for a single epitope can be produced and a panel of different monoclonal antibodies specific for various HLA antigen has been developed that permits serological tissue typing

"Polymorphism" refers to a variation in nucleotide sequence (and encoded polypeptide sequence, if relevant) at a given position in the genome within a population.

"Single Nucleotide Polymorphism" (SNP) refers to the occurrence of nucleotide variability at a single nucleotide position in the genome, within a population. An SNP may occur within a gene or within intergenic regions of the genome Many methods have been employed for detection of SNPs introduced by mutations. For instance a highly sensitive assay for mutant *ras* genes and its application to the study of presentation and relapse genotypes in acute leukemia has been described (Oncogene 9, 1994, 553-563). The two widely used methods are allele-specific amplification (ASA) and mutant-enriched PCR (ME-PCR). For the ASA process at least 3 primers are required. A common primer is used in reverse complement to the polymorphism being assayed. This common primer can be between 50 and 1500 bps from the polymorphic base. The other two (or more) primers are identical to each other except that the final 3' base wobbles to match one of the two (or more) alleles that make up the polymorphism. Two (or more) PCR reactions are then conducted on sample DNA, each using the common primer and one of the allele specific primers. For detecting inherited coding SNPs especially related to leukemia there is no typical limitation with regard to a small percentage of SNP carrying cells in a large background of normal cells as it is the case for cancer cells in general. On the other hand when analyzing blood cells detecting one SNP allele in a background of 10⁴-10⁶ mismatched type alleles may be helpful when it comes to analyzing post-transplant patients for recurring disease. Assays highly sensitive and specific for detection of SNPs have been described (Sidransky, Science 278, 1997, 1054-1058, Ahrendt et al., J. Natl. Cancer. Inst. 91, 1999, 332-339).

An important aspect with regard to this invention may be the need to carry out such assays in an automated and high-throughput manner to allow large-scale screening (Dong et al., J. Natl. Cancer Inst. 93, 2001, 858-865, Ahlquist et al., Gastroenterology 119, 2000, 1219-1227, Ahrendt et al., Proc. Natl. Acad. Sci. USA 96, 1999, 7382-7387).

"Splice Variant" as used herein refers to cDNA molecules produced from RNA molecules initially transcribed from the same genomic DNA sequence but which have undergone alternative RNA splicing. Alternative RNA splicing occurs when a primary RNA transcript undergoes splicing, generally for the removal of introns, which results in the production of more than one mRNA molecule each of that may encode different amino acid sequences. The term splice variant also refers to the proteins encoded by the above cDNA molecules.

"Identity" and "Similarity" reflect a relationship between two or more polypeptide sequences or two or more polynucleotide sequences, determined by comparing the sequences of very similar length, or over shorter, defined lengths (so-called local alignment), that is more suitable for sequences of unequal length.

Methods for comparing the identity and similarity of two or more sequences are well known in the art. Thus for instance, programs available in the Wisconsin Sequence Analysis Package, version 9.1 (Devereux J et al, Nucleic Acids Res, 12, 387-395,

Preferably, the BLOSUM62 amino acid substitution matrix (Henikoff S and Henikoff J G, Proc. Nat. Acad Sci. USA, 89, 10915-10919, 1992) is used in polypeptide sequence comparisons including where nucleotide sequences are first translated into amino acid sequences before comparison.

Polynucleotide sequence having an Identity Index of 0.95 compared to a reference polynucleotide sequence, an average of up to 5 in every 100 of the nucleotides of the in the reference sequence may be deleted, substituted or inserted, or any combination thereof, as hereinbefore described. The same applies *mutatis mutandis* for other values of the Identity Index, for instance 0.96, 0.97, 0.98 and 0.99.

"Fusion protein" refers to a protein encoded by two, unrelated, fused genes or fragments thereof. In the most general sense according to this invention at least two allo-antigens or fragments carrying the two amino acid version may be fused in a single polypeptide. In a more specific example said allo-antigens respectively fragments thereof would be fused with the Fc-portion of an immunoglobulin. Examples have been disclosed in US 5541087, 5726044. In the case of Fc-allo-antigen, employing an immunoglobulin Fc region as a part of a fusion protein is advantageous for performing the functional expression of Fc-allo-antigen or fragments of allo-antigen, to improve pharmacokinetic properties and to improve immunological properties of such a fusion protein when used for therapy. In some cases the generation of a dimerized Fc-allo-antigen might be especially beneficial. The Fc-DNA construct comprises in 5' to 3' direction, a secretion cassette, i.e. a signal sequence that triggers export from a mammalian cell, DNA encoding an immunoglobulin Fc region fragment, as a fusion partner, and a DNA encoding allo-antigen or fragments thereof. In some uses it would be desirable to be able to alter the intrinsic functional properties (complement binding, Fc-Receptor binding) by mutating the functional Fc sides while leaving the rest of the fusion protein untouched or delete the Fc part completely after expression.

"Tissue-specific" expression markers (such as CD proteins) are routinely applied in the diagnosis of leukemia and lymphoma and in addition have proven helpful in the diagnosis of solid tumors when conventional cytology alone does not provide a clear result. It is known in the art, that cells derived from hematopoetic origine are among the cell types expressing the highest number of tissue specific genes, generally refered to as lineage markers (CD proteins). The lineage cell populations include monocytes, NK cells, granulocytes (neutrophile, basophile, eosinophile), lymphocytes (T and B cells), dendritic cells and their precursors. Most human cancer reference cell lines and especially those related to leukemia and lymphoma are available from the DSMZ. Therefore, the routine test for the expression of tissue markers on all human cancer cell lines done with a panel of well-characterized monoclonal antibodies (mAbs) is common art. In general, the expression pattern of these antigens reflects that of the originating cell type. However, expression of proteins detected by individual mAbs, are not always stable over a long period of time. Therefore, not all markers reported for a given cell line are necessarily expressed on the DSMZ reference clones. In addition, different Abs against the same antigen does not always bind to the same extent leading to comparable staining intensities. Therefore, differences between reported results may occur and do not automatically question the identity of the cell line.

Tissue or tumor specificity is a nomenclature that can be used in different ways and can refer to a gene or molecule over expressed in a certain tissue as compared with other tissues or to tissue-unique genes or molecules which are expressed in 1 tissue but not in others. According to this invention both categories of genes have to be considered as target antigens is they carry polymorphisms coded by SNP.

### Example 1

### Specimen requirements for analysis of cancer markers

CD proteins and cancer markers are detected routinely by a diagnosis process that combines fluorescence-labeled, monospecific immunological reagents (antibody) and a flow cytometer to count and analyze the cell populations. The cells are then classified by size, marker reactivity, clonality, and proportion. The individual anti-CD antibodies and leukemia/lymphoma reference cell lines are readily available from reference cell collections such as ATCC or DSMZ. A desired panel of anti-CD antibodies is chosen to characterize and select the desired leukemia/lymphoma disease immuno phenotype and if necessary compare said phenotype with selected and standardized leukemia/lymphoma reference cell lines available from DSMZ. The procedure is widely used clinically in diagnosis, prognosis, residual disease assessment, therapeutic monitoring, and case management of leukemia, lymphomas and related conditions, and is well known to those skilled in the art. The reference anti-CD antibodies respectively the reference leukemia/lymphoma cell lines with characterized CD protein expression profiles are available for example through DSMZ - Deutsche Sammlung von Microorganism und Zellkulturen GmbH, Mascheroder Weg 1b, 38124 Braunschweig, GERMANY. A variety of tissues and body fluids may be analyzed. To ensure the quality and clinical utility of the interpretations, all cytometric data are interpreted in the context of a microscopic review of the specimen.

### Blood:

5-7 ml of blood in a sodium heparinized (green top) tube and 5-7 ml of blood in a EDTA (purple top) tube. Mix well by inverting. To ensure optimal results, samples should be received within 24 hours. If the sample cannot be received within 24 hours, the sample may stand at room temperature, however, a blood smear must be made from the EDTA blood and sent along with the specimen. Alternatively, a 7 or 10 ml ACD tube (yellow top) may be submitted accompanied by a white blood count and a differential blood count obtained at the same time the ACD tube was drawn (counts from a separate tube must be provided to avoid dilution effects of ACD).

### Bone Marrow:

1-2 ml of bone marrow drawn in a sodium heparinized syringe (approximately 500 USP sodium heparin per ml of specimen). Mix well. Transfer specimen to a sodium heparinized tube. More specimen may be required if marrow is hypo cellulare. Samples should be received within 24 hours. If samples cannot be shipped to arrive within 24 hours, the specimen should be put into transport media (a heparinized syringe is still necessary for the initial draw).

### Tissue:

Place tissue biopsy in sterile container with tissue culture media. Samples should be received within 24 hours to ensure optimum viability. A viability check is performed prior to analysis of cells isolated from tissue.

### Example 2

### Selection of leukemia-related tissue markers

Immunophenotyping of leukemia and lymphomas is the process used to identify and quantify cells of the blood, bone marrow and lymphatic tissues according to their biological lineage and stage of differentiation. The cell markers used are designated according to a standard nomenclature that defines CD proteins. CD-marker proteins are excellent choices, because of their expression by cells of hematopoetic origin, to start an SNP analysis according to this invention. A list of cell-surface markers relevant for the diagnosis of hematological diseases, such as leukemia and lymphomas, is given in Table 2. The final goal is to identify polymorphisms within these CD proteins that account for amino-acid changes in the corresponding proteins.

The classification of undifferentiated leukemia cells of lymphoid or myeloid origin, which may belong, e.g. to the B- or T-cell lineage, is a first step in the analysis and sub-classification of the leukemia cells within lineage types may follow. Collecting detailed information on the cell-surface marker expression is known in the art and is used for planning the experimental setup. As more markers, especially cancer marker in general, are being elucidated by expression profiling, the number of cancers applicable to the current approach increases gradually. The most prominent markers that can be used to define leukemia profiles have been tested in this invention and a list is given in Table 2. The expert in the field will readily be able to select those CD markers that are best suited to diagnose and treat a specific blood-born disease.

The following paragraphs summarize relevant CD-protein profiles that refer to specific diseases. Specific diseases include, but are not limited to, acute myeloid leukemia (AML), acute lymphoblastic leukemia (ALL), myelodysplasia syndrome (MDS) and other chronic myeloproliferative syndromes, chronic myeloid leukemia (CML), chronic lymphoblastic leukemia (CLL), multiple myeloma, lymphoma (Hodgkin or non-Hodgkin) including follicular lymphoma, intermediate-grade lymphoma, high-grade lymphoma B with small non-cleaved cell or T-cell lymphoblastic lymphoma, anaplastic large cell lymphoma, mantle cell lymphoma, peripheral T-cell lymphoma, Hodgkin's lymphoma with extra-nodal disease or B symptoms, or bulky tumor and severe aplastic anemia.

A combination of several SNPs that represent different proteins of a profile will be especially beneficial when applying the present invention for therapy and diagnosis of disease.

Acute leukemia profiles of putative or known leukemic cell populations are based on the analysis of T-cell markers (CD2, CD3, CD4, CD5, CD7, and CD8), B-cell markers are preferably CD10, CD19, CD20, CD21, CD22 and CD24 and myeloid/monocyte markers (CD13, CD14, CD15, and CD33). If useful additional B-cell markers such as CD23, 37, 38, 39, 40, 72, 73, 74, CDw75, CDw76, CD77, CDw78, CD79, CD80, CD81, CD82, CD83, CDw84, CD85, CD86 may be included. Acute megakaryoblastic leukemia may be proven by using the markers CD61, CD42 and CD41. The maturation status (non-lineage) is assessed with CD34, HLA-DR, and CD10 (CALLA). Terminal transferase (TdT) may also be requested as a separate test or added by the pathologist when warranted.

Chronic leukemia profiles and lymphoma profiles are evaluated by analyzing the total T-cell population for the presence of pan-T cell markers: CD2, CD3, CD7, CD5; the analysis of CD4 (T helper) and CD8 (T cytotoxic/ suppressor) subpopulations is routinely included. Myeloid/monocyte markers include CD14 and CD15. The total B-cell population might be narrowed down by determining the expression of B-cell markers CD19 and CD20. Co-expression of CD5 and CD20, is frequently associated with neoplastic proliferation. CD41 and CD42 may be included for differentiation of CML in blast crisis. CD10 (CALLA), CD22, CD23, CD38, CD45, FMC-1 and HLA-DR are included in the standard profile. Other markers may be added for assessing T-cell disorders (CD1, CD30), Hodgkin's disease (CD15, CD30), or anaplastic (Ki-1) lymphoma (CD30).

Hairy cell leukemia, prolymphocytic leukemia, or mantle cell lymphoma and leukemia are B-cell diseases which are characterized by a chronic leukemia profile and lymphoma profile (see above) plus expression of the hairy cell markers CD11c (complement receptor), CD 25 (IL-2 receptor), CD103, and the prolymphocytic hairy cell marker FMC-7. The B-lymphoid marker CD23 is evaluated in relation to CD5 expression for the different diagnosis of chronic leukemia vs. mantle cell lymphoma. CD23 is part of the fundamental lymphoma profile.

Anaplastic (Ki-1) lymphoma and Hodgkin's disease is evaluated via the markers CD1, CD15, and CD30 (Ki-1).

### Example 3

### New SNPs identified by screening DNA databases

SNPs are identified by screening DNA databases representing the allelic variation of the human genome, wherein the sequence of the DNA is derived from different individuals. The screening may be performed on various levels including EST, SNP or genomic DNA data. However, this will finally lead to the same result. The database useful for direct SNP screening may be selected from a group of databases comprising:
http://www.jbic.or.jp; http://snp.ims.u-tokyo.ac.ip; http://www.celera.com
http://www.ncbi.nlm.nih.gov/SNP/

To illustrate the identification of SNPs in CD-cluster proteins, various amino-acid sequences representing diverse cancer-related CD proteins have been applied for a *tblastn* search using the default mode (protein sequence against translated DNA database) of the program. Protein sequences, rather than nucleotide sequences, are particularly useful for screening, since SNPs with no effect of the exchange of an amino acid (silent mutations) can be excluded. Databank search was performed using one or more of the aforesaid public domain SNP databases.

Resulting alignments representing cDNA-sequence discrepancies (putative SNPs) among different clones of a particular CD-protein sequence were processed further according to the following rigid criteria:
(i) Alignment of genomic DNA or EST (expressed sequence tag) and CD sequence containing a X is a sequencing error and has to be ignored.
(ii) Alignment of genomic DNA clones and a CD sequence with mismatches adjacent to the 5' or 3' end are indicators for exon/intron boundaries and have to be ignored as well.
(iii) A single-base difference in the alignment between genomic DNA or EST sequences and CD sequence surrounded by perfectly matched DNA is a stringent indicator for the putative SNP.

SNPs identified on the DNA level have been further analyzed by comparing their protein sequence available through the accession code. *Tblastn* search led to the alignment as given in the CD42 example below and resulted in the identification of the putative SNPs present in the corresponding genomic clone. A list of relevant proteins that have been applied to SNP analysis is shown in Table 2.

### Example 4

### Analyzing the CD42 SNP variants

To illustrate the procedure described in example 3 in more detail, the CD42b protein has been selected as an example. The protein sequence available by accession code No. P07359 has been applied for screening of putative SNPs in the CD42b protein:
*tblastn* search led to the alignment as shown below and to the identification of two putative SNPs present in the genomic clone with acc no.AC032038.2.
>gi|121531|sp|P07359|GPBA_HUMAN PLATELET GLYCOPROTEIN IB ALPHA CHAIN PRECURSOR (GP-IB ALPHA) (GPIBA) (CD42B-ALPHA) (CD42B) [CONTAINS: GLYCOCALICIN]

### Amino acid sequence of CD42b:

MPLLLLLLLLPSPLHPHPICEVSKVASHLEVNCDKRNLTALPPDLPKDTTILHLSENLLYTFSLATLMP
YTRLTQLNLDRCELTKLQVDGTLPVLGTLDLSHNQLQSLPLLGQTLPALTVLDVSFNRLTSLPLGALRG
LGELQELYLKGNELKTLPPGLLTPTPKLEKLSLANNNLTELPAGLLNGLENLDTLLLQENSLYTIPKGF
FGSHLLPFAFLHGNPWLCNCEILYFRRWLQDNAENVYVWKQGVDVKAMTSNVASVQCDNSDKFPVYKYP
GKGCPTLGDEGDTDLYDYYPEEDTEGDKVRATRTVVKFPTKAHTTPWGLFYSWSTASLDSQMPSSLHPT
QESTKEQTTFPPRWTPNFTLHMESITFSKTPKSTTEPTPSPTTSEPVPEPAPNMTTLEPTPSPTTPEPT
SEPAPSPTTPEPTPIPTIATSPTILVSATSLITPKSTFLTTTKPVSLLESTKKTIPELDQPPKLRGVLQ
GHLESSRNDPFLHPDFCCLLPLGFYVLGLFWLLFASVVLILLLSWVGHVKPQALDSGQGAALTTATQTT
HLELQRGRQVTVPRAWLLFLRGSLPTFRSSLFLWVRPNGRVGPLVAGRRPSALSQGRGQDLLSTVSIRY
SGHSL

### CD42b fragment alignment:

Sequences representing allelic variants and comprising amino acid exchanges in the CD42b protein are labeled in bold.
>ss523802 allelePos=201 total len = 401 SC_JCM|AC032038.2_49155|taxid = 9606|mol = Genomic|subsnpClass = 1
Length = 401
Minus Strand HSPs:
Score = 736 (264.1 bits), Expect = 7.0e-71, P = 7.0eldentities = 131/133 (98%), Positives = 132/133 (99%), Frame = -1

Query: 192 TLLLQENSLYTIPKGFFGSHLLPFAFLHGNPWLCNCEILYFRRWLQDNAENVYVWKQGVD 251

         TLLLQENSLYTIPKGFFGSHLLPFAFLHGNPWLCNCEILYFRRWLQDNAENVYVWKQGVD
Sbjct:  401 TLLLQENSLYTIPKGFFGSHLLPFAFLHGNPWLCNCEILYFRRWLQDNAENVYVWKQGVD 222
Query:  252 VKAMTSNVASVQCDNSDKFPVYKYPGKGCPTLGDEGDTDLYDYYPEEDTEGDKVRATRTV 311
            VK+MTSNVASVQCDNSDKFPVYKYPGK CPTLGDEGDTDLYDYYPEEDTEGDKVRATRTV
Sbjct:  221 VKSMTSNVASVQCDNSDKFPVYKYPGKWCPTLGDEGDTDLYDYYPEEDTEGDKVRATRTV 42
Query:  312 VKFPTKAHTTPWG 324
            VKFPTKAHTTPWG
Sbjct:   41 VKFPTKAHTTPWG 3

### Example 5

### Testing cancer cell lines for HLA and SNP

Epstein Barr virus transformed leukemia/lymphoma cell lines with characterized CD protein expression profiles are available for example through DSMZ or may be isolated from patients. The cells were tested for the expression of HLA-A2 (or other desired class I expression) by immunofluorescence using the mAb BB7.2 (or other antibodies available through American Type Culture Collection, Manassas, VA). The expression and SNP-status of the hematopoetic cell and/or cancer cell was tested by genomic PCR. Poly (A)+ RNA was isolated with the QickPrep Micro mRNA purification kit (Amersham Pharmacia Biotech, Piscatawa,NJ. For the cell or tissue specific expression and detection of polymorphism of for example the CD42 gene (Acc. No J02940), PCR primers (5'-CAAGAGAACTCGCTGTATACA-3' and 5'-AAGGGGTGGTTTCGGGTATGT-3') corresponding base position 586 to 607 and base position 939 to 960, respectively of the cDNA of CD42 were used and give a 374 bp PCR product. The SNP detection was performed by subsequent sequencing of the PCR product using an ABI 310 capillary sequencer.

### Example 6

### Identification of SNP-encoded HLA-binding peptides in CD42b

In addition to the search for coding SNPs in DNA and protein sequences, the selection of an appropriate HLA class 1-binding motif is another important step towards defining a relevant SNP.

Several of the more representative HLA class I presentation molecules have been selected. The use of the SYVPEITHI algorithm has helped to generate predictions of HLA-binding peptides and the scores given in Table 4. Scores ranging from 8 to 27 indicate a high affinity of the individual peptide for binding to HLA class I molecules.

The outcome of this analysis are human sequences that are matched at 8 out of 9-residues of the peptide. Both human peptides are synthesized and tested for sensitizing activity. In rare occasions more then one amino acid mismatch may be observed within the 9-residues T-cell epitope.

### Example 7

### Selection of HLA-binding peptides for in vitro testing

For in vitro testing of the SNP-derived amino-acid exchange, the nonamer peptides preferentially possessing the known binding motifs for the HLA-A2 (HLA-A*02 or HLA-B51 or HLA-B62) have to be identified in the mature protein sequence as described above for the allo-antigen CD42.

In general the selection of HLA-A2 binding T-cell epitopes offers advantages over others due to the fact that cell lines exist in the art, which may be used for the study of antigen-presentation in vitro. Such a cell line is among others LCL.174 (a TAP-deficient mutant cell line) that carries HLA-A2 on the surface.

### Example 8

### Isolation of HLA-A2-bound peptides.

HLA-A2-bound peptides were isolated and sequenced according to standard protocols (Seeger et al., Immunogenetics, 49: 571-576, 1999, Falk et al., Nature, 351: 290-296, 1991 using the HLA-A2-specific antibody BB7.2, acid treatment, ultra filtration, and fractionation by HPLC. Peptide-containing HPLC fractions were pooled, and aliquots corresponding to peptide extracts from about 10¹⁰ cells were analyzed by nanocapillary HPLC ESI MS (Schirle et al., Eur. J. Immunol., 30: 2216-2225, 2000)

### Example 9

### Peptide synthesis

Peptides were synthesized by F-moc chemistry. F-moc chemistry is described in G. A. Grant Synthetic Peptides: A User's Guide, W. H. Freeman and Co. (1992). Identities of peptides were confirmed by amino acid analysis and matrix-assisted laser desorption/ionization mass spectrometry. Lyophilized peptides were dissolved in DMSO at 20 mM, aliquotted and stored at -80°C. Peptides were diluted to 4 mM with serum-free culture medium and used at the desired final concentrations.

### Example 10

### In vitro testing for synthetic HLA-binding peptides

Peptides with SNP-induced single amino-acid variations that can bind to HLA-A2 molecules were identified by their ability to increase the expression of HLA-A2 on the surface of TAP-deficient mutant cells of the line LCL.174. Briefly, LCL.174 cells were cultured in a round-bottomed 96-well plate at 2×10⁶ cells/well in 200 µl of RPMI together with 50 µM of peptide and incubated overnight at 37°C. The cells were then treated with HLA-A2-specific mAb, BB7.2 (ATCC, Rockville, Md.), followed by staining with FITC-conjugated goat anti-mouse IgG. Fluorescence intensity was analyzed by flow cytometry. Influenza virus matrix M1 protein peptide, FluMP58, is a known HLA-A2-restricted CTL epitope and was used as a positive control. Hepatitis B virus envelope antigen, HBenvAg125 peptide, does not bind HLA-A2 and was used as a negative control.

### Example 11

### Tetramer technology for the identification of antigen-reactive cells

Multimeric MHC class I/peptide complexes are usually generated by the expression of recombinant β2-microglobulin and heavy chain HLA molecules in bacteria. The heavy chain is mutated to remove the transmembrane region and to add a specific biotinylation sequence at the C-terminus. Purified proteins can be refolded in vitro in the presence of high concentrations of peptide/epitope to form stable and soluble HLA-peptide complexes. After enzymatic biotinylation, these complexes are multimerized with streptavidin which will bind four biotin molecules. Use of fluorescence-conjugated streptavidin allows the visualization of stained cells by flow cytometry.

In the event that MHC class I/peptide complexes are used to activate T-cells it may be helpful to combine the tetramer technology with cytokine-secretion analysis, to study the immune response in more details.

### Example 12

### In vitro stimulation of CTLs

Peripheral blood mononuclear cells (PBMCs) were prepared from 30 ml of heparinized peripheral blood from human HLA-A2⁺ subjects by centrifugation over Ficoll-Hypaque (Sigma, St. Louis, Mo.). CD8⁺ cells were positively selected from freshly isolated PBMCs, or sometimes from PBMCs frozen in liquid nitrogen, using magnetic micro beads coated with anti-CD8 antibodies according to the manufacturer's instructions (Milteny Biotec, Auburn, Calif.).

CD8⁺ cells were resuspended in serum-free DMEM and cultured in 500-µl aliquots in 48-well plates at 3×10⁶ cells/well. After 2 hr at 37°C, 5% CO₂, non-adherent cells were removed by repeated washing, and adherent monocytes were incubated for 4 hr with 50 µM peptide and 5 µg/ml human β2-microglobulin (Sigma, St. Louis, Mo.). After washing with serum-free DMEM, each well was supplemented with 1.5×10⁶ CD8⁺ cells (>90% pure by flow cytometry) in 500 µl of DMEM containing 10% human serum supplemented with rhIL-7 (0.5 ng/ml; R&D Systems, Minneapolis, Minn.).

rhIL-2 was given at 25 U/ml after 2 days and twice a week thereafter by replacing half of the culture medium. On day 10, CTL cultures were restimulated at a responder to stimulator ratio of 5 with irradiated (5000 rad) autologous LCLs. Alternatively, LCL.174 cells that had been incubated with 50 µM HLA-binding peptide defined according to this invention were used to restimulate CTL cultures obtained from HLA-A2⁺ subjects. CTL assays were performed a week after restimulation as described below.

After characterization peptide-stimulated CTLs could be frozen and stored in medium that consisted of 30% human serum, 10% DMSO and 60% DMEM, and could then be thawed and restimulated for further analysis. The peptide FIuMP58 (derived from influenza virus matrix M1 protein), was used as a positive control for in vitro stimulation of peptide-specific CTLs.

### Example 13

### Ex vivo generation of allo-antigen specific CTLs

HLA-binding peptides representing allelic versions of a preferred allo-antigen may be used and further characterized by isolating specific CTLs. The feasibility of this approach has been shown by ex vivo generation of allo-antigen-specific CTLs from unprimed amino acid-mismatched, allele-negative healthy blood donors. Synthetic peptide-pulsed dendritic cells may be used as APC to stimulate autologous or allogeneic unprimed CD8⁺ T cells. The ex vivo-generated, amino acid-mismatched, specific CTLs may then be used to efficiently lyse leukemic cells derived from acute myeloid leukemia (AML) and acute lymphoid leukemia (ALL) patients. No lytic reactivity should be detected against non-hematopoietic cells. Sufficient numbers of CTLs can be obtained for the adoptive immunotherapy purposes. It is important to note, that this technique is applicable, with no limitation, to every SNP-defined allo-antigen discovered via this invention.

### Example 14

### ⁵¹Cr release cytotoxicity test:

The cytolytic activity of these effector cells is measured by the release of isotope from the labeled target cells. The cytolytic activity is often tested in a chromium release assay but other methods are available. In chromium release assays the target cells, labeled with radioactive chromium (⁵¹Cr), are mixed with the activated CTLs. Radioactive chromium in the form of Na₂ ⁵¹CrO₄ is taken up by live cells, inside the cells the chromium is reduced. When the reduced chromium is released from lysed cells it can not be reutilized by other cells, thereby the amount of released chromium is a good measured of the cytolytic activity of the effector cells. In principle any cell type can be used as targets for measuring CTLs activity, although activated cells, such as lymphoblasts, tissue culture cells, or tumor cells have proven to be best. The natural killing activity is measured by the lysis of a reference cell line called K562. Here the test is used to measure activity of donor or recepient derived LAK cells or cytotoxic T cell clones (effector). The LAK cells or T cell clones kill target cells expressing the proper combination of HLA and allo-antigenic peptide.

A preferential target cells line pulsed with a selected allo-T cell epitope is human HLA-A2-positive EBV-LCL cell line. In addition a rich source of leukemia cell lines is available through DSMZ. 1×10⁶ cells representing a target are placed in a 5 ml falcon tube. The cells are centrifuged and resuspended by added 10µl of Na₂ ⁵²CrO₄ (high activity) mix and incubate 45 min at 37°C. The labeled cells are washed three times with PBS/FBS and resuspended in 1 ml medium and counted. The labeled cells are kept on ice until needed. For the test a total volume.of 5 ml form each target cell type, at a concentration of 5-104 cells/ml is needed and adjust the concentration.

Cytotoxic T lymphocytes (CTLs) may be generated from precursor T lymphocytes following: 1) specific stimulation by antigens carried on "stimulator" cells in the presence of accessory and helper T cells, or 2) polyclonal activation induced over four to five day by incubation with interleukin-2 and referred to as LAK cells. The preferred effector cells are moved to a 50 ml Falcon tube. The cells are washed and resuspended and adjust to a concentration of 2,5 x 10⁶ cells/ml. Effector and target cells should be mixed at 4 different effector:target ratios (50:1, 25:1, 12,5:1, 6:1). Each effector: target ratio should be tested in triplicate. A serial dilution of the effector cells is prepared in the plate so that the total volume of the effector cells is 100µl after dilution. Then 100µl of target cell suspension added. The number of target cells per well should be 5000. Include wells for spontaneous and maximum release 100µl medium that contain target cells only. Incubate plates in 37oC, 5% CO₂ during 4 hours. After 4 h harvest and count in gamma counter machine of cytotoxicity assay.

### Example 15

### Vaccine Protocol A

Selected SNP-encoded allo-antigens in form of the whole polymorph protein or at least the polymorph peptide portion thereof are used. Some proteins and peptides will be immunogenic, while others will lack immunogenicity. This lack is most readily overcome by coupling the protein or peptide to a carrier. Useful carriers include keyhole limpet hemocyanin (KLH), bovine serum albumine, BSA), *Mycobacterium bovis* BCG or purified protein derivative of tuberculin, or cholera toxin subunit B. The coupling can be achieved with any bifunctional cross-linker. A homobifunctional reagents such as Bis(sulfosuccinimidyl)Suberate, Disuccimidyl Suberate or Glutaraldehyde may be used. In cases where one of the protein or peptides is known not to display accessible groups for the cross-linking, the use of heterobifunctional reagents may advantageous. Heterobifunctional reagents such as m-Maleimdobenzoyl-N-Hydroxysuccinimide or Sulfo-m-Maleimdobenzoyl-N-Hydroxysuccinimide are among other known in the art and may be selected according to the biochemical properties of the compounds to be conjugated. The carrier conjugated to the antigen of choice in the preferred example is a KLH-antigen conjugate and may be preferentially used together with the immune adjuvant QS-21. Additional antigen formulations comprise ISCOMs, MDP, *Mycobacterium bovis* BCG, or Aluminium hydroxide. Saponins or CpG oligonucleotides are able to enhance immune responses and may be useful for selected sequences or antigen conjugates. Alternative procedures include repetitive vaccination with the selected antigen.

After thorough shaking, the administration to a human subject is done via the intravenous, intratumor, intradermal, subcutaneous or oral route. Administration should preferably be done on days 0, 7 and 14. Optionally, B-cell epitope peptides may also be included, as may booster applications.

### Vaccine Protocol B

The proposed vaccine agent is an attenuated strain of the bacterium *Salmonella typhimurium*, bearing a replicating plasmid into which the appropriate DNA sequence has been inserted, that is capable of expressing the SNP-encoding allo-antigen peptides of interest in vivo. As a vector, we propose attenuated *Salmonella typhimurium* strain X 4072 (Schödel et al., Infect. Immun. 1994, 62: 1669-1676) which has Δ crp-1 and Δ cya mutations that render it avirulent and a Δ asdA-1 mutation that renders it unviable unless a normal asdA gene is present on an indwelling plasmid. However, other safe bacterial strains me be used instead.

Plasmid pYAN is a form of pYA292 that is modified to have a Nco I site. (Schödel et al., supra). The presence of the Nco I site allows in-frame insertion of the AUG of the foreign protein or peptide of interest into the plasmid. pYAN lacks antibiotic resistance genes, allowing the use of antibiotics should symptoms suggestive of *Salmonella* pathology appear.

pYAN carries a normal asda gene, which maintains viability of only those bacteria that retain the plasmid. A DNA sequence is synthesized encoding an AUG followed by the sequences encoding the peptide. The suggested dose is 5×10⁴ colony-forming units for small children and 5×10⁵ colony-forming units for adults.

For adults, the bacteria will be administered with sodium bicarbonate (2 g of NaHCO₃ in 150 ml of distilled water). One should first drink 120 ml of the solution to neutralize gastric acid. One minute later, one drinks the remaining 30 ml of bicarbonate solution, now containing the bacteria. No food or drink is permitted for 90 minutes before or after vaccination.

### Vaccine Protocol C

Alternatively, the DNA may be delivered by other DNA delivery techniques such as those analogous to the vaccination protocol described by D. Zhang et al. (J. Infect. Dis. 1997,176: 1035-1040).

While the preferred embodiments have been described above, those skilled in the art will appreciate that other modifications can be made within the scope of the invention. For example, instead of expressing the DNA in *E. coli*, one might optimize the DNA for other hosts and express it in those hosts.

Further, while specific sequences have been identified, it is believed that the techniques of the present invention can be utilized to insert peptides longer than the desired 8-10mers having desirable CTL activation characteristics. Thus, the claims should be read understood in the broadest possible manner in order to judge the full scope of the invention.

### Vaccine Protocol D

Dendritic Cell (DC) are considered as life vectors for vaccination against cancer and are especially useful to deliver the antigens of this intervention.

Most of the more recent clinical studies have been performed by using DC generated ex vivo from CD14+ precursors (5,6) (so-called Monocyte-derived DC or Mo-DC) which are now considered as a gold standard (Thurner B et al. J Exp Med. 190: 1669, 1999; Schuler-Thurner B et al. J Exp Med. 195: 1279, 2002). The Mo-DC can be reproducibly generated within a few days in large numbers (300-500 million mature DC per aphaeresis) from precursors in blood (Feuerstein B et al. J Immunol Methods. 245: 15-29, 2000). In case of the Mo-DC the choice of maturation stimulus is critical for success and specifically, PGE2 has to be part of the maturation stimulus in order to obtain CCR7 expressing Mo-DC that migrate in response to CCL19 and CCL21 that guide DC into lymphoid organs (Luft T et al. Blood. 100: 1362, 2002; Scandella E et al. Blood. 100: 1354, 2002). Methods for the preparation of DCs, allowing GMP clinical production of a DC-based peptide vaccine against tumors are available. Selected SNP-encoded allo-antigen-carrying peptides may be used for peptide loading of DCs. Another approach to charge DC with antigens may be performed by up-take of naked RNA, encoding the desired antigen through transfection or electroporation protocol (Van Tendeloo VF et al. Blood. 98: 49, 2001) and subsequent induction of antigen-specific T cells in vitro as well as in vivo in patients.

The DCs may be loaded with up to 20 HLA class I-restricted and 10 HLA class II-restricted T-cell epitopes and act as a natural adjuvant for induction of antigen-specific CTL responses.

ELISPOT analysis (method described in example 15) performed ex vivo, in addition to tetramer technology and CTL frequency determination, may be used to monitor the immune response.

### Example 16

### Evaluating immunogenicity and efficacy of vaccination

In clinical trials with cancer antigens, the primary goal is to determine their immunogenicity. This is usually done by measuring surrogate markers for lymphocyte activation such as cytokines and interferones or end points of the immune reaction such as antibody response.

A standard assay known to the expert in the field determines whether there is induction of an (allo)antigen-specific antibody response. This is performed with a cell- or antigen-based enzyme-linked immunosorbent assays (ELISA) using serum obtained from the patients before and after vaccine administration. Autologous leukemia cells, or other disease-causing cells, or isolated antigens, especially the amino acid exchange-carrying allo-antigens thereof, are well suited targets for setting up an ELISA.

Another method is the assessment of immunogenicity by delayed-type hypersensitivity (DTH) skin testing. All patients receive intradermal injections of antigen, allo-antigen or irradiated autologous cancer cells, before and after vaccine administration. The degree of induration and erythema that is present 48 hrs after injection is measured. In addition, the DTH test may include the collection of biopsy samples that are subjected to immunohistochemical analysis to determine if there is an influx of cells of the immune system.

Another way to identify cancer-specific effector cells is based on a recently developed assay that has been useful to determine specifically activated T cells generated upon antigen encounter. The activated cells respond by release of cytokines and anti-cytokine antibodies are used to measure them, a technique known as enzyme-linked immunospot (ELISPOT) assays. ELISPOT allows performing and measuring of in vitro restimulation of the donor or patient lymphocytes with a high degree of sensitivity.

The cytokines secreted by effector cells are quantitated and additional flow cytometric analysis may be performed to measure the frequency of T-cells. Reference peptides or proteins of Influenza virus (FLU), Cytomegalovirus (CMV) or tetanus toxoid (TT) may be used in order to standardize the system. Detection of IL-4 secretion or IFN-gamma secretion is an indicator for antigen-specific CD4⁺ as well as CD8⁺ T-cells within normal PBMC. Memory-type cells are expressing CD27 and CD28, but not CD57. Following isolation and expansion with IL-2, recovered cells show antigen-specific cytotoxicity. The frequencies of antigen-specific T cells is lower for the infrequently encountered and only moderately immunogenic antigens such as TT (1 in 10.000 to 1.000.000), but much higher for the persisting virus CMV (1 in 100 to 10.000 PBMC of seropositive donors). These techniques are valuable tool in the analysis and isolation of T-cells specific for cancer and allo-antigens, according to the current invention.

### Example 17

### Measuring allo-activation

A method for enumerating and measuring the potency of the allo-activated T cells clinically may comprise distinguishing activated from inactive cells.

XTT Formazan reduction assay is beneficial to compare the activity of the allo-activated cells to a non-stimulated control. The assay is based on the ability of living cells to reduce XTT to red-orange Formazan dye, and is also helpful for distinguishing activated from inactive cells. It can be used for practically any cell in practically any media. The useful cell range is between 10⁵ and 5×10⁶ per mL. Reagents are: 96 well plates, flat bottom (not ELISA plates) 1 mg/mL MTT (2,3-bis(2-methoxy-4-nitro-5-sulfo-phenyl-2H- tetrasolium-5-carboxanilinide salt, Sigma) in PBS (fresh) 1.53 mg/mL PMS (phenylmethanesulfonyl fluoride, Sigma) in PBS (frozen, protected rom light). The assay is performed by placing 100 µl of culture media with cells in a 96 well plate in duplicate or triplicate. Use 100 µL of media alone for controls. Leave first column blank. For development pre-mix PMS with XTT immediately before use (5 µg per ml XTT) and add 50 µl of XTT to each well. Tap plate to mix. Cover plate and incubate 37° C. for 4 hours. Count plate at 470 nm (reference 650 nm).

### Example 18

### Flow cytometry for analysis of CD3/CD69 or CD3/FDA expression

This is an assay for analysis of T lymphocyte activation after allogeneic mixed lymphocyte stimulation. CD69 expression or esterase activity correlate with cytokine secretion and can be used as surrogate indicators of T lymphocyte activation. Non-stimulated lymphocytes do not express CD69 on their surface and have only low levels of non-specific esterases. Once activated by alloantigens or non-specific mitogens, the expression of CD69 appears within 48 hrs (peak at 24 hrs). Esterase activity increases shortly after stimulation, and continues for several days. Not all allo-stimulated lymphocyte reactions proceed with the same kinetics, and it is preferable to measure activation on day 1, 2 and 3 of the culture.

Test samples of donor and patient cells are mixed in small cultures at 0.5×10⁶ cells/ml in 2% FCS-RPMI. These cultures are maintained at 37° C in a 5% CO₂ incubator until testing.

### Example 19

### Cell Proliferation Assay

[³H]-thymidine incorporation into DNA is measured as follows: Responder lymphocytes are suspended at 1 million cells/ml in RPMI 1640-containing 10% fetal bovine serum, antibiotics (streptomycin/penicillin) and 5×10⁻⁵ M 2-mercaptoethanol. One hundred µl of these cells are seeded in triplicate wells of a round-bottomed microtiter plate (Costar). Allo-antigen-carrying stimulator cells are then prepared, in a way equivalent to the preparation of responder cells, but are irradiated with 3000 R (¹³⁷Cs source) prior to use. One hundred µl of the stimulator cells are added to the responder cells and the mixed lymphocyte culture is incubated at 37°C, 5% CO₂, for 7 days. Thereafter, 10 µl of [³H]-thymidine (0.5 mCi/ml, ICN Pharmaceuticals, Costa Mesa, Calif.) is added to each well for 6 hrs. The microtiter plate is then harvested, using a MASH harvester, and the amount of incorporated thymidine is determined by counting the harvested wells in a liquid scintillation counter. The stimulation index (SI) is then determined by calculating the ratio of the cpm of [³H]-thymidine incorporated into the mixed lymphocyte culture divided by the cpm of [³H]-thymidine incorporated into the control (non-stimulated) culture. Acridine orange incorporation may be used instead of [³H]-thymidine incorporation.

### Example 20

### Technique for the identification of tumor antigens

SEREX, a serological cloning approach (serological analysis of tumor antigens by recombinant cDNA expression cloning), may be performed as described by Salim et al. (Proc. Natl. Acad. Sci. USA 1995, 92: 11810-11813). Also, see U.S. Patent No. 5,698,396, incorporated herein by reference. According to this application, antisera from patients who have recently experienced allo-BMT are used to identify immunogenic protein antigens expressed in cancer cells by screening expression libraries constructed from the patients leukemia cell cDNA. Antigen-encoding clones so identified have been found to have elicited a high-titer humoral immune response in the patients' from which the antisera were obtained. Such a high-titer IgG response implies helper T cell recognition of the detected antigen and may be especially helpful to evaluate post allo-BMT patients. The expressed tumor antigens can then be screened for the presence of HLA class I and class II motifs and reactivity with CTLs. SEREX has been applied to a range of tumor types, and a number of novel cancer-associated immunogenic gene products have been cloned (Tiireci et al., Mol. Med. Today 1997, 3: 342-349; Sahin et al., Curr. Opin. Immunol. 1997, 9: 709-716; Old et al., J. Exp. Med. 1998, 187: 1163-1167). According to this patent, the antigens detected will specifically relate to allo-antigens, which are normally not accessible through this method, due to the lack of antibody responses directed to them.

### TABLES:

**Table 1 A: Minor histocompatibilty antigens from humans and mice characterized by multiple allelic variants.**

| mHAge | Protein/gene | Alleles defined | References |
|---|---|---|---|
| Human | | | |
| H-Y | SMCY protein | 1 | Wang et al. |
| H-X | SMCX protein | 1 | Goulmy et al |
| DFFRY | Y-specific gene | 2 Genes | Vogt et al |
| HA-1 | KIAA0223 gene | 2 | Den Haan et al. |
| HA-2 | Class1 myosin | 3 | Goulmy et al. |
| HA-3 | Unknown | n.n. | Goulmy et al. |
| HA-4 | Unknown | n.n. | Goulmy et al. |
| HA-5 | Unknown | 2 | Goulmy et al. |
| HA-8 | KAA0020 | 2 | Brickner et al. |
| HB-1 | HB-1 gene | 2 | Dolstra et al. |
| CD31 | PECAM-1 | 3 | Behar et al. |
| PR1 | Proteinase 3 | 2 | Molldrem et al. |
| Mouse | | | |
| AAPDNRETF | Unknown | 2 | Perreault et al. |
| COI | Cyt. iOxdase | 2 | Morse et al. |
| H-Y | SMCY protein | 1 | Meadows et al. |
| H-Y | UTY protein | 1 | Scott et al. |
| NDI | Dehydrogenas e | 4 | Loveland et al. |

**Table 1 B: Collection of human minor histocompatibilty antigens characterized by T cell epitopes.**

| MHAg | Gene/protein | T cell epitopes representing amino acid mismatches |
|---|---|---|
| Human | | |
| H-Y | UTY protein | LPHNHTDL |
| H-Y | SMCY protein AF273841 | SPSVDKARAEL RESEEESVSL |
| H-X | SMCX protein NM0044187 | FIDSYICQV |
| DFFRY | Y-specific gene Y13619 | IVDCLTEMY IVDSLTEMY |
| HA-1 | KIAA0223/AF092537 , CD49a | VLHDDLLEA VLRDDLLEA |
| HA-2 | Class1 myosin | YIGEVLVSV YLGEVLVSV YLGEVIVSV |
| HA-3 | n.n. | n.n. |
| HA-4 | n.n. | n.n. |
| HA-8 | KIAA0020 | PTLDKVLEV PTLDKVLEL RTLDKVLEV |
| HA-5 | n.n | n.n. |
| | | |
| HB-1 | CD83 XM004500, | EEKRGSLHVW EEKRGSLYVW |
| CD31 | | |
| Renal | Q9Y696 | FLDGNELTL |
| chloride | | FLDGNEMTL |
| channel | | |
| Proteinase 3 | | 119 Ile>Val |

**Table 2: Cell surface markers in hematological diseases**

| Marker | Synonyms | Specificity |
|---|---|---|
| CD 1 | | Thymocytes, Langerhans cells |
| CD 2 | | T and NK cells |
| CD 3 | | All thymocytes, T and NK cells |
| CD 4 | | Helper T cells |
| CD 5 | | All T cells, some B cells |
| CD 7 | | All T cells, some myeloid cells |
| CD 8 | | Cytotoxic T cells |
| CD 10 | CALLA antigen | Early precursor and pre-B cells |
| CD 13 | | Granulocytes, monocytes |
| CD 14 | | Monocytes |
| CD 15 | Leu M2 | All granulocytes, Reed Sternberg cells |
| CD 16 | | NK cells and granulocytes |
| CD 19 | | preB, B cells, but not plasma cells |
| CD 20 | L26 | preB, but not plasma cells |
| CD 21 | EBV-R | Mature B and follicular dendritic cells |
| CD 22 | | Mature B |
| CD 23 | | Activated marrow B |
| CD 30 | Ki-I | Activation marker for B, T, and monocytes |
| CD 33 | | Myeloid progenitor and monocytes |
| CD 34 | | Early pluripotent progenitor cell |
| CD 42 | platelet GPIb | Myeloid progenitor |
| CD 45 | LCA, leukocyte common All antigen | leukocytes |
| CD 61 | platelet glycophorin | Associated with M7 AML |
| S100 | | Interdigitating dendritic cells of the lymph node Para cortex. |
| CD 103 | alpha(E)beta7-intergrin | intraepithelial T cells |

**Table 3: Amino acid exchanges in antigens defined by coding SNPs:**

| Protein/clone | AA pos. in protein. | AA-exchange | Clone source | Remarks |
|---|---|---|---|---|
| CD1b | | | | |
| SS1509573 | 219 | R>H | cDNA | |
| CD5 | | | | |
| | 461 | R>T | Genomic | |
| CD10 | | | | |
| | 26 | R>P | Genomic | |
| | 44 | R>T | Genomic | |
| | 81 | R>T | Genomic | |
| CD11a | | | | |
| SS2883077 | 456 | I>V | Genomic (inverse) | |
| | 503 | L>V | Genomic (inverse) | E/Ibboundary ? |
| SS2077810 | 186 | T>S | Genomic | |
| | 101 | F>L | Genomic | |
| CD11c | | | | |
| | 201 | F>L | Genomic | |
| | 1126 | V>A | Genomic | |
| CD15 | | | | |
| SS602631 | 688 | D>G | Genomic | |
| | 699 | H>Y | Genomic | |
| | 717 | V>F | Genomic | |
| SS568511 | 577 | S>R | Genomic | |
| SS601426 | 623 | N>D | Genomic (inverse) | |
| SS599361 | 585 | S>N | Genomic (inverse) | |
| CD31 | | | | |
| | 125 | L>V | | |
| | 563 | N>S | | |
| | 80 | V>M | | |
| | 670 | G>R | | |
| CD32 | | | | |
| SS2972668 | 57 | Q>STOP | Genomic (inverse) | |
| | 83 | Q>P | Genomic (inverse) | |
| SS2707025 | 149 | R>H | Genomic (inverse) | |
| | 174 | Q>P | Genomic (inverse) | |
| | 180 | Q>H | Genomic (inverse) | |
| CD42b | | | | |
| ss523802 | 254 | A>S | Genomic (inverse) | |
| | 279 | G>W | Genomic (inverse) | |
| CD49a/HA-1 | | | | |
| BG220673 | 924 | F>V | | |
| BG199875 | 937 | A>V | | |
| | 961 | I>V | | |
| | 1018 | K>R | | |
| BG207145 | 984 | T>S | | |
| | 1097 | R>G | | |
| BG213953 | 1045 | N>D | | |
| | 1048 | S>Y | | |
| BG216186 | 980 | N>D | | |
| | 1019 | N>L | | |
| BG189057 | 1089 | V>G | | |
| BG181165 | 1072 | S>C | | |
| | | | | |
| CD64 | | | | |
| | 272 | R>H | Genomic | |
| SS2113895 | 224 | Q>STOP | Genomic | |
| | 272 | R>H | Genomic | |
| SS2628592 | 224 | Q>STOP | Genomic | |
| | 272 | R>H | Genomic | |
| SS848192 | 224 | Q>STOP | Genomic | same SS831265 |
| SS831265 | 115 | T>M | Genomic | * |
| SS2494502 | 115 | T>M | Genomic | * |
| SS791727 | 105 | L>P | Genomic | * |
| | 115 | T>M | Genomic | |
| | 115 | T>M | Genomic | |
| | 171 | M>K | Genomic | |
| | 175 | R>H | Genomic | |
| SS2771101 | 324 | D>N | Genomic | |
| | 338 | T>I | Genomic | |
| SS2763847 | 324 | D>N | Genomic | |
| | 338 | T>I | Genomic | |
| SS2771100 | 324 | D>N | Genomic | |
| | 338 | T>I | Genomic | |
| SS2776265 | 324 | D>N | Genomic | |
| | 338 | T>I | Genomic | |
| | 338 | T>I | Genomic | |
| SS2763848 | 324 | D>N | Genomic | |
| | 338 | T>I | Genomic | |
| CD65 | | | | |
| SS2676258 | 27 | W>R | Genomic | |
| CD83/HB-15 | | | | |
| BI7649019 | 53 | L>M | | |
| | 52 | L>V | | |
| | 51 | K>N | | |
| | 86 | N>S | | |
| BI915668 | 185 | F>S | | |
| BG705577 | 24 | K>Q | | |
| Desmin | | | | |
| SS2086285 | 23 | G>V | Genomic | * |
| | 25 | P>S | Genomic | * |
| | 39 | G>P | Genomic | * |
| | 66 | S>L | Genomic | |
| | 119 | F>S | Genomic | * |
| | 120 | A>P | Genomic | * |
| | 121 | N>I | Genomic | * |
| | 123 | I>M | Genomic | * |
| SS2857642 | 134 | A insertion | Genomic | * |
| Glycophorin A | | | | |
| SS1551184 | 13 | A>E | cDNA | |
| Rare | | | | |
| SS149153 | 206 | E>D | Genomic | |
| SS2969286 | 1760 | I>V | Genomic | |
| SS22973 | 1067 | N>K | Genomic | |
| SS1524550 | 464 | G>E | cDNA | |
| SS15224552 | 517 | Q>H | cDNA | |
| SMCY | | | | |
| SS2882267 | 748 | T>A | Genomic | |
| | 755 | I>V | Genomic | |
| | 804 | R>Q | Genomic | |
| | 817 | V>A | Genomic | |
| Vimentin | | | | |
| SS1554759 | 399 | T>A | cDNA | |

**Table 4: HLA class I binding peptides representing amino acid exchanges in CD42**

| CD42b peptide fragment | HLA type | Pos . | HLA-binding peptiden | score |
|---|---|---|---|---|
| WKQGVDVKAMTSNVASVQ | HLA-A*0201 | 9 | A M T S N V A S V | 27 |
| | HLA-A*0201 | 6 | D V K A M T S N V | 14 |
| | HLA-A*0201 | 1 | W K Q G V D V K A | 10 |
| | HLA-A*0201 | 2 | K Q G V D V K A M | 9 |
| | HLA-A*0201 | 8 | K A M T S N V A S | 9 |
| | HLA-A*0201 | 4 | G V D V K A M T S | 8 |
| | HLA-A*0201 | 7 | V K A M T S N V A | 8 |
| | HLA-A*0203 | 7 | V K A M T S N V A | 12 |
| | HLA-A*0203 | 1 | W K Q G V D V K A | 9 |
| | HLA A1 | 4 | G V D V K A M T S | 10 |
| | HLA A1 | 10 | M T S N V A S V Q | 9 |
| | HLA A3 | 4 | G V D V K A M T S | 19 |
| | HLA A3 | 6 | D V K A M T S N V | 16 |
| | HLA A3 | 10 | M T S N V A S V Q | 12 |
| | HLA A3 | 3 | Q G V D V K A M T | 8 |
| | HLA A3 | 8 | K A M T S N V A S | 8 |
| | HLA A3 | 9 | A M T S N V A S V | 8 |
| WKQGVDVKSMTSNVASVQ | HLA-A*0201 | 9 | S M T S N V A S V | 27 |
| | HLA-A*0201 | 6 | D V K S M T S N V | 14 |
| | HLA-A*0201 | 2 | K Q G V D V K S M | 10 |
| | HLA-A*0201 | 4 | G V D V K S M T S | 8 |
| | HLA-A*0203 | 7 | V K S M T S N V A | 9 |
| | HLA A1 | 4 | G V D V K S M T S | 10 |
| | HLA A1 | 10 | M T S N V A S V Q | 9 |
| | HLA A3 | 4 | G V D V K S M T S | 16 |
| | HLA A3 | 6 | D V K S M T S N V | 14 |
| | HLA A3 | 10 | M T S N V A S V Q | 12 |
| | HLA A3 | 3 | Q G V D V K S M T | 8 |
| | HLA A3 | 8 | K S M T S N V A S | 8 |
| PVYKYPGKGCPTLGDEG | HLA-A*0201 | 5 | Y P G K G C P T L | 17 |
| | HLA-A*0201 | 4 | K Y P G K G C P T | 8 |
| | HLA A1 | 3 | Y K Y P G K G C P | 8 |
| | HLA A3 | 1 | P V Y K Y P G K G | 16 |
| | HLA A3 | 3 | Y K Y P G K G C P | 10 |
| | HLA A3 | 4 | K Y P G K G C P T | 9 |
| PVYKYPGKWCPTLGDEG | HLA-A*0201 | 5 | Y P G K W C P T L | 15 |
| | HLA-A*0201 | 4 | K Y P G K W C P T | 8 |
| | HLA A1 | 3 | Y K Y P G K W C P | 8 |
| | HLA A3 | 1 | P V Y K Y P G K W | 16 |
| | HLA A3 | 3 | Y K Y P G K W C P | 9 |

**Table 5 HLA-binding peptides representing amino acid exchanges in selected CD proteins:**

| Protein | Peptide Seq. | HLA | Pos. | Peptide Seq. | Score |
|---|---|---|---|---|---|
| CD11c | FSNKFQTHFTFEEFRRT | HLA-A*0201 | 9 | F T F E E F R R T | 12 |
| | | HLA A1 | 9 | F T F E E F R R T | 10 |
| | | HLA A1 | 6 | Q T H F T F E E F | 9 |
| | | HLA A3 | 3 | N K F Q T H F T F | 8 |
| | FSNKFQTHLTFEEFRRT | HLA-A*0201 | 1 | F S N K F Q T H L | 13 |
| | | HLA-A*0201 | 9 | L T F E E F R R T | 13 |
| | | HLA A1 | 6 | Q T H L T F E E F | 9 |
| | | HLA A1 | 9 | L T F E E F R R T | 9 |
| | | HLA A3 | 8 | H L T F E E F R R | 14 |
| | | HLA A3 | 3 | N K F Q T H L T F | 11 |
| | LLLALITAVLYKVGFFK | HLA-A*0201 | 1 | L L L A L I T A V | 30 |
| | | HLA-A*0201 | 2 | L L A L I T A V L | 28 |
| | | HLA-A*0201 | 5 | L I T A V L Y K V | 26 |
| | | HLA-A*0201 | 4 | A L I T A V L Y K | 19 |
| | | HLA-A*0201 | 8 | A V L Y K V G F F | 12 |
| | | HLA-A*0201 | 9 | V L Y K V G F F K | 12 |
| | | HLA-A*0201 | 3 | L A L I T A V L Y | 10 |
| | | HLA-A*0201 | 6 | I T A V L Y K V G | 8 |
| | | HLA-A*0201 | 7 | T A V L Y K V G F | 8 |
| | | HLA A1 | 3 | L A L I T A V L Y | 17 |
| | | HLA A1 | 4 | A L I T A V L Y K | 9 |
| | | HLA A3 | 4 | A L I T A V L Y K | 29 |
| | | HLA A3 | 9 | V L Y K V G F F K | 28 |
| | | HLA A3 | 8 | A V L Y K V G F F | 21 |
| | | HLA A3 | 2 | L L A L I T A V L | 19 |
| | | HLA A3 | 1 | L L L A L I T A V | 16 |
| | | HLA A3 | 3 | L A L I T A V L Y | 16 |
| | | HLA A3 | 5 | L I T A V L Y K V | 11 |
| | | HLA A3 | 7 | T A V L Y K V G F | 9 |
| | LLLALITAALYKLGFFK | HLA-A*0201 | 2 | L L A L I T A A L | 29 |
| | | HLA-A*0201 | 5 | L I T A A L Y K L | 26 |
| | | HLA-A*0201 | 1 | L L L A L I T A A | 24 |
| | | HLA-A*0201 | 4 | A L I T A A L Y K | 15 |
| | | HLA-A*0201 | 9 | A L Y K L G F F K | 14 |
| | | HLA-A*0201 | 8 | A A L Y K L G F F | 12 |
| | | HLA-A*0201 | 3 | L A L I T A A L Y | 10 |
| | | HLA-A*0201 | 7 | T A A L Y K L G F | 9 |
| | | HLA-A*0201 | 6 | I T A A L Y K L G | 8 |
| | | HLA-A*0203 | 1 | L L L A L I T A A | 9 |
| | | HLA A1 | 3 | L A L I T A A L Y | 17 |
| | | HLA A1 | 4 | A L I T A A L Y K | 8 |
| | | HLA A1 | 6 | I T A A L Y K L G | 8 |
| | | HLA A3 | 4 | A L I T A A L Y K | 32 |
| | | HLA A3 | 9 | A L Y K L G F F K | 30 |
| | | HLA A3 | 1 | L L L A L I T A A | 16 |
| | | HLA A3 | 2 | L L A L I T A A L | 16 |
| | | HLA A3 | 3 | L A L I T A A L Y | 15 |
| | | HLA A3 | 8 | A A L Y K L G F F | 11 |
| | | HLA A3 | 5 | L I T A A L Y K L | 10 |
| | | HLA A3 | 7 | T A A L Y K L G F | 9 |
| CD15 | VTYLQNGKDRKYFHHN | HLA-A*0201 | 3 | Y L Q N G K D R K | 14 |
| | | HLA-A*0201 | 1 | V T Y L Q N G K D | 8 |
| | | HLA-A*0201 | 4 | L Q N G K D R K Y | 8 |
| | | HLA A1 | 4 | L Q N G K D R K Y | 18 |
| | | HLA A1 | 1 | V T Y L Q N G K D | 12 |
| | | HLA A1 | 7 | G K D R K Y F H H | 10 |
| | | HLA A3 | 3 | Y L Q N G K D R K | 23 |
| | | HLA A3 | 2 | T Y L Q N G K D R | 9 |
| | | HLA A3 | 4 | L Q N G K D R K Y | 9 |
| | | HLA A3 | 6 | N G K D R K Y F H | 8 |
| | | HLA A3 | 7 | G K D R K Y F H H | 8 |
| | VTYLQNGKGRKYFHHN | HLA-A*0201 | 3 | Y L Q N G K G R K | 14 |
| | | HLA-A*0201 | 1 | V T Y L Q N G K G | 8 |
| | | HLA-A*0201 | 4 | L Q N G K G R K Y | 8 |
| | | HLA A1 | 4 | L Q N G K G R K Y | 19 |
| | | HLA A1 | 1 | V T Y L Q N G K G | 12 |
| | | HLA A3 | 3 | Y L Q N G K G R K | 24 |
| | | HLA A3 | 4 | L Q N G K G R K Y | 12 |
| | | HLA A3 | 2 | T Y L Q N G K G R | 9 |
| | | HLA A3 | 5 | Q N G K G R K Y F | 9 |
| | | HLA A3 | 6 | N G K G R K Y F H | 9 |
| | | HLA A3 | 7 | G K G R K Y F H H | 8 |
| | GSYFCRGLVGSKNVSSE | HLA-A*0201 | 7 | G L V G S K N V S | 14 |
| | | HLA-A*0201 | 6 | R G L V G S K N V | 13 |
| | | HLA-A*0201 | 1 | G S Y F C R G L V | 11 |
| | | HLA-A*0201 | 3 | Y F C R G L V G S | 11 |
| | | HLA-A*0201 | 8 | L V G S K N V S S | 11 |
| | | HLA-A*0201 | 4 | F C R G L V G S K | 10 |
| | | HLA A1 | 2 | S Y F C R G L V G | 10 |
| | | HLA A3 | 4 | F C R G L V G S K | 18 |
| | | HLA A3 | 8 | L V G S K N V S S | 17 |
| | | HLA A3 | 7 | G L V G S K N V S | 16 |
| | | HLA A3 | 2 | S Y F C R G L V G | 13 |
| | | HLA A3 | 3 | Y F C R G L V G S | 9 |
| | | HLA A3 | 5 | C R G L V G S K N | 8 |
| | | HLA A3 | 6 | R G L V G S K N V | 8 |
| | GSYFCRGLFGSKNVSSE | HLA-A*0201 | 7 | G L F G S K N V S | 15 |
| | | HLA-A*0201 | 6 | R G L F G S K N V | 12 |
| | | HLA-A*0201 | 3 | Y F C R G L F G S | 9 |
| | | HLA A1 | 2 | S Y F C R G L F G | 9 |
| | | HLA A3 | 4 | F C R G L F G S K | 18 |
| | | HLA A3 | 7 | G L F G S K N V S | 16 |
| | | HLA A3 | 1 | G S Y F C R G L F | 10 |
| | | HLA A3 | 2 | S Y F C R G L F G | 9 |
| | | HLA A3 | 6 | R G L F G S K N V | 8 |
| | VTYLQNGKDRKYFHHN | HLA-A*0201 | 3 | Y L Q N G K D R K | 14 |
| | | HLA-A*0201 | 1 | V T Y L Q N G K D | 8 |
| | | HLA-A*0201 | 4 | L Q N G K D R K Y | 8 |
| | | HLA A1 | 4 | L Q N G K D R K Y | 18 |
| | | HLA A1 | 1 | V T Y L Q N G K D | 12 |
| | | HLA A1 | 7 | G K D R K Y F H H | 10 |
| | | HLA A3 | 3 | Y L Q N G K D R K | 23 |
| | | HLA A3 | 2 | T Y L Q N G K D R | 9 |
| | | HLA A3 | 4 | L Q N G K D R K Y | 9 |
| | | HLA A3 | 6 | N G K D R K Y F H | 8 |
| | | HLA A3 | 7 | G K D R K Y F H H | 8 |
| | VTYLQNGKGRKYFHHN | HLA-A*0201 | 3 | Y L Q N G K G R K | 14 |
| | | HLA-A*0201 | 1 | V T Y L Q N G K G | 8 |
| | | HLA-A*0201 | 4 | L Q N G K G R K Y | 8 |
| | | HLA A1 | 4 | L Q N G K G R K Y | 19 |
| | | HLA A1 | 1 | V T Y L Q N G K G | 12 |
| | | HLA A3 | 3 | Y L Q N G K G R K | 24 |
| | | HLA A3 | 4 | L Q N G K G R K Y | 12 |
| | | HLA A3 | 2 | T Y L Q N G K G R | 9 |
| | | HLA A3 | 5 | Q N G K G R K Y F | 9 |
| | | HLA A3 | 6 | N G K G R K Y F H | 9 |
| | | HLA A3 | 7 | G K G R K Y F H H | 8 |
| | SGSYFCRGLVGSKNVSSE | HLA-A*0201 | 8 | G L V G S K N V S | 14 |
| | | HLA-A*0201 | 1 | S G S Y F C R G L | 13 |
| | | HLA-A*0201 | 7 | R G L V G S K N V | 13 |
| | | HLA-A*0201 | 2 | G S Y F C R G L V | 11 |
| | | HLA-A*0201 | 4 | Y F C R G L V G S | 11 |
| | | HLA-A*0201 | 9 | L V G S K N V S S | 11 |
| | | HLA-A*0201 | 5 | F C R G L V G S K | 10 |
| | | HLA A1 | 3 | S Y F C R G L V G | 10 |
| | | HLA A3 | 5 | F C R G L V G S K | 18 |
| | | HLA A3 | 9 | L V G S K N V S S | 17 |
| | | HLA A3 | 8 | G L V G S K N V S | 16 |
| | | HLA A3 | 3 | S Y F C R G L V G | 13 |
| | | HLA A3 | 4 | Y F C R G L V G S | 9 |
| | | HLA A3 | 6 | C R G L V G S K N | 8 |
| | | HLA A3 | 7 | R G L V G S K N V | 8 |
| | SGSYFCRGLFGSKNVSSE | HLA-A*0201 | 8 | G L F G S K N V S | 15 |
| | | HLA-A*0201 | 1 | S G S Y F C R G L | 13 |
| | | HLA-A*0201 | 7 | R G L F G S K N V | 12 |
| | | HLA-A*0201 | 4 | Y F C R G L F G S | 9 |
| | | HLA A1 | 3 | S Y F C R G L F G | 9 |
| | | HLA A3 | 5 | F C R G L F G S K | 18 |
| | | HLA A3 | 8 | G L F G S K N V S | 16 |
| | | HLA A3 | 2 | G S Y F C R G L F | 10 |
| | | HLA A3 | 3 | S Y F C R G L F G | 9 |
| | | HLA A3 | 7 | R G L F G S K N V | 8 |
| | VFLEPQWYSVLEKDSV | HLA-A*0201 | 2 | F L E P Q W Y S V | 25 |
| | | HLA-A*0201 | 8 | Y S V L E K D S V | 14 |
| | | HLA-A*0201 | 3 | L E P Q W Y S V L | 12 |
| | | HLA A1 | 2 | F L E P Q W Y S V | 18 |
| | | HLA A3 | 2 | F L E P Q W Y S V | 15 |
| | | HLA A3 | 5 | P Q W Y S V L E K | 13 |
| | VFLEPQWYRVLEKDSV | HLA-A*0201 | 2 | F L E P Q W Y R V | 23 |
| | | HLA-A*0201 | 8 | Y R V L E K D S V | 14 |
| | | HLA-A*0201 | 3 | L E P Q W Y R V L | 12 |
| | | HLA A1 | 2 | F L E P Q W Y R V | 18 |
| | | HLA A3 | 5 | P Q W Y R V L E K | 15 |
| | | HLA A3 | 2 | F L E P Q W Y R V | 13 |
| | | HLA A3 | 6 | Q W Y R V L E K D | 10 |
| | VTYLQNGKDRKYFHHNS | HLA-A*0201 | 3 | Y L Q N G K D R K | 14 |
| | | HLA-A*0201 | 1 | V T Y L Q N G K D | 8 |
| | | HLA-A*0201 | 4 | L Q N G K D R K Y | 8 |
| | | HLA A1 | 4 | L Q N G K D R K Y | 18 |
| | | HLA A1 | 1 | V T Y L Q N G K D | 12 |
| | | HLA A1 | 7 | G K D R K Y F H H | 10 |
| | | HLA A3 | 3 | Y L Q N G K D R K | 23 |
| | | HLA A3 | 2 | T Y L Q N G K D R | 9 |
| | | HLA A3 | 4 | L Q N G K D R K Y | 9 |
| | | HLA A3 | 6 | N G K D R K Y F H | 8 |
| | | HLA A3 | 7 | G K D R K Y F H H | 8 |
| | VTYLQNGKGRKYFHHNS | HLA-A*0201 | 3 | Y L Q N G K G R K | 14 |
| | | HLA-A*0201 | 1 | VT Y L Q N G K G | 8 |
| | | HLA-A*0201 | 4 | L Q N G K G R K Y | 8 |
| | | HLA A1 | 4 | L Q N G K G R K Y | 19 |
| | | HLA A1 | 1 | V T Y L Q N G K G | 12 |
| | | HLA A3 | 3 | Y L Q N G K G R K | 24 |
| | | HLA A3 | 4 | L Q N G K G R K Y | 12 |
| | | HLA A3 | 2 | T Y L Q N G K G R | 9 |
| | | HLA A3 | 5 | Q N G K G R K Y F | 9 |
| | | HLA A3 | 6 | N G K G R K Y F H | 9 |
| | | HLA A3 | 7 | G K G R K Y F H H | 8 |
| | VFLEPQWYSVLEKDSVTYFI DAA | HLA-A*0201 | 2 | F L E P Q W Y S V | 25 |
| | | HLA-A*0201 | 9 | S V L E K D S V T | 15 |
| | | HLA-A*0201 | 8 | Y S V L E K D S V | 14 |
| | | HLA-A*0201 | 10 | V L E K D S V T Y | 14 |
| | | HLA-A*0201 | 15 | S V T Y F I D A A | 14 |
| | | HLA-A*0201 | 3 | L E P Q W Y S V L | 12 |
| | | HLA-A*0201 | 12 | E K D S V T Y F I | 8 |
| | | HLA-A*0203 | 14 | D S V T Y F I D A | 9 |
| | | HLA-A*0203 | 15 | S V T Y F I D A A | 9 |
| | | HLA A1 | 10 | V L E K D S V T Y | 27 |
| | | HLA A1 | 2 | F L E P Q W Y S V | 18 |
| | | HLA A1 | 12 | E K D S V T Y F I | 12 |
| | | HLA A1 | 14 | D S V T Y F I D A | 12 |
| | | HLA A3 | 10 | V L E K D S V T Y | 24 |
| | | HLA A3 | 9 | S V L E K D S V T | 22 |
| | | HLA A3 | 2 | F L E P Q W Y S V | 15 |
| | | HLA A3 | 5 | P Q W Y S V L E K | 13 |
| | | HLA A3 | 15 | S V T Y F I D A A | 12 |
| | VFLEPQWYRVLEKDSVTYFI DAA | HLA-A*0201 | 2 | F L E P Q W Y R V | 23 |
| | | HLA-A*0201 | 8 | Y R V L E K D S V | 14 |
| | | HLA-A*0201 | 10 | V L E K D S V T Y | 14 |
| | | HLA-A*0201 | 15 | S V T Y F I D A A | 14 |
| | | HLA-A*0201 | 9 | R V L E K D S V T | 13 |
| | | HLA-A*0201 | 3 | L E P Q W Y R V L | 12 |
| | | HLA-A*0201 | 12 | E K D S V T Y F I | 8 |
| | | HLA-A*0203 | 14 | D S V T Y F I D A | 9 |
| | | HLA A1 | 15 | S V T Y F I D A A | 9 |
| | | HLA A1 | 10 | V L E K D S V T Y | 27 |
| | | HLA A1 | 2 | F L E P Q W Y R V | 18 |
| | | HLA A1 | 12 | E K D S V T Y F I | 12 |
| | | HLA A1 | 14 | D S V T Y F I D A | 12 |
| | | HLA A1 | 5 | P Q W Y R V L E K | 7 |
| | | HLA A3 | 9 | R V L E K D S V T | 25 |
| | | HLA A3 | 10 | V L E K D S V T Y | 24 |
| | | HLA A3 | 5 | P Q W Y R V L E K | 15 |
| | | HLA A3 | 2 | F L E P Q W Y R V | 13 |
| | | HLA A3 | 15 | S V T Y F I D A A | 12 |
| | | HLA A3 | 6 | Q W Y R V L E K D | 10 |
| | TVNDSGEYRCQ | HLA A1 | 2 | V N D S G E Y R C | 13 |
| | | HLA A3 | 1 | T V N D S G E Y R | 18 |
| | TVDDSGEYRCQ | HLA A1 | 2 | V D D S G E Y R C | 13 |
| | | HLA A1 | 1 | T V D D S G E Y R | 11 |
| | | HLA A3 | 1 | T V D D S G E Y R | 18 |
| | STQWFHNESLISSQASS | HLA-A*0201 | 9 | S L I S S Q A S S | 18 |
| | | HLA-A*0201 | 2 | T Q W F H N E S L | 12 |
| | | HLA-A*0201 | 1 | S T Q W F H N E S | 10 |
| | | HLA-A*0201 | 5 | F H N E S L I S S | 10 |
| | | HLA-A*0201 | 3 | Q W F H N E S L I | 9 |
| | | HLA-A*0203 | 7 | N E S L I S S Q A | 9 |
| | | HLA A1 | 6 | H N E S L I S S Q | 10 |
| | | HLA A1 | 1 | S T Q W F H N E S | 8 |
| | | HLA A3 | 9 | S L I S S Q A S S | 19 |
| | STQWFHNENLISSQASS | HLA-A*0201 | 9 | N L I S S Q A S S | 16 |
| | | HLA-A*0201 | 1 | S T Q W F H N E N | 10 |
| | | HLA-A*0201 | 2 | T Q W F H N E N L | 10 |
| | | HLA-A*0201 | 3 | Q W F H N E N L I | 10 |
| | | HLA-A*0201 | 5 | F H N E N L I S S | 10 |
| | | HLA-A*0203 | 7 | N E N L I S S Q A | 9 |
| | | HLA A1 | 6 | H N E N L I S S Q | 10 |
| | | HLA A1 | 1 | S T Q W F H N E N | 8 |
| | | HLA A3 | 9 | N L I S S Q A S S | 18 |
| CD1b | PGRLQLVCHVS | HLA-A*0201 | 2 | G R L Q L V C H V | 18 |
| | | HLA-A*0201 | 3 | R L Q L V C H V S | 11 |
| | | HLA A3 | 3 | R L Q L V C H V S | 20 |
| | | HLA A3 | 1 | P G R L Q L V C H | 12 |
| | PGHLQLVCHVS | HLA-A*0201 | 2 | G H L Q L V C H V | 18 |
| | | HLA-A*0201 | 3 | H L Q L V C H V S | 11 |
| | | HLA A3 | 3 | H L Q L V C H V S | 16 |
| | | HLA A3 | 1 | P G H L Q L V C H | 9 |
| CD32 | HSPESDSIQWFHNGNLI | HLA-A*0201 | 7 | S I Q W F H N G N | 13 |
| | | HLA-A*0201 | 8 | I Q W F H N G N L | 12 |
| | | HLA-A*0201 | 9 | Q W F H N G N L I | 10 |
| | | HLA A1 | 2 | S P E S D S I Q W | 16 |
| | | HLA A1 | 4 | E S D S I Q W F H | 14 |
| | | HLA A3 | 7 | S I Q W F H N G N | 9 |
| | HSPESDSIPWFHNGNLI | HLA-A*0201 | 7 | S I P W F H N G N | 13 |
| | | HLA-A*0201 | 8 | I P W F H N G N L | 12 |
| | | HLA A1 | 2 | S P E S D S I P W | 16 |
| | | HLA A1 | 4 | E S D S I P W F H | 14 |
| | | HLA A1 | 6 | D S I P W F H N G | 11 |
| | | HLA A3 | 7 | S I P W F H N G N | 9 |
| | GVPGGRNHRAEVPQLEG | HLA-A*0201 | 4 | G G R N H R A E V | 15 |
| | | HLA-A*0201 | 7 | N H R A E V P Q L | 15 |
| | | HLA-A*0201 | 1 | G V P G G R N H R | 10 |
| | | HLA-A*0201 | 2 | V P G G R N H R A | 8 |
| | | HLA-A*0203 | 2 | V P G G R N H R A | 9 |
| | | HLA A1 | 9 | R A E V P Q L E G | 17 |
| | | HLA A3 | 1 | G V P G G R N H R | 19 |
| | | HLA A3 | 6 | R N H R A E V P Q | 11 |
| | | HLA A3 | 5 | G R N H R A E V P | 9 |
| | | HLA A3 | 4 | G G R N H R A E V | 8 |
| | | HLA A3 | 7 | N H R A E V P Q L | 8 |
| | GVPGGRNHHAEVPQLEG | HLA-A*0201 | 4 | G G R N H H A E V | 15 |
| | | HLA-A*0201 | 7 | N H H A E V P Q L | 15 |
| | | HLA-A*0201 | 1 | G V P G G R N H H | 10 |
| | | HLA-A*0201 | 2 | V P G G R N H H A | 8 |
| | | HLA-A*0203 | 2 | V P G G R N H H A | 9 |
| | | HLA A1 | 9 | H A E V P Q L E G | 17 |
| | | HLA A3 | 1 | G V P G G R N H H | 19 |
| | | HLA A3 | 6 | R N H H A E V P Q | 8 |
| | HILPEWKIQEIFPFGSQLLH PTSKP | HLA-A*0201 | 10 | E I F P F G S Q L | 19 |
| | | HLA-A*0201 | 17 | Q L L H P T S K P | 17 |
| | | HLA-A*0201 | 3 | L P E W K I Q E I | 16 |
| | | HLA-A*0201 | 2 | I L P E W K I Q E | 14 |
| | | HLA-A*0201 | 11 | I F P F G S Q L L | 13 |
| | | HLA-A*0201 | 7 | K I Q E I F P F G | 12 |
| | | HLA-A*0201 | 1 | H I L P E W K I Q | 11 |
| | | HLA-A*0201 | 14 | F G S Q L L H P T | 11 |
| | | HLA A1 | 3 | L P E W K I Q E I | 10 |
| | | HLA A1 | 8 | I Q E I F P F G S | 10 |
| | | HLA A1 | 12 | F P F G S Q L L H | 10 |
| | | HLA A1 | 13 | P F G S Q L L H P | 8 |
| | | HLA A3 | 10 | E I F P F G S Q L | 20 |
| | | HLA A3 | 16 | S Q L L H P T S K | 18 |
| | | HLA A3 | 17 | Q L L H P T S K P | 17 |
| | | HLA A3 | 2 | I L P E W K I Q E | 16 |
| | | HLA A3 | 1 | H I L P E W K I Q | 13 |
| | | HLA A3 | 7 | K I Q E I F P F G | 12 |
| | | HLA A3 | 12 | F P F G S Q L L H | 9 |
| | | HLA A3 | 9 | Q E I F P F G S Q | 8 |
| | HILPEWKIPEILPFGSHLLH PTSKP | HLA-A*0201 | 11 | I L P F G S H L L | 24 |
| | | HLA-A*0201 | 10 | E I L P F G S H L | 20 |
| | | HLA-A*0201 | 3 | L P E W K I P E I | 17 |
| | | HLA-A*0201 | 17 | H L L H P T S K P | 17 |
| | | HLA-A*0201 | 7 | K I P E I L P F G | 16 |
| | | HLA-A*0201 | 2 | I L P E W K I P E | 14 |
| | | HLA-A*0201 | 1 | H I L P E W K I P | 11 |
| | | HLA-A*0201 | 14 | F G S H L L H P T | 11 |
| | | HLA-A*0201 | 6 | W K I P E I L P F | 9 |
| | | HLA-A*0201 | 4 | P E W K I P E I L | 8 |
| | | HLA A1 | 3 | L P E W K I P E I | 10 |
| | | HLA A1 | 6 | W K I P E I L P F | 10 |
| | | HLA A1 | 8 | I P E I L P F G S | 10 |
| | | HLA A1 | 12 | L P F G S H L L H | 9 |
| | | HLA A3 | 10 | E I L P F G S H L | 19 |
| | | HLA A3 | 16 | S H L L H P T S K | 18 |
| | | HLA A3 | 17 | H L L H P T S K P | 15 |
| | | HLA A3 | 11 | I L P F G S H L L | 14 |
| | | HLA A3 | 1 | H I L P E W K I P | 13 |
| | | HLA A3 | 2 | I L P E W K I P E | 13 |
| | | HLA A3 | 6 | W K I P E I L P F | 12 |
| | | HLA A3 | 7 | K I P E I L P F G | 11 |
| | | HLA A3 | 9 | P E I L P F G S H | 11 |
| | | HLA A3 | 12 | L P F G S H L L H | 9 |
| CD42b | WKQGVDVKAMTSNVASVQ | HLA-A*0201 | 9 | A M T S N V A S V | 27 |
| | | HLA-A*0201 | 6 | D V K A M T S N V | 14 |
| | | HLA-A*0201 | 1 | W K Q G V D V K A | 10 |
| | | HLA-A*0201 | 2 | K Q G V D V K A M | 9 |
| | | HLA-A*0201 | 8 | K A M T S N V A S | 9 |
| | | HLA-A*0201 | 4 | G V D V K A M T S | 8 |
| | | HLA-A*0201 | 7 | V K A M T S N V A | 8 |
| | | HLA-A*0203 | 7 | V K A M T S N V A | 12 |
| | | HLA-A*0203 | 1 | W K Q G V D V K A | 9 |
| | | HLA A1 | 4 | G V D V K A M T S | 10 |
| | | HLA A1 | 10 | M T S N V A S V Q | 9 |
| | | HLA A3 | 4 | G V D V K A M T S | 19 |
| | | HLA A3 | 6 | D V K A M T S N V | 16 |
| | | HLA A3 | 10 | M T S N V A S V Q | 12 |
| | | HLA A3 | 3 | Q G V D V K A M T | 8 |
| | | HLA A3 | 8 | K A M T S N V A S 8 | 8 |
| | | HLA A3 | 9 | A M T S N V A S V | 8 |
| | WKQGVDVKSMTSNVASVQ | HLA-A*0201 | 9 | S M T S N V A S V | 27 |
| | | HLA-A*0201 | 6 | D V K S M T S N V | 14 |
| | | HLA-A*0201 | 2 | K Q G V D V K S M | 10 |
| | | HLA-A*0201 | 4 | G V D V K S M T S | 8 |
| | | HLA-A*0203 | 7 | V K S M T S N V A | 9 |
| | | HLA A1 | 4 | G V D V K S M T S | 10 |
| | | HLA A1 | 10 | M T S N V A S V Q | 9 |
| | | HLA A3 | 4 | G V D V K S M T S | 16 |
| | | HLA A3 | 6 | D V K S M T S N V | 14 |
| | | HLA A3 | 10 | M T S N V A S V Q | 12 |
| | | HLA A3 | 3 | Q G V D V K S M T | 8 |
| | | HLA A3 | 8 | K S M T S N V A S | 8 |
| | PVYKYPGKGCPTLGDEG | HLA-A*0201 | 5 | Y P G K G C P T L | 17 |
| | | HLA-A*0201 | 4 | K Y P G K G C P T | 8 |
| | | HLA A1 | 3 | Y K Y P G K G C P | 8 |
| | | HLA A3 | 1 | P V Y K Y P G K G | 16 |
| | | HLA A3 | 3 | Y K Y P G K G C P | 10 |
| | | HLA A3 | 4 | K Y P G K G C P T | 9 |
| | PVYKYPGKWCPTLGDEG | HLA-A*0201 | 5 | Y P G K W C P T L | 15 |
| | | HLA-A*0201 | 4 | K Y P G K W C P T | 8 |
| | | HLA A1 | 3 | Y K Y P G K W C P | 8 |
| | | HLA A3 | 1 | P V Y K Y P G K W | 16 |
| | | HLA A3 | 3 | Y K Y P G K W C P | 9 |
| CD64 | TEDGNVLKRSPELELQV | HLA-A*0201 | 5 | N V L K R S P E L | 19 |
| | | HLA-A*0201 | 7 | L K R S P E L E L | 16 |
| | | HLA-A*0201 | 9 | R S P E L E L Q V | 14 |
| | | HLA-A*0201 | 6 | V L K R S P E L E | 10 |
| | | HLA-A*0201 | 1 | T E D G N V L K R | 9 |
| | | HLA A1 | 1 | T E D G N V L K R | 20 |
| | | HLA A1 | 9 | R S P E L E L Q V | 10 |
| | | HLA A3 | 6 | V L K R S P E L E | 15 |
| | | HLA A3 | 5 | N V L K R S P E L | 14 |
| | | HLA A3 | 9 | R S P E L E L Q V | 11 |
| | | HLA A3 | 1 | T E D G N V L K R | 9 |
| | TEDGNVLKHSPELELQV | HLA-A*0201 | 5 | N V L K H S P E L | 19 |
| | | HLA-A*0201 | 7 | L K H S P E L E L | 16 |
| | | HLA-A*0201 | 9 | H S P E L E L Q V | 14 |
| | | HLA-A*0201 | 6 | V L K H S P E L E | 10 |
| | | HLA-A*0201 | 1 | T E D G N V L K H | 9 |
| | | HLA A1 | 1 | T E D G N V L K H | 20 |
| | | HLA A1 | 9 | H S P E L E L Q V | 10 |
| | | HLA A3 | 5 | N V L K H S P E L | 12 |
| | | HLA A3 | 6 | V L K H S P E L E | 12 |
| | | HLA A3 | 1 | T E D G N V L K H | 9 |
| | LLQVSSRVFTEGEPLALR | HLA-A*0201 | 9 | F T E G E P L A L | 18 |
| | | HLA-A*0201 | 7 | R V F T E G E P L | 15 |
| | | HLA-A*0201 | 1 | L L Q V S S R V F | 12 |
| | | HLA-A*0201 | 3 | Q V S S R V F T E | 10 |
| | | HLA-A*0201 | 8 | V F T E G E P L A | 9 |
| | | HLA-A*0201 | 10 | T E G E P L A L R | 9 |
| | | HLA-A*0201 | 2 | L Q V S S R V F T | 8 |
| | | HLA-A*0203 | 8 | V F T E G E P L A | 9 |
| | | HLA A1 | 9 | F T E G E P L A L | 24 |
| | | HLA A1 | 4 | V S S R V F T E G | 10 |
| | | HLA A3 | 1 | L L Q V S S R V F | 18 |
| | | HLA A3 | 3 | Q V S S R V F T E | 18 |
| | | HLA A3 | 7 | R V F T E G E P L | 17 |
| | | HLA A3 | 10 | T E G E P L A L R | 8 |
| | LLQVSSRVFMEGEPLALR | HLA-A*0201 | 9 | F M E G E P L A L | 22 |
| | | HLA-A*0201 | 7 | R V F M E G E P L | 15 |
| | | HLA-A*0201 | 1 | L L Q V S S R V F | 12 |
| | | HLA-A*0201 | 8 | V F M E G E P L A | 11 |
| | | HLA-A*0201 | 10 | M E G E P L A L R | 9 |
| | | HLA-A*0201 | 2 | L Q V S S R V F M | 8 |
| | | HLA-A*0201 | 3 | Q V S S R V F M E | 8 |
| | | HLA-A*0203 | 8 | V F M E G E P L A | 9 |
| | | HLA A1 | 9 | F M E G E P L A L | 18 |
| | | HLA A1 | 4 | V S S R V F M E G | 10 |
| | | HLA A3 | 1 | L L Q V S S R V F | 18 |
| | | HLA A3 | 7 | R V F M E G E P L | 17 |
| | | HLA A3 | 3 | Q V S S R V F M E | 15 |
| | | HLA A3 | 10 | M E G E P L A L R | 8 |
| | NGTYHCSGMGKHRYTSAGI | HLA-A*0201 | 8 | G M G K H R Y T S | 12 |
| | | HLA-A*0201 | 11 | K H R Y T S A G I | 11 |
| | | HLA-A*0201 | 7 | S G M G K H R Y T | 10 |
| | | HLA-A*0203 | 9 | M G K H R Y T S A | 9 |
| | | HLA A1 | 6 | C S G M G K H R Y | 21 |
| | | HLA A3 | 3 | T Y H C S G M G K | 14 |
| | | HLA A3 | 5 | H C S G M G K H R | 9 |
| | | HLA A3 | 11 | K H R Y T S A G I | 9 |
| | | HLA A3 | 6 | C S G M G K H R Y | 8 |
| | NGTYHCSGKGKHHYTSAGI | HLA-A*0201 | 11 | K H H Y T S A G I | 11 |
| | | HLA-A*0201 | 7 | S G K G K H H Y T | 9 |
| | | HLA-A*0201 | 2 | G T Y H C S G K G | 8 |
| | | HLA-A*0203 | 9 | K G K H H Y T S A | 9 |
| | | HLA A1 | 6 | C S G K G K H H Y | 21 |
| | | HLA A1 | 2 | G T Y H C S G K G | 8 |
| | | HLA A3 | 1 | N G T Y H C S G K | 13 |
| | | HLA A3 | 3 | T Y H C S G K G K | 13 |
| | | HLA A3 | 5 | H C S G K G K H H | 10 |
| | | HLA A3 | 6 | C S G K G K H H Y | 8 |
| | ELKRKKKWDLEISLDSGHEK | HLA-A*0201 | 6 | K K W D L E I S L | 16 |
| | | HLA-A*0201 | 9 | D L E I S L D S G | 14 |
| | | HLA-A*0201 | 2 | L K R K K K W D L | 13 |
| | | HLA-A*0201 | 4 | R K K K W D L E I | 10 |
| | | HLA-A*0201 | 12 | I S L D S G H E K | 8 |
| | | HLA A1 | 7 | K W D L E I S L D | 12 |
| | | HLA A1 | 9 | D L E I S L D S G | 11 |
| | | HLA A3 | 12 | I S L D S G H E K | 18 |
| | | HLA A3 | 1 | E L K R K K K W D | 16 |
| | | HLA A3 | 9 | D L E I S L D S G | 13 |
| | | HLA A3 | 10 | L E I S L D S G H | 10 |
| | | HLA A3 | 11 | E I S L D S G H E | 10 |
| | | HLA A3 | 4 | R K K K W D L E I | 8 |
| | ELKRKKKWNLEISLDSGHEK | HLA-A*0201 | 6 | K K W N L E I S L | 15 |
| | | HLA-A*0201 | 9 | N L E I S L D S G | 15 |
| | | HLA-A*0201 | 2 | L K R K K K W N L | 13 |
| | | HLA-A*0201 | 4 | R K K K W N L E I | 10 |
| | | HLA-A*0201 | 12 | I S L D S G H E K | 8 |
| | | HLA A1 | 9 | N L E I S L D S G | 11 |
| | | HLA A3 | 12 | I S L D S G H E K | 18 |
| | | HLA A3 | 1 | E L K R K K K W N | 16 |
| | | HLA A3 | 9 | N L E I S L D S G | 13 |
| | | HLA A3 | 10 | L E I S L D S G H | 10 |
| | | HLA A3 | 11 | E I S L D S G H E | 10 |
| | | HLA A3 | 4 | R K K K W N L E I | 8 |
| | KVTSSLQEDRH | HLA-A*0201 | 1 | K V T S S L Q E D | 10 |
| | | HLA A3 | 1 | K V T S S L Q E D | 13 |
| | KVISSLQEDRH | HLA-A*0201 | 1 | K V I S S L Q E D | 13 |
| | | HLA-A*0201 | 2 | V I S S L Q E D R | 10 |
| | | HLA A3 | 1 | K V I S S L Q E D | 16 |
| | | HLA A3 | 2 | V I S S L Q E D R | 12 |
| | VSSRVFTEGEPLALR | HLA-A*0201 | 6 | F T E G E P L A L | 18 |
| | | HLA-A*0201 | 4 | R V F T E G E P L | 15 |
| | | HLA-A*0201 | 5 | V F T E G E P L A | 9 |
| | | HLA-A*0201 | 7 | T E G E P L A L R | 9 |
| | | HLA-A*0203 | 5 | V F T E G E P L A | 9 |
| | | HLA A1 | 6 | F T E G E P L A L | 24 |
| | | HLA A1 | 1 | V S S R V F T E G | 10 |
| | | HLA A3 | 4 | R V F T E G E P L | 17 |
| | | HLA A3 | 7 | T E G E P L A L R | 8 |
| | VSSRVFMEGEPLALR | HLA-A*0201 | 6 | F M E G E P L A L | 22 |
| | | HLA-A*0201 | 4 | R V F M E G E P L | 15 |
| | | HLA-A*0201 | 5 | V F M E G E P L A | 11 |
| | | HLA-A*0201 | 7 | M E G E P L A L R | 9 |
| | | HLA-A*0203 | 5 | V F M E G E P L A | 9 |
| | | HLA A1 | 6 | F M E G E P L A L | 18 |
| | | HLA A1 | 1 | V S S R V F M E G | 10 |
| | | HLA A3 | 4 | R V F M E G E P L | 17 |
| | | HLA A3 | 7 | M E G E P L A L R | 8 |
| | LQVSSRVFTEGEPLALR | HLA-A*0201 | 8 | F T E G E P L A L | 18 |
| | | HLA-A*0201 | 6 | R V F T E G E P L | 15 |
| | | HLA-A*0201 | 2 | Q V S S R V F T E | 10 |
| | | HLA-A*0201 | 7 | V F T E G E P L A | 9 |
| | | HLA-A*0201 | 9 | T E G E P L A L R | 9 |
| | | HLA-A*0201 | 1 | L Q V S S R V F T | 8 |
| | | HLA-A*0203 | 7 | V F T E G E P L A | 9 |
| | | HLA A1 | 8 | F T E G E P L A L | 24 |
| | | HLA A1 | 3 | V S S R V F T E G | 10 |
| | | HLA A3 | 2 | Q V S S R V F T E | 18 |
| | | HLA A3 | 6 | R V F T E G E P L | 17 |
| | | HLA A3 | 9 | T E G E P L A L R | 8 |
| | LQVSSRVFMEGEPLALR | HLA-A*0201 | 8 | F M E G E P L A L | 22 |
| | | HLA-A*0201 | 6 | R V F M E G E P L | 15 |
| | | HLA-A*0201 | 7 | V F M E G E P L A | 11 |
| | | HLA-A*0201 | 9 | M E G E P L A L R | 9 |
| | | HLA-A*0201 | 1 | L Q V S S R V F M | 8 |
| | | HLA-A*0203 | 7 | V F M E G E P L A | 9 |
| | | HLA A1 | 8 | F M E G E P L A L | 18 |
| | | HLA A1 | 3 | V S S R V F M E G | 10 |
| | | HLA A3 | 6 | R V F M E G E P L | 17 |
| | | HLA A3 | 2 | Q V S S R V F M E | 15 |
| | | HLA A3 | 9 | M E G E P L A L R | 8 |
| CD65 | PATTPTPWRTRMLWPS | HLA-A*0201 | 5 | P T P W R T R M L | 13 |
| | | HLA-A*0201 | 2 | A T T P T P W R T | 11 |
| | | HLA A1 | 2 | A T T P T P W R T | 11 |
| | | HLA A3 | 7 | P W R T R M L W P | 9 |
| | | HLA A3 | 3 | T T P T P W R T R | 8 |
| | PATTPTPRRTRMLWPS | HLA-A*0201 | 5 | P T P R R T R M L | 13 |
| | | HLA-A*0201 | 2 | A T T P T P R R T | 11 |
| | | HLA A1 | 2 | A T T P T P R R T | 11 |
| | | HLA A1 | 3 | T T P T P R R T R | 8 |
| | | HLA A3 | 3 | T T P T P R R T R | 9 |
| | | HLA A3 | 7 | P R R T R M L W P | 9 |
| | | HLA A3 | 6 | T P R R T R M L W | 8 |
| desmin | GGAGGSGSLRASRL | HLA-A*0201 | 1 | G G A G G S G S L | 19 |
| | | HLA-A*0201 | 6 | S G S L R A S R L | 12 |
| | | HLA-A*0201 | 3 | A G G S G S L R A | 9 |
| | | HLA-A*0201 | 4 | G G S G S L R A S | 8 |
| | | HLA-A*0203 | 3 | A G G S G S L R A | 10 |
| | | HLA A1 | 3 | A G G S G S L R A | 9 |
| | | HLA A3 | 2 | G A G G S G S L R | 12 |
| | | HLA A3 | 5 | G S G S L R A S R | 10 |
| | | HLA A3 | 6 | S G S L R A S R L | 10 |
| | | HLA A3 | 1 | G G A G G S G S L | 8 |
| | GGAGGLGSLRASRL | HLA-A*0201 | 1 | G G A G G L G S L | 23 |
| | | HLA-A*0201 | 5 | G L G S L R A S R | 16 |
| | | HLA-A*0201 | 6 | L G S L R A S R L | 12 |
| | | HLA-A*0201 | 4 | G G L G S L R A S | 10 |
| | | HLA-A*0201 | 3 | A G G L G S L R A | 9 |
| | | HLA-A*0201 | 2 | G A G G L G S L R | 8 |
| | | HLA-A*0203 | 3 | A G G L G S L R A | 10 |
| | | HLA A1 | 3 | A G G L G S L R A | 9 |
| | | HLA A3 | 5 | G L G S L R A S R | 20 |
| | | HLA A3 | 2 | G A G G L G S L R | 12 |
| | | HLA A3 | 3 | A G G L G S L R A | 9 |
| | | HLA A3 | 6 | L G S L R A S R L | 9 |
| | | HLA A3 | 1 | G G A G G L G S L | 8 |
| | RRTFGGAPGFPLGSPLSSPV FPRAGFGSKGSSS | HLA-A*0201 | 12 | L G S P L S S P V | 15 |
| | | HLA-A*0201 | 11 | P L G S P L S S P | 14 |
| | | HLA-A*0201 | 4 | F G G A P G F P L | 12 |
| | | HLA-A*0201 | 15 | P L S S P V F P R | 12 |
| | | HLA-A*0201 | 2 | R T F G G A P G F | 11 |
| | | HLA-A*0201 | 8 | P G F P L G S P L | 11 |
| | | HLA-A*0201 | 6 | G A P G F P L G S | 10 |
| | | HLA-A*0201 | 16 | L S S P V F P R A | 9 |
| | | HLA-A*0201 | 5 | G G A P G F P L G | 8 |
| | | HLA-A*0201 | 7 | A P G F P L G S P | 8 |
| | | HLA-A*0201 | 10 | F P L G S P L S S | 8 |
| | | HLA-A*0203 | 16 | L S S P V F P R A | 9 |
| | | HLA A1 | 16 | L S S P V F P R A | 13 |
| | | HLA A1 | 5 | G G A P G F P L G | 11 |
| | | HLA A1 | 2 | R T F G G A P G F | 9 |
| | | HLA A1 | 10 | F P L G S P L S S | 8 |
| | | HLA A3 | 21 | F P R A G F G S K | 20 |
| | | HLA A3 | 19 | P V F P R A G F G | 19 |
| | | HLA A3 | 2 | R T F GG A P G F | 15 |
| | | HLA A3 | 11 | P L G S P L S S P | 15 |
| | | HLA A3 | 15 | P L S S P V F P R | 14 |
| | | HLA A3 | 1 | R R T F G G A P G | 12 |
| | | HLA A3 | 10 | F P L G S P L S S | 10 |
| | | HLA A3 | 8 | P G F P L G S P L | 9 |
| | | HLA A3 | 13 | G S P L S S P V F | 9 |
| | | HLA A3 | 18 | S P V F P R A G F | 9 |
| | | HLA A3 | 22 | P R A G F G S K G | 9 |
| | | HLA A3 | 25 | G F G S K G S S S | 9 |
| | | HLA A3 | 14 | S P L S S P V F P | 8 |
| | | HLA A3 | 24 | A G F G S K G S S | 8 |
| | RRTFGGAPVFSLGSPLSSPV FPRAPFGSKGSSS | HLA-A*0201 | 11 | S L G S P L S S P | 19 |
| | | HLA-A*0201 | 4 | F G G A P V F S L | 18 |
| | | HLA-A*0201 | 12 | L G S P L S S P V | 15 |
| | | HLA-A*0201 | 8 | P V F S L G S P L | 13 |
| | | HLA-A*0201 | 1 | R R T F G G A P V | 12 |
| | | HLA-A*0201 | 15 | P L S S P V F P R | 12 |
| | | HLA-A*0201 | 2 | R T F G G A P V F | 10 |
| | | HLA-A*0201 | 16 | L S S P V F P R A | 9 |
| | | HLA-A*0201 | 6 | G A P V F S L G S | 8 |
| | | HLA-A*0201 | 10 | F S L G S P L S S | |
| | | HLA-A*0203 | 16 | L S S P V F P R A | 9 |
| | | HLA A1 | 16 | L S S P V F P R A | 13 |
| | | HLA A1 | 10 | F S L G S P L S S | 12 |
| | | HLA A1 | 5 | G G A P V F S L G | 11 |
| | | HLA A1 | 2 | R T F G G A P V F | 10 |
| | | HLA A3 | 2 | R T F G G A P V F | 19 |
| | | HLA A3 | 19 | P V F P R A P F G | 18 |
| | | HLA A3 | 21 | F P R A P F G S K | 18 |
| | | HLA A3 | 8 | P V F S L G S P L | 16 |
| | | HLA A3 | 11 | S L G S P L S S P | 16 |
| | | HLA A3 | 15 | P L S S P V F P R | 14 |
| | | HLA A3 | 1 | R R T F G G A P V | 12 |
| | | HLA A3 | 10 | F S L G S P L S S | 10 |
| | | HLA A3 | 22 | P R A P F G S K G | 10 |
| | | HLA A3 | 13 | G S P L S S P V F | 9 |
| | | HLA A3 | 18 | S P V F P R A P F | 9 |
| | | HLA A3 | 25 | P F G S K G S S S | 9 |
| | | HLA A3 | 5 | G G A P V F S L G | 8 |
| | | HLA A3 | 14 | S P L S S P V F P | 8 |
| | | HLA A3 | 24 | A P F G S K G S S | 8 |
| | VRFLEQQNALAAEVNRLK | HLA-A*0201 | 9 | A L A A E V N R L | 29 |
| | | HLA-A*0201 | 2 | R F L E Q Q N A L | 16 |
| | | HLA-A*0201 | 3 | F L E Q Q N A L A | 16 |
| | | HLA-A*0201 | 6 | Q Q N A L A A E V | 16 |
| | | HLA-A*0201 | 10 | L A A E V N R L K | 10 |
| | | HLA-A*0201 | 4 | L E Q Q N A L A A | 8 |
| | | HLA-A*0201 | 8 | N A L A A E V N R | 8 |
| | | HLA-A*0203 | 1 | V R F L E Q Q N A | 9 |
| | | HLA-A*0203 | 3 | F L E Q Q N A L A | 9 |
| | | HLA-A*0203 | 4 | L E Q Q N A L A A | 9 |
| | | HLA A1 | 3 | F L E Q Q N A L A | 17 |
| | | HLA A3 | 9 | A L A A E V N R L | 16 |
| | | HLA A3 | 3 | F L E Q Q N A L A | 13 |
| | | HLA A3 | 10 | L A A E V N R L K | 13 |
| | | HLA A3 | 8 | N A L A A E V N R | 12 |
| | | HLA A3 | 7 | Q N A L A A E V N | 11 |
| | | HLA A3 | 6 | Q Q N A L A A E V | 10 |
| | | HLA A3 | 2 | R F L E Q Q N A L | 8 |
| | RFLEQQNAALAAEVNRLK | HLA-A*0201 | 9 | A L A A E V N R L | 29 |
| | | HLA-A*0201 | 2 | F L E Q Q N A A L | 23 |
| | | HLA-A*0201 | 6 | Q N A A L A A E V | 17 |
| | | HLA-A*0201 | 1 | R F L E Q Q N A A | 10 |
| | | HLA-A*0201 | 8 | A A L A A E V N R | 10 |
| | | HLA-A*0201 | 10 | L A A E V N R L K | 10 |
| | | HLA-A*0201 | 3 | L E Q Q N A A L A | 8 |
| | | HLA-A*0201 | 5 | Q Q N A A L A A E | 8 |
| | | HLA-A*0203 | 1 | R F L E Q Q N A A | 9 |
| | | HLA-A*0203 | 3 | L E Q Q N A A L A | 9 |
| | | HLA-A*0203 | 4 | E Q Q N A A L A A | 9 |
| | | HLA A1 | 2 | F L E Q Q N A A L | 15 |
| | | HLA A3 | 9 | A L A A E V N R L | 16 |
| | | HLA A3 | 8 | A A L A A E V N R | 15 |
| | | HLA A3 | 2 | F L E Q Q N A A L | 14 |
| | | HLA A3 | 10 | L A A E V N R L K | 13 |
| | | HLA A3 | 6 | Q N A A L A A E V | 11 |
| | | HLA A3 | 7 | N A A L A A E V N | 9 |
| | | HLA A3 | 1 | R F L E Q Q N A A | 8 |
| | | HLA A3 | 4 | E Q Q N A A L A A | 8 |
| | | HLA A3 | 5 | Q Q N A A L A A E | 8 |
| Glycophorin a | KIIFVLLLSAIVSISASS | HLA-A*0201 | 6 | L L L S A I V S I | 30 |
| | | HLA-A*0201 | 2 | I I F V L L L S A | 23 |
| | | HLA-A*0201 | 4 | F V L L L S A I V | 20 |
| | | HLA-A*0201 | 3 | I F V L L L S A I | 16 |
| | | HLA-A*0201 | 7 | L L S A I V S I S | 16 |
| | | HLA-A*0201 | 1 | K I I F V L L L S | 15 |
| | | HLA-A*0201 | 10 | A I V S I S A S S | 14 |
| | | HLA-A*0201 | 5 | V L L L S A I V S | 13 |
| | | HLA-A*0201 | 9 | S A I V S I S A S | 13 |
| | | HLA-A*0201 | 8 | L S A I V S I S A | 10 |
| | | HLA-A*0203 | 8 | L S A I V S I S A | 12 |
| | | HLA-A*0203 | 2 | I I F V L L L S A | 9 |
| | | HLA A1 | 1 | K I I F V L L L S | 10 |
| | | HLA A1 | 8 | L S A I V S I S A | 10 |
| | | HLA A3 | 5 | V L L L S A I V S | 20 |
| | | HLA A3 | 1 | K I I F V L L L S | 18 |
| | | HLA A3 | 6 | L L L S A I V S I | 18 |
| | | HLA A3 | 4 | F V L L L S A I V | 16 |
| | | HLA A3 | 10 | A I V S I S A S S | 16 |
| | | HLA A3 | 2 | I I F V L L L S A | 15 |
| | | HLA A3 | 7 | L L S A I V S I S | 15 |
| | | HLA A3 | 9 | S A I V S I S A S | 8 |
| | KIIFVLLLSEIVSISASS | HLA-A*0201 | 6 | L L L S E I V S I | 30 |
| | | HLA-A*0201 | 2 | I I F V L L L S E | 19 |
| | | HLA-A*0201 | 4 | F V L L L S E I V | 18 |
| | | HLA-A*0201 | 7 | L L S E I V S I S | 18 |
| | | HLA-A*0201 | 3 | I F V L L L S E I | 17 |
| | | HLA-A*0201 | 1 | K I I F V L L L S | 15 |
| | | HLA-A*0201 | 5 | V L L L S E I V S | 13 |
| | | HLA-A*0201 | 9 | S E I V S I S A S | 9 |
| | | HLA-A*0201 | 10 | E I V S I S A S S | 9 |
| | | HLA-A*0203 | 8 | L S E I V S I S A | 9 |
| | | HLA A1 | 8 | L S E I V S I S A | 20 |
| | | HLA A1 | 1 | K I I F V L L L S | 10 |
| | | HLA A3 | 1 | K I I F V L L L S | 18 |
| | | HLA A3 | 6 | L L L S E I V S I | 18 |
| | | HLA A3 | 5 | V L L L S E I V S | 17 |
| | | HLA A3 | 2 | I I F V L L L S E | 15 |
| | | HLA A3 | 7 | L L S E I V S I S | 14 |
| | | HLA A3 | 4 | F V L L L S E I V | 13 |
| | | HLA A3 | 10 | E I V S I S A S S | 13 |
| | | HLA A3 | 9 | S E I V S I S A S | 8 |
| Rare | RTVCLDHANLGEGKLSP | HLA-A*0201 | 7 | H A N L G E G K L | 19 |
| | | HLA-A*0201 | 9 | N L G E G K L S P | 17 |
| | | HLA-A*0201 | 2 | T V C L D H A N L | 16 |
| | | HLA-A*0201 | 4 | C L D H A N L G E | 12 |
| | | HLA-A*0201 | 5 | L D H A N L G E G | 8 |
| | | HLA A1 | 4 | C L D H A N L G E | 18 |
| | | HLA A1 | 9 | N L G E G K L S P | 9 |
| | | HLA A3 | 9 | N L G E G K L S P | 18 |
| | | HLA A3 | 6 | D H A N L G E G K | 15 |
| | | HLA A3 | 2 | T V C L D H A N L | 14 |
| | | HLA A3 | 4 | C L D H A N L G E | 13 |
| | | HLA A3 | 8 | A N L G E G K L S | 10 |
| | RTVCLDHAKLGEGKLSP | HLA-A*0201 | 2 | T V C L D H A K L | 18 |
| | | HLA-A*0201 | 9 | K L G E G K L S P | 18 |
| | | HLA-A*0201 | 7 | H A K L G E G K L | 17 |
| | | HLA-A*0201 | 4 | C L D H A K L G E | 12 |
| | | HLA-A*0201 | 5 | L D H A K L G E G | 9 |
| | | HLA A1 | 4 | C L D H A K L G E | 17 |
| | | HLA A1 | 9 | K L G E G K L S P | 9 |
| | | HLA A3 | 9 | K L G E G K L S P | 21 |
| | | HLA A3 | 1 | R T V C L D H A K | 15 |
| | | HLA A3 | 2 | T V C L D H A K L | 14 |
| | | HLA A3 | 6 | D H A K L G E G K | 14 |
| | | HLA A3 | 4 | C L D H A K L G E | 13 |
| | | HLA A3 | 8 | A K L G E G K L S | 10 |
| | KLAWDFSPGQLDHLFDCFKA SW | HLA-A*0201 | 6 | F S P G Q L D H L | 17 |
| | | HLA-A*0201 | 1 | K L A W D F S P G | 13 |
| | | HLA-A*0201 | 12 | H L F D C F K A S | 12 |
| | | HLA-A*0201 | 2 | L A W D F S P G Q | 11 |
| | | HLA-A*0201 | 3 | A W D F S P G Q L | 11 |
| | | HLA-A*0201 | 10 | Q L D H L F D C F | 11 |
| | | HLA-A*0201 | 9 | G Q L D H L F D C | 8 |
| | | HLA-A*0203 | 12 | D H L F D C F K A | 9 |
| | | HLA A1 | 10 | Q L D H L F D C F | 14 |
| | | HLA A1 | 5 | D F S P G Q L D H | 12 |
| | | HLA A1 | 3 | A W D F S P G Q L | 11 |
| | | HLA A1 | 14 | L F D C F K A S W | 10 |
| | | HLA A1 | 6 | F S P G Q L D H L | 8 |
| | | HLA A3 | 1 | K L A W D F S P G | 17 |
| | | HLA A3 | 10 | Q L D H L F D C F | 16 |
| | | HLA A3 | 5 | D F S P G Q L D H | 12 |
| | | HLA A3 | 13 | H L F D C F K A S | 12 |
| | | HLA A3 | 11 | L D H L F D C F K | 11 |
| | | HLA A3 | 3 | A W D F S P G Q L | 8 |
| | KLAWDFSPEQLDHLFDCFKA SW | HLA-A*0201 | 6 | F S P E Q L D H L | 17 |
| | | HLA-A*0201 | 1 | K L A W D F S P E | 13 |
| | | HLA-A*0201 | 2 | L A W D F S P E Q | 12 |
| | | HLA-A*0201 | 13 | H L F D C F K A S | 12 |
| | | HLA-A*0201 | 3 | A W D F S P E Q L | 11 |
| | | HLA-A*0201 | 10 | Q L D H L F D C F | 11 |
| | | HLA-A*0203 | 12 | D H L F D C F K A | 9 |
| | | HLA A1 | 7 | S P E Q L D H L F | 14 |
| | | HLA A1 | 10 | Q L D H L F D C F | 14 |
| | | HLA A1 | 3 | A W D F S P E Q L | 11 |
| | | HLA A1 | 5 | D F S P E Q L D H | 10 |
| | | HLA A1 | 14 | L F D C F K A S W | 10 |
| | | HLA A3 | 1 | K L A W D F S P E | 17 |
| | | HLA A3 | 10 | Q L D H L F D C F | 16 |
| | | HLA A3 | 13 | H L F D C F K A S | 12 |
| | | HLA A3 | 11 | L D H L F D C F K | 11 |
| | | HLA A3 | 5 | D F S P E Q L D H | 10 |
| | VLNLLWNLAQSDDVPV | HLA-A*0201 | 1 | V L N L L W N L A | 18 |
| | | HLA-A*0201 | 3 | N L L W N L A Q S | 18 |
| | | HLA-A*0201 | 8 | L A Q S D D V P V | 18 |
| | | HLA-A*0201 | 4 | L L W N L A Q S D | 16 |
| | | HLA-A*0201 | 6 | W N L A Q S D D V | 12 |
| | | HLA-A*0201 | 7 | N L A Q S D D V P | 12 |
| | | HLA-A*0203 | 1 | V L N L L W N L A | 9 |
| | | HLA A3 | 3 | N L L W N L A Q S | 18 |
| | | HLA A3 | 4 | L L W N L A Q S D | 16 |
| | | HLA A3 | 7 | N L A Q S D D V P | 15 |
| | | HLA A3 | 1 | V L N L L W N L A | 11 |
| | VLNLLWNLAHSDDVPV | HLA-A*0201 | 1 | V L N L L W N L A | 18 |
| | | HLA-A*0201 | 3 | N L L W N L A H S | 18 |
| | | HLA-A*0201 | 8 | L A H S D D V P V | 18 |
| | | HLA-A*0201 | 4 | L L W N L A H S D | 17 |
| | | HLA-A*0201 | 6 | W N L A H S D D V | 12 |
| | | HLA-A*0201 | 7 | N L A H S D D V P | 12 |
| | | HLA-A*0203 | 1 | V L N L L W N L A | 9 |
| | | HLA A3 | 4 | L L W N L A H S D | 16 |
| | | HLA A3 | 3 | N L L W N L A H S | 15 |
| | | HLA A3 | 7 | N L A H S D D V P | 15 |
| | | HLA A3 | 1 | V L N L L W N L A | 11 |
| | | HLA A3 | 2 | L N L L W N L A H | 11 |
| | FSPGQLDHLFDC | HLA-A*0201 | 1 | F S P G Q L D H L | 17 |
| | | HLA-A*0201 | 4 | G Q L D H L F D C | 8 |
| | | HLA A1 | 1 | F S P G Q L D H L | 8 |
| | FSPEQLDHLFDC | HLA-A*0201 | 1 | F S P E Q L D H L | 17 |
| | | HLA A1 | 2 | S P E Q L D H L F | 14 |
| Smcy | RYTLDELPTMLHKLKIR | HLA-A*0201 | 6 | E L P T M L H K L | 24 |
| | | HLA-A*0201 | 3 | T L D E L P T M L | 23 |
| | | HLA-A*0201 | 2 | Y T L D E L P T M | 20 |
| | | HLA-A*0201 | 9 | T M L H K L K I R | 14 |
| | | HLA-A*0201 | 8 | P T M L H K L K I | 13 |
| | | HLA A1 | 8 | P T M L H K L K I | 14 |
| | | HLA A1 | 4 | L D E L P T M L H | 13 |
| | | HLA A1 | 3 | T L D E L P T M L | 12 |
| | | HLA A1 | 5 | D E L P T M L H K | 10 |
| | | HLA A1 | 2 | Y T L D E L P T M | 8 |
| | | HLA A3 | 5 | D E L P T M L H K | 18 |
| | | HLA A3 | 3 | T L D E L P T M L | 14 |
| | | HLA A3 | 6 | E L P T M L H K L | 10 |
| | | HLA A3 | 7 | L P T M L H K L K | 10 |
| | | HLA A3 | 1 | R Y T L D E L P T | 8 |
| | RYTLDELPAMLHKLKVR | HLA-A*0201 | 3 | T L D E L P A M L | 25 |
| | | HLA-A*0201 | 6 | E L P A M L H K L | 24 |
| | | HLA-A*0201 | 2 | Y T L D E L P A M | 19 |
| | | HLA-A*0201 | 9 | A M L H K L K V R | 16 |
| | | HLA-A*0201 | 8 | P A M L H K L K V | 15 |
| | | HLA-A*0203 | 1 | R Y T L D E L P A | 9 |
| | | HLA A1 | 4 | L D E L P A M L H | 13 |
| | | HLA A1 | 3 | T L D E L P A M L | 12 |
| | | HLA A1 | 5 | D E L P A M L H K | 10 |
| | | HLA A1 | 2 | Y T L D E L P A M | 8 |
| | | HLA A1 | 8 | P A M L H K L K V | 8 |
| | | HLA A3 | 5 | D E L P A M L H K | 18 |
| | | HLA A3 | 3 | T L D E L P A M L | 16 |
| | | HLA A3 | 9 | A M L H K L K V R | 14 |
| | | HLA A3 | 6 | E L P A M L H K L | 12 |
| | | HLA A3 | 7 | L P A M L H K L K | 11 |
| | | HLA A3 | 4 | L D E L P A M L H | 9 |
| | | HLA A3 | 1 | R Y T L D E L P A | 8 |
| Vimen- tin | MALDIEIATYRKLLEGE | HLA-A*0201 | 2 | A L D I E I A T Y | 20 |
| | | HLA-A*0201 | 6 | E I A T Y R K L L | 17 |
| | | HLA-A*0201 | 5 | I E I A T Y R K L | 16 |
| | | HLA-A*0201 | 8 | A T Y R K L L E G | 15 |
| | | HLA-A*0201 | 1 | M A L D I E I A T | 12 |
| | | HLA-A*0201 | 4 | D I E I A T Y R K | 9 |
| | | HLA A1 | 2 | A L D I E I A T Y | 27 |
| | | HLA A1 | 8 | A T Y R K L L E G | 13 |
| | | HLA A1 | 4 | D I E I A T Y R K | 10 |
| | | HLA A1 | 7 | I A T Y R K L L E | 8 |
| | | HLA A3 | 2 | A L D I E I A T Y | 27 |
| | | HLA A3 | 4 | D I E I A T Y R K | 19 |
| | | HLA A3 | 8 | A T Y R K L L E G | 14 |
| | | HLA A3 | 3 | L D I E I A T Y R | 12 |
| | | HLA A3 | 6 | E I A T Y R K L L | 10 |
| | MALDIEIAAYRKLLEGE | HLA-A*0201 | 2 | A L D I E I A A Y | 19 |
| | | HLA-A*0201 | 6 | E I A A Y R K L L | 17 |
| | | HLA-A*0201 | 5 | I E I A A Y R K L | 16 |
| | | HLA-A*0201 | 8 | A A Y R K L L E G | 15 |
| | | HLA-A*0201 | 1 | M A L D I E I A A | 12 |
| | | HLA-A*0201 | 3 | L D I E I A A Y R | 8 |
| | | HLA-A*0201 | 7 | I A A Y R K L L E | 8 |
| | | HLA-A*0201 | 9 | A Y R K L L E G E | 8 |
| | | HLA-A*0203 | 1 | M A L D I E I A A | 11 |
| | | HLA A1 | 2 | A L D I E I A A Y | 27 |
| | | HLA A1 | 4 | D I E I A A Y R K | 10 |
| | | HLA A1 | 7 | I A A Y R K L L E | 8 |
| | | HLA A3 | 2 | A L D I E I A A Y | 24 |
| | | HLA A3 | 4 | D I E I A A Y R K | 22 |
| | | HLA A3 | 3 | L D I E I A A Y R | 14 |
| | | HLA A3 | 8 | A A Y R K L L E G | 14 |
| | | HLA A3 | 6 | E I A A Y R K L L | 12 |
| | | HLA A3 | 7 | I A A Y R K L L E | 8 |
| | | | | | |

**Table 6: HLA-binding peptides representing amino acid exchanges outside the hypervariable regions of HLA-antigens. Class I molecules allelic variants have been identified by comparisons with reference HLA-A*2502:**

| HLA-A Allele | | | | | AA position | AA-exchange compared to (reference HLAA*2502) |
|---|---|---|---|---|---|---|
| A*0230 | | | | | 3 | H>Q |
| A*2408 | | | | | | |
| A*2420 | | | | | | |
| A*2305 | | | | | 7 | Y>C |
| A*2425 | | | | | | |
| A*01011 | A*02016 | A*0225 | A*03011 | A*7404 | 9 | Y>F |
| A*01012 | A*0202 | A*0226 | A*03012 | A*7405 | | |
| A*0103 | A*0203 | A*0227 | A*03013 | A*7406 | | |
| A*0106 | A*0204 | A*0229 | A*0302 | A*7407 | | |
| A*0107 | A*0205 | A*0230 | A*0304 | A*7408 | | |
| A*0108 | A*0206 | A*0231 | A*0305 | A*8001 | | |
| A*0109 | A*0207 | A*0233 | A*0306 | | | |
| A*02011 | A*0208 | A*0234 | A*0307 | | | |
| A*02012 | A*0209 | A*0235 | A*0308 | | | |
| A*02013 | A*0210 | A*0236 | A*0309 | | | |
| A*02014 | A*0211 | A*0237 | A*2503 | | | |
| A*02015 | A*0212 | A*0238 | A*3201 | | | |
| A*02016 | A*0213 | A*0239 | A*3202 | | | |
| A*0202 | A*0216 | A*0240 | A*3203 | | | |
| A*0203 | A*02171 | A*0242 | A*3204 | | | |
| A*0204 | A*02172 | A*0245 | A*3205 | | | |
| A*0207 | A*0218 | A*0246 | A*3206 | | | |
| A*0209 | A*0219 | A*0247 | A*3601 | | | |
| A*02011 | A*02201 | A*0248 | A*3602 | | | |
| A*02012 | A*02202 | A*0249 | A*3603 | | | |
| A*02013 | A*0222 | A*0250 | A*7401 | | | |
| A*02014 | A*0224 | A*0252 | A*7402 | | | |
| A*02015 | | | A*7403 | | | |
| A*0102 | A*2415 | A*2616 | | | 9 | Y>S |
| A*2301 | A*2417 | A*3001 | | | | |
| A*2302 | A*2418 | A*3002 | | | | |
| A*2303 | A*2419 | A*3003 | | | | |
| A*2305 | A*2420 | A*3004 | | | | |
| A*2306 | A*2421 | A*3006 | | | | |
| A*2402101 | A*2422 | A*3007 | | | | |
| A*2402102 | A*2423 | A*3009 | | | | |
| A*24022 | A*2424 | A*3010 | | | | |
| A*24031 | A*2425 | A*3011 | | | | |
| A*24032 | A*2426 | A*3207 | | | | |
| A*2404 | A*2427 | | | | | |
| A*2405 | A*2428 | | | | | |
| A*2406 | A*2429 | | | | | |
| A*2407 | A*2430 | | | | | |
| A*2408 | A*2431 | | | | | |
| A*2410 | A*2432 | | | | | |
| A*2413 | A*2433 | | | | | |
| A*2414 | A*2434 | | | | | |
| | | | | | | |
| A*2416 | A*3103 | | | | 9 | Y>T |
| A*2901101 | A*3104 | | | | | |
| A*2902 | A*3105 | | | | | |
| A*2903 | A*3106 | | | | | |
| A*2904 | A*3301 | | | | | |
| A*2905 | A*3303 | | | | | |
| A*2906 | A*3304 | | | | | |
| A*31012 | A*3305 | | | | | |
| A*3102 | A*3306 | | | | | |
| A*0250 | | | | | 12 | V>M |
| A*6802 | | | | | | |
| A*6815 | | | | | | |
| A*0102 | A*3007 | | | | 17 | R>S |
| A*3001 | A*3008 | | | | | |
| A*3002 | A*3009 | | | | | |
| A*3003 | A*3010 | | | | | |
| A*3004 | A*3011 | | | | | |
| A*3006 | | | | | | |
| A*1102 | | | | | 19 | E>K |
| A*0242 | | | | | 24 | A>S |
| A*0221 | | | | | 30 | D>N |
| A*3006 | | | | | 31 | T>A |
| A*8001 | | | | | 31 | T>S |
| A*0109 | | | | | 33 | F>L |
| A*8001 | | | | | 35 | R>Q |
| A*2615 | | | | | 36 | F>L |
| A*0231 | | | | | 41 | A>G |
| A*0202 | | | | | 43 | Q>R |
| A*0205 | | | | | | |
| A*0208 | | | | | | |
| A*0214 | | | | | | |
| A*0247 | | | | | | |
| A*01011 | A*0108 | | | | 44 | R>K |
| A*01012 | A*0109 | | | | | |
| A*0102 | A*3601 | | | | | |
| A*0103 | A*3602 | | | | | |
| A*0106 | A*3603 | | | | | |
| A*0107 | | | | | | |
| A*3306 | | | | | 52 | I>M |
| A*3305 | | | | | 54 | I>M |
| A*0107 | A*3010 | | | | 56 | G>R |
| A*3001 | A*31012 | | | | | |
| A*3002 | A*3102 | | | | | |
| A*3004 | A*3103 | | | | | |
| A*3006 | A*3104 | | | | | |
| A*3007 | A*3105 | | | | | |
| A*3008 | A*3106 | | | | | |
| A*3009 | A*3404 | | | | | |
| | | | | | | |
| A*0228 | | | | | 56 | G>S |
| A*8008 | | | | | 56 | G>E |

| | | | | | | |
|---|---|---|---|---|---|---|
| http://www.eurekah.com/reports/vaccines/kast/01/ | | | | | | |

## Claims

1. An ex-vivo method for determining the progression, regression or onset of a specific pathogenic condition or disease in an individual, the treatment of the individual comprises allogeneic transplantation of hematopoetic stem cells from a donor to said individual, the method comprising
(i) providing a sample of the diseased individual before and / or after allo-transplantation, and
(ii) monitoring the sample for
(a) a protein, a peptide or polypeptide representing a single amino acid mismatch, or
(b) an antibody which selectively binds to said protein, peptide or polypeptide, or
(c) cytotoxic T-cells that specifically recognize said protein, peptide or polypeptide bound to the HLA molecule complex, and
(iii) determining thereby the progression, regression or onset of a specific pathogenic condition or disease in the individual,
wherein said single amino acid mismatch is obtained by the following steps:
(ai) providing a sample of selected tissue or organ derived from the individual suffering from said disease or pathogenic condition as well as a sample derived from the donor individual that does not suffer from said disease or pathogenic condition
(aii) screening each sample for at least one single amino-acid mismatch of allelic variants of said protein, which binds to HLA protein and is encoded by a single SNP, or expression product thereof, or a fragment of this expression product, and (aiii) selecting a single amino acid mismatch which occurs only in the diseased individual.

2. An ex-vivo method according to claim 1, wherein the peptide or polypeptide representing said single amino-acid mismatch binds only or preferably to the antigen presenting cell or HLA protein of the diseased individual.

3. An ex-vivo method according to claim 2, wherein the peptide or polypeptide representing said single amino-acid mismatch is recognized by cytotoxic T-lymphocytes (CTLs).

4. An ex-vivo method according to any of the claims 1-3, wherein the disease or pathogenic condition is cancer.

5. An ex-vivo method of claim 4, wherein the cancer is leukemia.

6. An ex-vivo method of claim 5, wherein the specific protein screened for and related to said specific disease is selected from the group consisting of the tissue markers given in Table 2.

## Patentansprüche

1. Ex-vivo-Verfahren zur Bestimmung der Progression, Regression oder dem Beginn eines spezifischen pathogenen Zustands oder einer spezifischen Erkrankung bei einem Individuum, wobei die Behandlung des Individuums die allogene Transplantation von hämatopoetischen Stammzellen von einem Spender auf dieses Individuum umfasst, wobei das Verfahren folgende Schritte umfasst
(i) Bereitstellen einer Probe des erkrankten Individuums vor und/oder nach der Allotransplantation; und
(ii) Untersuchung der Probe auf
(a) ein Protein, ein Peptid oder ein Polypeptid, das eine einzelne Aminosäurefehlpaarung darstellt, oder
(b) einen Antikörper, der selektiv an dieses Protein, Peptid oder Polypeptid bindet, oder
(c) cytotoxische T-Zellen, die spezifisch dieses an den HLA-Molekülkomplex gebundene Protein, Peptid oder Polypeptid erkennen, und
(iii) **dadurch** Bestimmen der Progression, Regression oder des Beginns eines spezifischen pathogenen Zustands oder einer spezifischen Erkrankung bei einem Individuum,
wobei diese einzelne Aminosäurefehlpaarung durch die folgenden Schritte erhalten wird:
(ai) Bereitstellen einer Probe eines gewählten Gewebes oder Organs, die von dem an dieser Erkrankung oder dem pathogenen Zustand leidenden Individuum stammt, sowie einer von dem Spenderindividuum stammenden Probe, wobei der Spender nicht an dieser Erkrankung oder dem pathogenen Zustand leidet,
(aii) Screenen von jeder Probe auf mindestens eine einzelne Aminosäurefehlpaarung von Allelvarianten dieses Proteins, das an das HLA-Protein bindet und durch einen einzelnen SNP kodiert wird, oder nach einem Expressionsprodukt davon oder einem Fragment dieses Expressionsprodukts, und (aiii) Auswählen einer einzelnen Aminosäurefehlpaarung, die nur bei dem erkrankten Individuum auftritt.

2. Ex-vivo-Verfahren nach Anspruch 1, wobei ein diese einzelne Aminosäurefehlpaarung darstellendes Peptid oder Polypeptid nur oder vorzugsweise an die Antigen präsentierende Zelle oder das HLA-Protein des erkrankten Individuums bindet.

3. Ex-vivo-Verfahren nach Anspruch 2, wobei das diese einzelne Aminosäurefehlpaarung darstellende Peptid oder Polypeptid von cytotoxischen T-Lymphozyten (CTLs) erkannt wird.

4. Ex-vivo-Verfahren nach einem der Ansprüche 1-3, wobei diese Erkrankung oder der pathogene Zustand Krebs ist.

5. Ex-vivo-Verfahren nach Anspruch 4, wobei der Krebs Leukämie ist.

6. Ex-vivo-Verfahren nach Anspruch 5, wobei das spezifische Protein, auf das gescreent wird und das mit dieser spezifischen Erkrankung im Zusammenhang steht, aus der Gruppe der in Tabelle 2 angegebenen Gewebemarker ausgewählt wird.

## Revendications

1. Procédé ex vivo pour déterminer la progression, la régression ou l'attaque d'une maladie ou condition pathogène spécifique chez un individu, le traitement de l'individu comprend une transplantation allogénique de cellules souches hématopoétiques depuis un donneur chez ledit individu, le procédé comprenant
(i) la fourniture d'un échantillon de l'individu malade avant et/ou après allotransplantation et
(ii) la surveillance de l'échantillon quant à
(a) une protéine, un peptide ou un polype ptide représentant une discordance d'unique acide aminé, ou
(b) un anticorps qui se lie de façon sélective à ladite protéine, audit peptide ou audit polypeptide, ou
(c) des cellules T cytotoxiques qui reconnaissent de façon spécifique ladite protéine, ledit peptide ou ledit polypeptide lié(e) au complexe de molécule HLA, et
(iii) la détermination ainsi de la progression, la régression ou l'attaque d'une maladie ou condition pathogène spécifique chez l'individu
dans lequel ladite discordance d'unique acide aminé est obtenue au moyen des étapes qui suivent :
(ai) fourniture d'un échantillon de tissu ou d'organe sélectionné dérivé de l'individu qui souffre de ladite maladie ou condition pathogène de même que d'un échantillon dérivé de l'individu donneur qui ne souffre pas de ladite maladie ou condition pathogène
(aii) dépistage au niveau de chaque échantillon d'au moins une discordance d'unique acide aminé de variantes alléliques de ladite protéine, laquelle est liée à une protéine HLA et est codée au moyen d'un unique SNP (polymorphisme d'unique nucléotide) ou de son produit d'expression ou d'un fragment de ce produit d'expression, et
(aiii) sélection d'une discordance d'unique acide aminé qui survient seulement chez l'individu malade.

2. Procédé ex vivo selon la revendication 1, dans lequel le peptide ou polypeptide représentant ladite discordance d'unique acide aminé se lie seulement ou de préférence sur la cellule de présentation d'antigène ou protéine HLA de l'individu malade.

3. Procédé ex vivo selon la revendication 2, dans lequel le peptide ou polypeptide représentant ladite discordance d'unique acide aminé est reconnu par des lymphocytes T cytotoxiques (CTL).

4. Procédé ex vivo selon l'une quelconque des revendications 1 à 3, dans lequel la maladie ou condition pathogène est un cancer.

5. Procédé ex vivo selon la revendication 4, dans lequel le cancer est une leucémie.

6. Procédé ex vivo selon la revendication 5, dans lequel la protéine spécifique dépistée pour et rapportée à ladite maladie spécifique est choisie parmi le groupe constitué par les marqueurs tissulaires présentés dans le Tableau 2.
